# FASCICULE DE BREVET EUROPEEN

(11) **EP 1 330 530 B1**
(45) Date de publication et mention de la délivrance du brevet: **02.09.2009**
(21) Numéro de dépôt: 01983651.9
(22) Date de dépôt: 30.10.2001
(51) Int. Cl.: C12N 15/82, C12N 9/02, A01H 5/00

(54) **Procédé pour rendre des plantes tolérantes aux herbicides inhibiteurs d'HPPD par contournement de la voie métabolique de l'HPPD.**
Prozess zur Herstellung von HPPD-Inhibitor-Herbizid toleranten Planzen durch Umgehung des HPPD-Stoffwechselwegs.
Process to provide HPPD inhibitor herbicide tolerant plants through bypassing HPPD metabolic pathway.

(30) Priorité: 30.10.2000 FR 0013942
(43) Date de publication de la demande: 30.07.2003
(60) Demande divisionnaire: 09164940.0
(73) Titulaire: Bayer CropScience S.A., 69009 Lyon (FR)
(72) Inventeur: ZINK, Olivier, F-63000 Clermont-Ferrand (FR); PAGET, Eric, F-69300 Caluire (FR); ROLLAND, Anne, F-69009 Lyon (FR); SAILLAND, Alain, F-63370 Saint-Didier-au-Mont-d'Or (FR); FREYSSINET, Georges, F-69450 Saint-Cyr-au-Mont-d'Or (FR)
(74) Mandataire: Guitton, Carole
(86) Numéro de dépôt international: PCT/FR2001/003364
(87) Numéro de publication internationale: WO 2002/036787

(56) Documents cités:
- EP-A- 0 343 100
- EP-A- 0 633 317
- WO-A-96/38567
- WO-A-98/21348
- WO-A-99/24585
- WO-A-99/34008
- WO-A-99/53081
- PRIETO M A ET AL: "Molecular characterization of the 4-hydrophenylacetate catabolic pathway of Escherichia coli W: Engineering a mobile aromatic degradative cluster." JOURNAL OF BACTERIOLOGY, vol. 178, no. 1, 1996, pages 111-120, XP002172217 ISSN: 0021-9193
- GRAUSEM B. ET AL: 'Functional expression of Saccharomyces cerevisiae CYP51A1 encoding lanosterol-14-demethylase in tobacco results in bypass of endogenous sterol biosynthetic pathway and resistance to an obtusifoliol-14-demethylase herbicide inhibitor' THE PLANT JOURNAL vol. 7, no. 5, Mai 1995, pages 761 - 770, XP002063588
- MATRINGE M. ET AL: 'p-hydroxyphenylpyruvate dioxygenase inhibitor-resistant plants' PEST MANAGEMENT SCIENCE vol. 61, 2005, pages 269 - 276

### LISTE DE SEQUENCES

<110> Aventis CropScience S.A.
<120> Plantes tolérantes aux herbicides par contournement de voie métabolique
<130> gènes du shunt
<140>
   <141>
<160> 22
<170> PatentIn Ver. 2.1
<210> 1
   <211> 1683
   <212> ADN
   <213> Arthrobacter globiformis
<220>
   <221> CDS
   <222> (1)..(1683)
<400> 1
   atg act tca ctt aca gtg tcc ggc cgg gtg gcg cag gtc ctc agc agc    48
   Met Thr Ser Leu Thr Val Ser Gly Arg Val Ala Gln Val Leu Ser Ser
    1               5                  10                  15
   tat gtc agc gat gtg ttc ggt gtg atg ggc aac gga aac gtc tac ttc    96
   Tyr Val Ser Asp Val Phe Gly Val Met Gly Asn Gly Asn Val Tyr Phe
               20                  25                  30
   ctg gac gcc gcc gag aag gag ggc ctc cgc ttc acg gcc gta cgc cat    144
   Leu Asp Ala Ala Glu Lys Glu Gly Leu Arg Phe Thr Ala Val Arg His
           35                  40                  45
   gaa ggt gcc gcc atc gcg gcg gcg gac gcc tac tat cgg gca tcc ggg    192
   Glu Gly Ala Ala Ile Ala Ala Ala Asp Ala Tyr Tyr Arg Ala Ser Gly
       50                  55                  60
   cgc ctg gcg gcg ggg acc acc acc tac ggc ccc ggt tac acc aac gcc    240
   Arg Leu Ala Ala Gly Thr Thr Thr Tyr Gly Pro Gly Tyr Thr Asn Ala
   65                  70                  75                  80
   ctg acg gcc ctc gcc gag gcg gtc cag gcg cag atc ccc gtg gtg ctc    288
   Leu Thr Ala Leu Ala Glu Ala Val Gln Ala Gln Ile Pro Val Val Leu
                   85                  90                  95
   gtc acc ggg gac gcc ccg agc agc ggc gcc cgg cct tgg gac gtg gac    336
   Val Thr Gly Asp Ala Pro Ser Ser Gly Ala Arg Pro Trp Asp Val Asp
               100                 105                 110
   cag gcc gcg atc gcc gcc ggg ctg ggg gcg gcg acc ttc acg gtc acc    384
   Gln Ala Ala Ile Ala Ala Gly Leu Gly Ala Ala Thr Phe Thr Val Thr
           115                 120                 125
   cgt gaa gcc gca ggc tcc atc acg cag gaa gcg gtg gag tac gca ctt    432
   Arg Glu Ala Ala Gly Ser Ile Thr Gln Glu Ala Val Glu Tyr Ala Leu
       130                 135                 140
   gcc cgg cgg acc gcc gtc gtg atc gcc gtt cca tac gac ctg tcg gcc    480
   Ala Arg Arg Thr Ala Val Val Ile Ala Val Pro Tyr Asp Leu Ser Ala
   145                 150                 155                 160
   ctt gag gcg gcg gag gaa gat ctt ccc gtg ccg ccg gcg gcc tcg gtt    528
   Leu Glu Ala Ala Glu Glu Asp Leu Pro Val Pro Pro Ala Ala Ser Val
               165                 170                 175
   ccg gac gcc atc ggc ggc gga ctc gga cgg gcg gcc gaa gtg cgg gcg     576
   Pro Asp Ala Ile Gly Gly Gly Leu Gly Arg Ala Ala Glu Val Arg Ala
           180                 185                 190
   gcc gaa ttg ctg gcg ggc gcg aag cgg ccg ctc atc ctt gcc ggc cgc     624
   Ala Glu Leu Leu Ala Gly Ala Lys Arg Pro Leu Ile Leu Ala Gly Arg
           195                 200                 205
   ggt gcg cac ctc gca gga gcc ggc ccc gaa ctc cgg gaa ctc gcc gac     672
   Gly Ala His Leu Ala Gly Ala Gly Pro Glu Leu Arg Glu Leu Ala Asp
       210                 215                 220
   cgc ctc ggc gcg ctc acg gcc ggc acc gca ctg gcg ctg aac ctg ctg     720
   Arg Leu Gly Ala Leu Thr Ala Gly Thr Ala Leu Ala Leu Asn Leu Leu
   225                 230                 235                 240
   cag ggc gag ggg tac ctc ggc gtc gcg ggc ggc ttc ggc acg gat acc     768
   Gln Gly Glu Gly Tyr Leu Gly Val Ala Gly Gly Phe Gly Thr Asp Thr
               245                 250                 255
   gcc gcc ggg ctc atg ggc gag gcg gac gtg gtg ctc gtg gcg gga gcc     816
   Ala Ala Gly Leu Met Gly Glu Ala Asp Val Val Leu Val Ala Gly Ala
           260                 265                 270
   agc ctg acc ccc ttc acc atg cgc ttc ggc cac ctg atc ggc ccg gac     864
   Ser Leu Thr Pro Phe Thr Met Arg Phe Gly His Leu Ile Gly Pro Asp
           275                 280                 285
   gcc acc gtg atc cag atc gac acc gcc atg gag ccg acg gac ccg cgg     912
   Ala Thr Val Ile Gln Ile Asp Thr Ala Met Glu Pro Thr Asp Pro Arg
       290                 295                 300
   gtg gac ctg ttt gtc agt gcg gac gcg aag gcc gct gcc ggc cgg atc     960
   Val Asp Leu Phe Val Ser Ala Asp Ala Lys Ala Ala Ala Gly Arg Ile
   305                 310                 315                 320
   ctc cgg ctg ctg gat gac gcc gcc ggg gcc aat gcg tcg aag gcc tgg     1008
   Leu Arg Leu Leu Asp Asp Ala Ala Gly Ala Asn Ala Ser Lys Ala Trp
               325                 330                 335
   cgc gcg gaa gca ctc aag cgt ctg gcc gaa gga ccc tgc cac cac ccc     1056
   Arg Ala Glu Ala Leu Lys Arg Leu Ala Glu Gly Pro Cys His His Pro
           340                 345                 350
   ggc acc gca gag acc acg gac ggc cgc ctt gac ccc cgg gcg ctt gct     1104
   Gly Thr Ala Glu Thr Thr Asp Gly Arg Leu Asp Pro Arg Ala Leu Ala
           355                 360                 365
   tcg gca ctg gat gcc gtc ctg ccg gaa cgc cgc acc gtg gtc cag gac     1152
   Ser Ala Leu Asp Ala Val Leu Pro Glu Arg Arg Thr Val Val Gln Asp
       370                 375                 380
   ggc ggg cac ttc ctg ggc tgg gca ccc atg tac tgg cgc atc ccc cgt     1200
   Gly Gly His Phe Leu Gly Trp Ala Pro Met Tyr Trp Arg Ile Pro Arg
   385                 390                 395                 400
   cct cag gac ctg gtc atg gtg ggg acc gcg tac cag tcg atc ggg ctt     1248
   Pro Gln Asp Leu Val Met Val Gly Thr Ala Tyr Gln Ser Ile Gly Leu
               405                 410                 415
   ggc ctg gcc agc gcc gtg ggg gcg tcc cgg gcc gtg gac gac ggc aat     1296
   Gly Leu Ala Ser Ala Val Gly Ala Ser Arg Ala Val Asp Asp Gly Asn
           420                 425                 430
   atc ctg gtg ctg gcg gcg ggc gac ggc gga ttc ctg atg ggc ctg tcc     1344
   Ile Leu Val Leu Ala Ala Gly Asp Gly Gly Phe Leu Met Gly Leu Ser
           435                 440                 445
   gac ctg gaa tcg ctc gtg ggc gcg gcg agc agc gcc gtc gtg gtg atc    1392
   Asp Leu Glu Ser Leu Val Gly Ala Ala Ser Ser Ala Val Val Val Ile
       450                 455                 460
   tac aac gac gcc gcc tac ggg gcc gag atc cat cag tac ggc tca cgg    1440
   Tyr Asn Asp Ala Ala Tyr Gly Ala Glu Ile His Gln Tyr Gly Ser Arg
   465                 470                 475                 480
   ggg ctc acc gaa aag ccc atg ctg atc ccc gaa gtg gac ttc agc ggg    1488
   Gly Leu Thr Glu Lys Pro Met Leu Ile Pro Glu Val Asp Phe Ser Gly
                   485                 490                 495
   att gcc cgc gcg atc ggg gcg gaa tcc gca atc atc cgc aag ctg tcg    1536
   Ile Ala Arg Ala Ile Gly Ala Glu Ser Ala Ile Ile Arg Lys Leu Ser
               500                 505                 510
   gac ctc tcc gcg ctc acg gac tgg atc gag gcc ggc gcc agg gga acc    1584
   Asp Leu Ser Ala Leu Thr Asp Trp Ile Glu Ala Gly Ala Arg Gly Thr
           515                 520                 525
   ttc gtg gcc gac tgc cgc atc acc tca agc gtc cgg gcc ccg tgg ctg    1632
   Phe Val Ala Asp Cys Arg Ile Thr Ser Ser Val Arg Ala Pro Trp Leu
       530                 535                 540
   agc gaa tgg atg agg gcc tcg caa gcg gcg aag gag gcg gtg gcg ggc    1680
   Ser Glu Trp Met Arg Ala Ser Gln Ala Ala Lys Glu Ala Val Ala Gly
   545                 550                 555                 560
   tag                                                                1683
<210> 2
   <211> 561
   <212>
   <213> Arthrobacter globiformis
<400> 2
<210> 3
   <211> 1944
   <212> ADN
   <213> Séquence artificielle
<220>
   <223> Description de la séquence artificielle: mutant d'HPPO d'A. globiformis
<220>
   <221> CDS
   <222> (55)..(1737)
<400> 3
   ccgacgtcgc atgctcccgg ccgccatggc ggccgcggga attcgattga attc atg    57
                                                               Met
                                                                 1
   act tca ctt aca gtg tcc ggc cgg gtg gcg cag gtc ctc agc agc tat   105
   Thr Ser Leu Thr Val Ser Gly Arg Val Ala Gln Val Leu Ser Ser Tyr
                   5                   10                  15
   gtc agc gat gtg ttc ggt gtg atg ggc aac gga aac gtc tac ttc ctg   153
   Val Ser Asp Val Phe Gly Val Met Gly Asn Gly Asn Val Tyr Phe Leu
               20                  25                  30
   gac gcc gcc gag aag gag ggc ctc cgc ttc acg gcc gta cgc cat gaa   201
   Asp Ala Ala Glu Lys Glu Gly Leu Arg Phe Thr Ala Val Arg His Glu
           35                  40                  45
   ggt gcc gcc atc gcg gcg gcg gac gcc tac tat cgg gca tcc ggg cgc   249
   Gly Ala Ala Ile Ala Ala Ala Asp Ala Tyr Tyr Arg Ala Ser Gly Arg
       50                  55                  60                  65
   ctg gcg gcg ggg acc acc acc tac ggc ccc ggt tac acc aac gcc ctg   297
   Leu Ala Ala Gly Thr Thr Thr Tyr Gly Pro Gly Tyr Thr Asn Ala Leu
                       70                  75                  80
   acg gcc ctc gcc gag gcg gtc cag gcg cag atc ccc gtg gtg ctc gtc   345
   Thr Ala Leu Ala Glu Ala Val Gln Ala Gln Ile Pro Val Val Leu Val
                   85                  90                  95
   acc ggg gac gcc ccg agc agc ggc gcc cgg cct tgg gac gtg gac cag   393
   Thr Gly Asp Ala Pro Ser Ser Gly Ala Arg Pro Trp Asp Val Asp Gln
               100                 105                 110
   gcc gcg atc gcc gcc ggg ctg ggg gcg gcg acc ttc acg gtc acc cgt   441
   Ala Ala Ile Ala Ala Gly Leu Gly Ala Ala Thr Phe Thr Val Thr Arg
           115                 120                 125
   gaa gcc gca ggc tcc atc acg cag gaa gcg gtg gag tac gca ctt gcc   489
   Glu Ala Ala Gly Ser Ile Thr Gln Glu Ala Val Glu Tyr Ala Leu Ala
       130                 135                 140                 145
   cgg cgg acc gcc gtc gtg atc gcc gtt cca tac gac ctg tcg gcc ctt   537
   Arg Arg Thr Ala Val Val Ile Ala Val Pro Tyr Asp Leu Ser Ala Leu
                   150                 155                 160
   gag gcg gcg gag gaa gat ctt ccc gtg ccg ccg gcg gcc tcg gtt ccg    585
   Glu Ala Ala Glu Glu Asp Leu Pro Val Pro Pro Ala Ala Ser Val Pro
               165                 170                 175
   gac gcc atc ggc ggc gga ctc gga cgg gcg gcc gaa gtg cgg gcg gcc    633
   Asp Ala Ile Gly Gly Gly Leu Gly Arg Ala Ala Glu Val Arg Ala Ala
           180                 185                 190
   gaa ttg ctg gcg ggc gcg aag cgg ccg ctc atc ctt gcc ggc cgc ggt    681
   Glu Leu Leu Ala Gly Ala Lys Arg Pro Leu Ile Leu Ala Gly Arg Gly
       195                 200                 205
   gcg cac ctc gca gga acc ggc ccc gaa ctc cgg gaa ctc gcc gac cgc    729
   Ala His Leu Ala Gly Thr Gly Pro Glu Leu Arg Glu Leu Ala Asp Arg
   210                 215                 220                 225
   ctc ggc gcg ctc acg gcc ggc acc gca ctg gcg ctg aac ctg ctg cag    777
   Leu Gly Ala Leu Thr Ala Gly Thr Ala Leu Ala Leu Asn Leu Leu Gln
                   230                 235                 240
   ggc gag ggg tac ctc ggc gtc gcg ggc ggc ttc ggc acg gat acc gcc    825
   Gly Glu Gly Tyr Leu Gly Val Ala Gly Gly Phe Gly Thr Asp Thr Ala
               245                 250                 255
   gcc ggg ctc atg ggc gag gcg gac gtg gtg ctc gtg gcg gga gcc agc    873
   Ala Gly Leu Met Gly Glu Ala Asp Val Val Leu Val Ala Gly Ala Ser
           260                 265                 270
   ctg acc ccc ttc acc atg cgc ttc ggc cac ctg atc ggc ccg gac gcc    921
   Leu Thr Pro Phe Thr Met Arg Phe Gly His Leu Ile Gly Pro Asp Ala
       275                 280                 285
   acc gtg atc cag atc gac acc gcc atg gag ccg acg gac ccg cgg gtg    969
   Thr Val Ile Gln Ile Asp Thr Ala Met Glu Pro Thr Asp Pro Arg Val
   290                 295                 300                 305
   gac ctg ttt gtc agt gcg gac gcg aag gcc gct gcc ggc cgg atc ctc    1017
   Asp Leu Phe Val Ser Ala Asp Ala Lys Ala Ala Ala Gly Arg Ile Leu
                   310                 315                 320
   cgg ctg ctg gat gac gcc gcc ggg gcc aat gcg tcg aag gcc tgg cgc    1065
   Arg Leu Leu Asp Asp Ala Ala Gly Ala Asn Ala Ser Lys Ala Trp Arg
               325                 330                 335
   gcg gaa gca ctc aag cgt ctg gcc gaa gga ccc tgc cac cac ccc ggc    1113
   Ala Glu Ala Leu Lys Arg Leu Ala Glu Gly Pro Cys His His Pro Gly
           340                 345                 350
   acc gca gag acc acg gac ggc cgc ctt gac ccc cgg gcg ctt gct tcg    1161
   Thr Ala Glu Thr Thr Asp Gly Arg Leu Asp Pro Arg Ala Leu Ala Ser
       355                 360                 365
   gca ctg gat gcc gtc ctg ccg gaa cgc cgc acc gtg gtc cag gac ggc    1209
   Ala Leu Asp Ala Val Leu Pro Glu Arg Arg Thr Val Val Gln Asp Gly
   370                 375                 380                 385
   ggg cac ttc ctg ggc tgg gca ccc atg tac tgg cgc atc ccc cgt cct    1257
   Gly His Phe Leu Gly Trp Ala Pro Met Tyr Trp Arg Ile Pro Arg Pro
                   390                 395                 400
   cag gac ctg gtc atg gtg ggg acc gcg tac cag tcg atc ggg ctt ggc    1305
   Gln Asp Leu Val Met Val Gly Thr Ala Tyr Gln Ser Ile Gly Leu Gly
               405                 410                 415
   ctg gcc agc gcc gtg ggg gcg tcc cgg gcc gtg gac gac ggc aat atc    1353
   Leu Ala Ser Ala Val Gly Ala Ser Arg Ala Val Asp Asp Gly Asn Ile
           420                     425                     430
   ctg gtg ctg gcg gcg ggc gac ggc gga ttc ctg atg ggc ctg tcc gac     1401
   Leu Val Leu Ala Ala Gly Asp Gly Gly Phe Leu Met Gly Leu Ser Asp
       435                     440                     445
   ctg gaa tcg ctc gtg ggc gcg gcg agc agc gcc gtc gtg gtg atc tac     1449
   Leu Glu Ser Leu Val Gly Ala Ala Ser Ser Ala Val Val Val Ile Tyr
   450                     455                     460                     465
   aac gac gcc gcc tac ggg gcc gag atc cat cag tac ggc tca cgg ggg     1497
   Asn Asp Ala Ala Tyr Gly Ala Glu Ile His Gln Tyr Gly Ser Arg Gly
                   470                     475                     480
   ctc acc gaa aag ccc atg ctg atc ccc gaa gtg gac ttc agc ggg att     1545
   Leu Thr Glu Lys Pro Met Leu Ile Pro Glu Val Asp Phe Ser Gly Ile
               485                     490                     495
   gcc cgc gcg atc ggg gcg gaa tcc gca atc atc cgc aag ctg tcg gac     1593
   Ala Arg Ala Ile Gly Ala Glu Ser Ala Ile Ile Arg Lys Leu Ser Asp
           500                     505                     510
   ctc tcc gcg ctc acg gac tgg atc gag gcc ggc gcc agg gga acc ttc     1641
   Leu Ser Ala Leu Thr Asp Trp Ile Glu Ala Gly Ala Arg Gly Thr Phe
       515                     520                     525
   gtg gcc gac tgc cgc atc acc tca agc gtc cgg gcc ccg tgg ctg agc     1689
   Val Ala Asp Cys Arg Ile Thr Ser Ser Val Arg Ala Pro Trp Leu Ser
   530                     535                     540                     545
   gaa tgg atg agg gcc tcg caa gcg gcg aag gag gcg gtg gcg ggc tag     1737
   Glu Trp Met Arg Ala Ser Gln Ala Ala Lys Glu Ala Val Ala Gly
                   550                     555                     560
   ggccggcctc gtcgaaatgc cgccctccaa cccaactcag taccagctca gggcgttctc 1797
   agggctggga acgccctgag ctgctactca tttgttcgaa ctcgagaatt caatcactag 1857
   tgaattcgcg gccgcctgca ggtcgaccat atgggagagc tcccaacgcg ttggatgcat 1917
   agcttgagta ttctatagtg tcaccta                                     1944
<210> 4
   <211> 561
   <212>
   <213> Séquence artificielle
   <223> Description de la séquence artificielle: mutant d'HPPO d'A. globiformis
<400> 4
<210> 5
   <211> 1962
   <212> ADN
   <213> Séquence artificielle
<220>
   <223> Description de la séquence artificielle: mutant d'HPPO d'A. globiformis
<220>
   <221> CDS
   <222> (111)..(1793)
<400> 5
   aggtgacact atagaatact caagctatgc atccaacgcg ttgggagctc tcccatatgg 60
   tcgacctgca ggcggccgcg aattcactag tgattggaag gatccggtgc atg act    116
                                                         Met Thr
                                                          1
   tca ctt aca gtg tcc ggc cgg gtg gcg cag gtc ctc agc agc tat gtc   164
   Ser Leu Thr Val Ser Gly Arg Val Ala Gln Val Leu Ser Ser Tyr Val
            5                  10                  15
   agc gat gtg ttc ggt gtg atg ggc aac gga aac gtc tac ttc ctg gac   212
   Ser Asp Val Phe Gly Val Met Gly Asn Gly Asn Val Tyr Phe Leu Asp
        20                  25                  30
   gcc gcc gag aag gag ggc ctc cgc ttc acg gcc gta cgc cat gaa ggt   260
   Ala Ala Glu Lys Glu Gly Leu Arg Phe Thr Ala Val Arg His Glu Gly
    35                  40                  45                  50
   gcc gcc atc gcg gcg gcg gac gcc tac tat cgg gca tcc ggg cgc ctg   308
   Ala Ala Ile Ala Ala Ala Asp Ala Tyr Tyr Arg Ala Ser Gly Arg Leu
                    55                  60                  65
   gcg gcg ggg acc acc acc tac ggc ccc ggt tac acc aac gcc ctg acg   356
   Ala Ala Gly Thr Thr Thr Tyr Gly Pro Gly Tyr Thr Asn Ala Leu Thr
                70                  75                  80
   gcc ctc gcc gag gcg gtc cag gcg cag atc ccc gtg gtg ctc gtc acc   404
   Ala Leu Ala Glu Ala Val Gln Ala Gln Ile Pro Val Val Leu Val Thr
            85                  90                  95
   ggg gac gcc ccg agc agc ggc gcc cgg cct tgg gac gtg gac cag gcc   452
   Gly Asp Ala Pro Ser Ser Gly Ala Arg Pro Trp Asp Val Asp Gln Ala
       100                 105                 110
   gcg atc gcc ggc ggg ctg ggg gcg gcg acc ttc acg gtc acc cgt gaa      500
   Ala Ile Ala Gly Gly Leu Gly Ala Ala Thr Phe Thr Val Thr Arg Glu
   115                 120                 125                 130
   gcc gca ggc tcc atc acg cag gaa gcg gtg gag tac gca ctt gcc cgg      548
   Ala Ala Gly Ser Ile Thr Gln Glu Ala Val Glu Tyr Ala Leu Ala Arg
                   135                 140                 145
   cgg acc gcc gtc gtg atc gcc gtt cca tac gac ctg tcg gcc ctt gag      596
   Arg Thr Ala Val Val Ile Ala Val Pro Tyr Asp Leu Ser Ala Leu Glu
               150                 155                 160
   gcg gca gag gaa gat ctt ccc gtg ccg ccg gcg gcc tcg gtt ccg gac      644
   Ala Ala Glu Glu Asp Leu Pro Val Pro Pro Ala Ala Ser Val Pro Asp
           165                 170                 175
   gcc atc ggc ggc gga ctc gga cgg gcg gcc gaa gtg cgg gcg gcc gaa      692
   Ala Ile Gly Gly Gly Leu Gly Arg Ala Ala Glu Val Arg Ala Ala Glu
       180                 185                 190
   ttg ctg gcg ggc gcg aag cgg ccg ctc atc ctt gcc ggc cgc ggt gcg      740
   Leu Leu Ala Gly Ala Lys Arg Pro Leu Ile Leu Ala Gly Arg Gly Ala
   195                 200                 205                 210
   cac ctc gca gga gcc ggc ccc gaa ctc cgg gaa ctc gcc gac cgc ctc      788
   His Leu Ala Gly Ala Gly Pro Glu Leu Arg Glu Leu Ala Asp Arg Leu
                   215                 220                 225
   ggc gcg ctc acg gcc ggc acc gca ctg gcg ctg aac ctg ctg cag ggc      836
   Gly Ala Leu Thr Ala Gly Thr Ala Leu Ala Leu Asn Leu Leu Gln Gly
               230                 235                 240
   gag ggg tac ctc ggc gtc gcg ggc ggc ttc ggc acg gat acc gcc gcc      884
   Glu Gly Tyr Leu Gly Val Ala Gly Gly Phe Gly Thr Asp Thr Ala Ala
           245                 250                 255
   ggg ctc atg ggc gag gcg gac gtg gtg ctc gtg gcg gga gcc agc ctg      932
   Gly Leu Met Gly Glu Ala Asp Val Val Leu Val Ala Gly Ala Ser Leu
       260                 265                 270
   acc ccc ttc acc atg cgc ttc ggc cac ctg atc ggc ccg gac gcc acc      980
   Thr Pro Phe Thr Met Arg Phe Gly His Leu Ile Gly Pro Asp Ala Thr
   275                 280                 285                 290
   gtg atc cag atc gac acc gcc atg gag ccg acg gac ccg cgg gtg gac     1028
   Val Ile Gln Ile Asp Thr Ala Met Glu Pro Thr Asp Pro Arg Val Asp
                   295                 300                 305
   ctg ttt gtc agt gcg gac gcg aag gcc gct gcc ggc cgg atc ctc cgg     1076
   Leu Phe Val Ser Ala Asp Ala Lys Ala Ala Ala Gly Arg Ile Leu Arg
               310                 315                 320
   ctg ctg gat gac gcc gcc ggg gcc aat gcg tcg aag gcc tgg cgc gcg     1124
   Leu Leu Asp Asp Ala Ala Gly Ala Asn Ala Ser Lys Ala Trp Arg Ala
           325                 330                 335
   gaa gca ctc aag cgt ctg gcc gaa gga ccc tgc cac cac ccc ggc acc     1172
   Glu Ala Leu Lys Arg Leu Ala Glu Gly Pro Cys His His Pro Gly Thr
       340                 345                 350
   gca gag acc acg gac ggc cgc ctt gac ccc cgg gcg ctt gct tcg gca     1220
   Ala Glu Thr Thr Asp Gly Arg Leu Asp Pro Arg Ala Leu Ala Ser Ala
   355                 360                 365                 370
   ctg gat gcc gtc ctg ccg gaa cgc cgc acc gtg gtc cag gac ggc ggg     1268
   Leu Asp Ala Val Leu Pro Glu Arg Arg Thr Val Val Gln Asp Gly Gly
                   375                 380                 385
   cac ttc ctg ggc tgg gca ccc atg tac tgg cgc atc ccc cgt cct cag    1316
   His Phe Leu Gly Trp Ala Pro Met Tyr Trp Arg Ile Pro Arg Pro Gln
               390                 395                 400
   gac ctg gtc atg gtg ggg acc gcg tac cag tcg atc ggg ctt ggc ctg    1364
   Asp Leu Val Met Val Gly Thr Ala Tyr Gln Ser Ile Gly Leu Gly Leu
           405                 410                 415
   gcc agc gcc gtg ggg gcg tcc cgg gcc gtg gac gac ggc aat atc ctg    1412
   Ala Ser Ala Val Gly Ala Ser Arg Ala Val Asp Asp Gly Asn Ile Leu
       420                 425                 430
   gtg ctg gcg gcg ggc gac ggc gga ttc ctg atg ggc ctg tcc gac ctg    1460
   Val Leu Ala Ala Gly Asp Gly Gly Phe Leu Met Gly Leu Ser Asp Leu
   435                 440                 445                 450
   gaa tcg ctc gtg ggc gcg gcg agc agc gcc gtc gtg gtg atc tac aac    1508
   Glu Ser Leu Val Gly Ala Ala Ser Ser Ala Val Val Val Ile Tyr Asn
                   455                 460                 465
   gat gcc gcc tac ggg gcc gag atc cat cag tac ggc tca cgg ggg ctc    1556
   Asp Ala Ala Tyr Gly Ala Glu Ile His Gln Tyr Gly Ser Arg Gly Leu
               470                 475                 480
   acc gaa aag ccc atg ctg atc ccc gaa gtg gac ttc agc ggg att gcc    1604
   Thr Glu Lys Pro Met Leu Ile Pro Glu Val Asp Phe Ser Gly Ile Ala
           485                 490                 495
   cgc gcg atc ggg gcg gaa tcc gca atc atc cgc aag ctg tcg gac ctc    1652
   Arg Ala Ile Gly Ala Glu Ser Ala Ile Ile Arg Lys Leu Ser Asp Leu
       500                 505                 510
   tcc gcg ctc acg gac tgg atc gag gcc ggc gcc agg gga acc ttc gtg    1700
   Ser Ala Leu Thr Asp Trp Ile Glu Ala Gly Ala Arg Gly Thr Phe Val
   515                 520                 525                 530
   gcc gac tgc cgc atc acc tca agc gtc cgg gcc ccg tgg ctg agc gaa    1748
   Ala Asp Cys Arg Ile Thr Ser Ser Val Arg Ala Pro Trp Leu Ser Glu
                   535                 540                 545
   tgg atg agg gcc tcg caa gcg gcg aag gag gcg gtg gcg ggc tag        1793
   Trp Met Arg Ala Ser Gln Ala Ala Lys Glu Ala Val Ala Gly
               550                 555                 560
   ggccggcctc gtcgaaatgc cgccctccaa cccaactcag taccagctca gggcgttctc 1853
   agggctggga acgccctgag ctgctactca gtttgttcga actcgagaat tcaatcgaat 1913
   tcccgcggcc gccatggcgg ccgggagcat gcgacgtcgg gcccattcg             1962
<210> 6
   <211> 541
   <212>
   <213> Séquence artificielle
   <223> Description de la séquence artificielle: mutant d'HPPO d'A. globiformis
<400> 6
<210> 7
   <211> 1692
   <212> ADN
   <213> Pseudomonas acidovorans
<220>
   <221> CDS
   <222> (1)..(1692)
<400> 7
   atg tcc cac ccc gcc atc tcc ctg caa gcg ctg cgc ggc agc ggc gca      48
   Met Ser His Pro Ala Ile Ser Leu Gln Ala Leu Arg Gly Ser Gly Ala
    1               5                  10                  15
   gac ata cag tcc atc cac atc ccc tac gag cgc cat gcc gac cag gac      96
   Asp Ile Gln Ser Ile His Ile Pro Tyr Glu Arg His Ala Asp Gln Asp
               20                  25                  30
   gcc ggt gcg gac acg ccc gcc cgg cat ccc gtc gtc atc gtc ggc gcc     144
   Ala Gly Ala Asp Thr Pro Ala Arg His Pro Val Val Ile Val Gly Ala
           35                  40                  45
   ggc ccc gtg ggc ctg tcg ctg gcc atc gac ctg gcc cag cgc ggc cag     192
   Gly Pro Val Gly Leu Ser Leu Ala Ile Asp Leu Ala Gln Arg Gly Gln
       50                  55                  60
   cgc gtg gtg ctg ctg gac aac gac tgc cgg ctg tcc acg ggc tcg cgc     240
   Arg Val Val Leu Leu Asp Asn Asp Cys Arg Leu Ser Thr Gly Ser Arg
   65                  70                  75                  80
   gcc atc tgc ttt tcc aag cgc acg ctg gag atc tgg gac cgc ctg ggc     288
   Ala Ile Cys Phe Ser Lys Arg Thr Leu Glu Ile Trp Asp Arg Leu Gly
                   85                  90                  95
   gtg ggc cag ccc atg gtg gac aag ggc gtg tcc tgg aac ctg ggc aag     336
   Val Gly Gln Pro Met Val Asp Lys Gly Val Ser Trp Asn Leu Gly Lys
               100                 105                 110
   gtc ttc ttc aag gac cag ccg ctg tac cgc ttc gac ctg ctg ccc gag     384
   Val Phe Phe Lys Asp Gln Pro Leu Tyr Arg Phe Asp Leu Leu Pro Glu
           115                 120                 125
   gac ggc cac gag cgc ccg gcc ttc atc aac ctg cag cag tac tac gcc      432
   Asp Gly His Glu Arg Pro Ala Phe Ile Asn Leu Gln Gln Tyr Tyr Ala
       130                 135                 140
   gag gcc tat ctg gtc gag cgc gca ctg cag ctg ccg ctg atc gac ctg      480
   Glu Ala Tyr Leu Val Glu Arg Ala Leu Gln Leu Pro Leu Ile Asp Leu
   145                 150                 155                 160
   cgc tgg cac agc aag gtc acg gca ctg gag ccg cag gcc gag ggc gcg      528
   Arg Trp His Ser Lys Val Thr Ala Leu Glu Pro Gln Ala Glu Gly Ala
                   165                 170                 175
   ctg ctg acc gtg gag acg cct gac ggc agc tac cgc atc gat gcg caa      576
   Leu Leu Thr Val Glu Thr Pro Asp Gly Ser Tyr Arg Ile Asp Ala Gln
               180                 185                 190
   tgg gtc ctg gcc tgc gat ggc tcg cgc tcg ccg ctg cgc ggc ctg ctg      624
   Trp Val Leu Ala Cys Asp Gly Ser Arg Ser Pro Leu Arg Gly Leu Leu
           195                 200                 205
   ggc cag gaa agc cat ggc cgc atc ttc cgc gac cgc ttc ctg atc gcc      672
   Gly Gln Glu Ser His Gly Arg Ile Phe Arg Asp Arg Phe Leu Ile Ala
       210                 215                 220
   gac gtg aag atg cac gcc gaa ttc ccc acc gag cgc tgg ttc tgg ttc      720
   Asp Val Lys Met His Ala Glu Phe Pro Thr Glu Arg Trp Phe Trp Phe
   225                 230                 235                 240
   gac ccg ccc ttc cac ccg ggc cag agc gtg ctg ctg cac cgc cag ccc      768
   Asp Pro Pro Phe His Pro Gly Gln Ser Val Leu Leu His Arg Gln Pro
                   245                 250                 255
   gac gat gtc tgg cgc atc gac ttc cag ctg ggc tgg gac gcg gac ccc      816
   Asp Asp Val Trp Arg Ile Asp Phe Gln Leu Gly Trp Asp Ala Asp Pro
               260                 265                 270
   gag gaa gag aaa aag ccc gag aac atc gtg ccg cgc atc cgc gcc ctg      864
   Glu Glu Glu Lys Lys Pro Glu Asn Ile Val Pro Arg Ile Arg Ala Leu
           275                 280                 285
   ctg ggc aag gac gcg ccc ttc gag ctg gaa tgg gcc agc gtc tac acc      912
   Leu Gly Lys Asp Ala Pro Phe Glu Leu Glu Trp Ala Ser Val Tyr Thr
       290                 295                 300
   ttc gcc tgc ctg cgc atg gac cgc ttc gtc cat ggc cgc gtg gtc ttt      960
   Phe Ala Cys Leu Arg Met Asp Arg Phe Val His Gly Arg Val Val Phe
   305                 310                 315                 320
   gcg ggc gac agc gcc cac ggc gtc tcg ccg ttt ggc gca cgc ggc gcc      1008
   Ala Gly Asp Ser Ala His Gly Val Ser Pro Phe Gly Ala Arg Gly Ala
                   325                 330                 335
   aac agc ggc gtg cag gat gcc gag aac ctg gca tgg aag ctg gac cgc      1056
   Asn Ser Gly Val Gln Asp Ala Glu Asn Leu Ala Trp Lys Leu Asp Arg
               340                 345                 350
   gtg ctg cgc ggc cag gcc gat gcc tcg ctg atc gcc acc tac ggc gcc      1104
   Val Leu Arg Gly Gln Ala Asp Ala Ser Leu Ile Ala Thr Tyr Gly Ala
           355                 360                 365
   gag cgc gaa tac gcg gcc gac gag aac atc cgc aac tcc acg cgc gcc      1152
   Glu Arg Glu Tyr Ala Ala Asp Glu Asn Ile Arg Asn Ser Thr Arg Ala
       370                 375                 380
   acc gac ttc atc acg ccc aag agc gag atc agc cgc ctg ttt cgc gac      1200
   Thr Asp Phe Ile Thr Pro Lys Ser Glu Ile Ser Arg Leu Phe Arg Asp
   385                 390                 395                 400
   gcc gtg ctg gac ctg gcg cgc gac cat gaa ttc gcg cgc cgc atc gtc    1248
   Ala Val Leu Asp Leu Ala Arg Asp His Glu Phe Ala Arg Arg Ile Val
                   405                 410                 415
   aac agc ggg cgg ctg tcc gtg ccg gcc acg ctg cac ggc tcc gcg ctc    1296
   Asn Ser Gly Arg Leu Ser Val Pro Ala Thr Leu His Gly Ser Ala Leu
               420                 425                 430
   aac acg cct gac acc gac acc ttc gac gga acg cag ctg ccc ggc gcc    1344
   Asn Thr Pro Asp Thr Asp Thr Phe Asp Gly Thr Gln Leu Pro Gly Ala
           435                 440                 445
   gtg ctg gcc gat gcg ccc atg cgc cgg ccc ggc gca gac ggc acg gcc    1392
   Val Leu Ala Asp Ala Pro Met Arg Arg Pro Gly Ala Asp Gly Thr Ala
       450                 455                 460
   tgg ctg ctg cgc gca ctg gga ccg gac ttc acg ctg ctg cac ttc gac    1440
   Trp Leu Leu Arg Ala Leu Gly Pro Asp Phe Thr Leu Leu His Phe Asp
   465                 470                 475                 480
   ccc acg ccc gcc tgg gcg cag gcg ctg ccc ggc gtg ctc aac ctg tcc    1488
   Pro Thr Pro Ala Trp Ala Gln Ala Leu Pro Gly Val Leu Asn Leu Ser
                   485                 490                 495
   atc gcg gcc gag ggc gag gcc cat gcg cca gac gcc gac ctc atc gat    1536
   Ile Ala Ala Glu Gly Glu Ala His Ala Pro Asp Ala Asp Leu Ile Asp
               500                 505                 510
   gcg cgc ggc ctg gcg gcc aaa cgc ctg gat gca cgc ccc ggc acc agc    1584
   Ala Arg Gly Leu Ala Ala Lys Arg Leu Asp Ala Arg Pro Gly Thr Ser
           515                 520                 525
   tac ctg ctg cgg cct gac cag cat gtc tgc gcg cgc tgg cgc cgc ccc    1632
   Tyr Leu Leu Arg Pro Asp Gln His Val Cys Ala Arg Trp Arg Arg Pro
       530                 535                 540
   gac gaa gcc agc gtg cgc gcc gcg ctg caa aga gcc tgc ggc gcc gcc    1680
   Asp Glu Ala Ser Val Arg Ala Ala Leu Gln Arg Ala Cys Gly Ala Ala
   545                 550                 555                 560
   gcc acg gcc tga                                                    1692
   Ala Thr Ala
<210> 8
   <211> 564
   <212>
   <213> Pseudomonas acidovorans
<400> 8
<210> 9
   <211> 966
   <212> ADN
   <213> Pseudomonas acidovorans
<220>
   <221> CDS
   <222> (1)..(966)
<400> 9
   atg acc acc aag acc ttt gcc tcc gcc gcc gac ctc gaa atc aag cag    48
   Met Thr Thr Lys Thr Phe Ala Ser Ala Ala Asp Leu Glu Ile Lys Gln
    1               5                  10                  15
   gtc agc ttc gac aag ctc tcc gag cac gcc tat gcc tac acg gcc gaa    96
   Val Ser Phe Asp Lys Leu Ser Glu His Ala Tyr Ala Tyr Thr Ala Glu
               20                  25                  30
   ggc gac ccc aac acc ggc atc atc att ggc gac gac gcg gtg atg gtg   144
   Gly Asp Pro Asn Thr Gly Ile Ile Ile Gly Asp Asp Ala Val Met Val
           35                  40                  45
   atc gac acc cag gcc acg ccc gtc atg gcc cag gac gtg atc cgc cgc   192
   Ile Asp Thr Gln Ala Thr Pro Val Met Ala Gln Asp Val Ile Arg Arg
       50                  55                  60
   atc cgt gag gtc acg gac aag ccc atc aag tac gtg acg ctg tcg cac   240
   Ile Arg Glu Val Thr Asp Lys Pro Ile Lys Tyr Val Thr Leu Ser His
   65                  70                  75                  80
   tac cac gcg gtg cgc gtg ctg ggc gcc tcg gcc ttc ttc gcg gaa ggc   288
   Tyr His Ala Val Arg Val Leu Gly Ala Ser Ala Phe Phe Ala Glu Gly
                   85                  90                  95
   gcc gaa cac atc att gcc agc cag gac acc tac gac ctc atc gtg gag   336
   Ala Glu His Ile Ile Ala Ser Gln Asp Thr Tyr Asp Leu Ile Val Glu
               100                 105                 110
   cgc ggc gag cag gac aag gcc agc gag atc ggc cgc ttt ccc cgc ctg   384
   Arg Gly Glu Gln Asp Lys Ala Ser Glu Ile Gly Arg Phe Pro Arg Leu
           115                 120                 125
   ttc cag aac gtg gaa agc gtg ccc gat ggc atg acc tgg ccc acc ctc   432
   Phe Gln Asn Val Glu Ser Val Pro Asp Gly Met Thr Trp Pro Thr Leu
       130                 135                 140
   acc ttc acc ggc aag atg acg ctg tgg ctg ggc aag ctg gaa gtg cag      480
   Thr Phe Thr Gly Lys Met Thr Leu Trp Leu Gly Lys Leu Glu Val Gln
   145                 150                 155                 160
   atc ctg cag ctg ggc cgc ggc cac acc aag ggc gac acc gtg gtc tgg      528
   Ile Leu Gln Leu Gly Arg Gly His Thr Lys Gly Asp Thr Val Val Trp
                   165                 170                 175
   ctg ccc cag gac aag gtg ctg ttc agc ggc gac ctg gtg gag ttc ggc      576
   Leu Pro Gln Asp Lys Val Leu Phe Ser Gly Asp Leu Val Glu Phe Gly
               180                 185                 190
   gcc acg ccc tat gcg ggc gat gcc tac ttc cag gac tgg ccg cac acg      624
   Ala Thr Pro Tyr Ala Gly Asp Ala Tyr Phe Gln Asp Trp Pro His Thr
           195                 200                 205
   ctg gac gcc atc gcc gcc ctg cag ccc gaa aag ctc gtg ccc ggc cgg      672
   Leu Asp Ala Ile Ala Ala Leu Gln Pro Glu Lys Leu Val Pro Gly Arg
       210                 215                 220
   ggc gcc gcg ctg cag acg ccg gcc gag gtg cag gcc ggc ctg gcc ggc      720
   Gly Ala Ala Leu Gln Thr Pro Ala Glu Val Gln Ala Gly Leu Ala Gly
   225                 230                 235                 240
   acg cgc gac ttc atc agc gac ctg tgg acc gag gtc aag gcc ggc gcc      768
   Thr Arg Asp Phe Ile Ser Asp Leu Trp Thr Glu Val Lys Ala Gly Ala
                   245                 250                 255
   gat gcc cag cag gac ctg cgc aag gtc tac gag gcc gcc ttc gcc aag      816
   Asp Ala Gln Gln Asp Leu Arg Lys Val Tyr Glu Ala Ala Phe Ala Lys
               260                 265                 270
   ctg cag ccc aag tac ggc cag tgg gtg atc ttc aac cac tgc atg ccc      864
   Leu Gln Pro Lys Tyr Gly Gln Trp Val Ile Phe Asn His Cys Met Pro
           275                 280                 285
   ttc gat gtg acc cgc gcc tat gac gag gca tcg ggc cac gcc gac cca      912
   Phe Asp Val Thr Arg Ala Tyr Asp Glu Ala Ser Gly His Ala Asp Pro
       290                 295                 300
   cgc atc tgg acc gcc gag cgc gac cgc cag atg tgg ctg gcg ctc gaa      960
   Arg Ile Trp Thr Ala Glu Arg Asp Arg Gln Met Trp Leu Ala Leu Glu
   305                 310                 315                 320
   ggc tga                                                              966
   Gly
<210> 10
   <211> 322
   <212>
   <213> Pseudomonas acidovorans
<400> 10
<210> 11
   <211> 966
   <212> ADN
   <213> Séquence artificielle
<220>
   <223> Description de la séquence artificielle: mutant d'HPAC de P. acidovorans
<220>
   <221> CDS
   <222> (1)..(966)
<400> 11
   atg tcc acc aag acc ttt gcc tcc gcc gcc gac ctc gaa atc aag cag 48
   Met Ser Thr Lys Thr Phe Ala Ser Ala Ala Asp Leu Glu Ile Lys Gln
   1 5 10 15
   gtc agc ttc gac aag ctc tcc gag cac gcc tat gcc tac acg gcc gaa 96
   Val Ser Phe Asp Lys Leu Ser Glu His Ala Tyr Ala Tyr Thr Ala Glu
   20 25 30
   ggc gac ccc aac acc ggc atc atc att ggc gac gac gcg gtg atg gtg 144
   Gly Asp Pro Asn Thr Gly Ile Ile Ile Gly Asp Asp Ala Val Met Val
   35 40 45
   atc gac acc cag gcc acg ccc gtc atg gcc cag gac gtg atc cgc cgc 192
   Ile Asp Thr Gln Ala Thr Pro Val Met Ala Gln Asp Val Ile Arg Arg
   50 55 60
   atc cgt gag gtc acg gac aag ccc atc aag tac gtg acg ctg tcg cac 240
   Ile Arg Glu Val Thr Asp Lys Pro Ile Lys Tyr Val Thr Leu Ser His
   65 70 75 80
   tac cac gcg gtg cgc gtg ctg ggc gcc tcg gcc ttc ttc gcg gaa ggc 288
   Tyr His Ala Val Arg Val Leu Gly Ala Ser Ala Phe Phe Ala Glu Gly
   85 90 95
   gcc gaa cac atc att gcc agc cag gac acc tac gac ctc atc gtg gag 336
   Ala Glu His Ile Ile Ala Ser Gln Asp Thr Tyr Asp Leu Ile Val Glu
   100 105 110
   cgc ggc gag cag gac aag gcc agc gag atc ggc cgc ttt ccc cgc ctg 384
   Arg Gly Glu Gln Asp Lys Ala Ser Glu Ile Gly Arg Phe Pro Arg Leu
   115 120 125
   ttc cag aac gtg gaa agc gtg ccc gat ggc atg acc tgg ccc acc ctc 432
   Phe Gln Asn Val Glu Ser Val Pro Asp Gly Met Thr Trp Pro Thr Leu
   130 135 140
   acc ttc acc ggc aag atg acg ctg tgg ctg ggc aag ctg gaa gtg cag 480
   Thr Phe Thr Gly Lys Met Thr Leu Trp Leu Gly Lys Leu Glu Val Gln
   145 150 155 160
   atc ctg cag ctg ggc cgc ggc cac acc aag ggc gac acc gtg gtc tgg 528
   Ile Leu Gln Leu Gly Arg Gly His Thr Lys Gly Asp Thr Val Val Trp
   165 170 175
   ctg ccc cag gac aag gtg ctg ttc agc ggc gac ctg gtg gag ttc ggc 576
   Leu Pro Gln Asp Lys Val Leu Phe Ser Gly Asp Leu Val Glu Phe Gly
   180 185 190
   gcc acg ccc tat gcg ggc gat gcc tac ttc cag gac tgg ccg cac acg 624
   Ala Thr Pro Tyr Ala Gly Asp Ala Tyr Phe Gln Asp Trp Pro His Thr
   195 200 205
   ctg gac acc atc gcc gcc ctg cag ccc gaa aag ctc gtg ccc ggc cgg 672
   Leu Asp Thr Ile Ala Ala Leu Gln Pro Glu Lys Leu Val Pro Gly Arg
   210 215 220
   ggc gcc gcg ctg cag acg ccg gcc gag gtg cag gcc ggc ctg gcc ggc 720
   Gly Ala Ala Leu Gln Thr Pro Ala Glu Val Gln Ala Gly Leu Ala Gly
   225 230 235 240
   acg cgc gac ttc atc agc gac ctg tgg acc gag gtc aag gcc ggc gcc 768
   Thr Arg Asp Phe Ile Ser Asp Leu Trp Thr Glu Val Lys Ala Gly Ala
   245 250 255
   gat gcc cag cag gac ctg cgc aag gtc tac gag gcc gcc ttc gcc aag 816
   Asp Ala Gln Gln Asp Leu Arg Lys Val Tyr Glu Ala Ala Phe Ala Lys
   260 265 270
   ctg cag ccc aag tac ggc cag tgg gtg atc ttc aac cac tgc atg ccc 864
   Leu Gln Pro Lys Tyr Gly Gln Trp Val Ile Phe Asn His Cys Met Pro
           275                 280                 285
   ttc gat gtg acc cgc gcc tat gac gag gca tcg ggc cac gcc gac cca      912
   Phe Asp Val Thr Arg Ala Tyr Asp Glu Ala Ser Gly His Ala Asp Pro
       290                 295                 300
   cgc atc tgg acc gcc gag cgc gac cgc cag atg tgg ctg gcg ctc gaa      960
   Arg Ile Trp Thr Ala Glu Arg Asp Arg Gln Met Trp Leu Ala Leu Glu
   305                 310                 315                 320
   ggc tga                                                              966
   Gly
<210> 12
   <211> 322
   <212>
   <213> Séquence artificielle
   <223> Description de la séquence artificielle: mutant d'HPAC de P. acidovorans
<400> 12
<210> 13
   <211> 966
   <212> ADN
   <213> Séquence artificielle
<220>
   <223> Description de la séquence artificielle: mutant d'HPAC de P. acidovorans
<220>
   <221> CDS
   <222> (1)..(966)
<400> 13
   atg tcc acc aag acc ttt gcc tcc gcc gcc gac ctc gaa atc aag cag      48
   Met Ser Thr Lys Thr Phe Ala Ser Ala Ala Asp Leu Glu Ile Lys Gln
    1               5                  10                  15
   gtc agc ttc gac aag ctc tcc gag cac gcc tat gcc tac acg gcc gaa      96
   Val Ser Phe Asp Lys Leu Ser Glu His Ala Tyr Ala Tyr Thr Ala Glu
               20                  25                  30
   ggc gac ccc aac acc ggc atc atc att ggc gac gac gcg gtg atg gtg     144
   Gly Asp Pro Asn Thr Gly Ile Ile Ile Gly Asp Asp Ala Val Met Val
           35                  40                  45
   atc gac acc cag gcc acg ccc gtc atg gcc cag gac gtg atc cgc cgc     192
   Ile Asp Thr Gln Ala Thr Pro Val Met Ala Gln Asp Val Ile Arg Arg
       50                  55                  60
   atc cgt gag gtc acg gac aag ccc atc aag tac gtg acg ctg tcg cac     240
   Ile Arg Glu Val Thr Asp Lys Pro Ile Lys Tyr Val Thr Leu Ser His
   65                  70                  75                  80
   tac cac gcg gtg cgc gtg ctg ggc gcc tcg gcc ttc ttc gcg gaa ggc     288
   Tyr His Ala Val Arg Val Leu Gly Ala Ser Ala Phe Phe Ala Glu Gly
                   85                  90                  95
   gcc gaa cac atc att gcc agc cag gac acc tac gac ctc atc gtg gag     336
   Ala Glu His Ile Ile Ala Ser Gln Asp Thr Tyr Asp Leu Ile Val Glu
               100                 105                 110
   cgc ggc gag cag gac aag gcc agc gag atc ggc cgc ttt ccc cgc ctg     384
   Arg Gly Glu Gln Asp Lys Ala Ser Glu Ile Gly Arg Phe Pro Arg Leu
           115                 120                 125
   ttc cag aac gtg gaa agc gtg ccc gat ggc atg acc tgg ccc acc ctc     432
   Phe Gln Asn Val Glu Ser Val Pro Asp Gly Met Thr Trp Pro Thr Leu
       130                 135                 140
   acc ttc acc ggc aag atg acg ctg tgg ctg ggc aag ctg gaa gtg cag     480
   Thr Phe Thr Gly Lys Met Thr Leu Trp Leu Gly Lys Leu Glu Val Gln
   145                 150                 155                 160
   atc ctg cag ctg ggc cgc ggc cac acc aag ggc gac acc gtg gtc tgg      528
   Ile Leu Gln Leu Gly Arg Gly His Thr Lys Gly Asp Thr Val Val Trp
                   165                 170                 175
   ctg ccc cag gac aag gtg ctg ttc agc ggc gac ctg gtg gag ttc ggc      576
   Leu Pro Gln Asp Lys Val Leu Phe Ser Gly Asp Leu Val Glu Phe Gly
               180                 185                 190
   gcc acg ccc tat gcg ggc gat gcc tac ttc cag gac tgg ccg cac acg      624
   Ala Thr Pro Tyr Ala Gly Asp Ala Tyr Phe Gln Asp Trp Pro His Thr
           195                 200                 205
   ctg gac gcc atc gcc gcc ctg cag ccc gaa aag ctc gtg ccc ggc cgg      672
   Leu Asp Ala Ile Ala Ala Leu Gln Pro Glu Lys Leu Val Pro Gly Arg
       210                 215                 220
   ggc gcc gcg ctg cag acg ccg gcc gag gtg cag gcc ggc ctg gcc ggc      720
   Gly Ala Ala Leu Gln Thr Pro Ala Glu Val Gln Ala Gly Leu Ala Gly
   225                 230                 235                 240
   acg cgc gac ttc atc agc gac ctg tgg acc gag gtc aag gcc ggc gcc      768
   Thr Arg Asp Phe Ile Ser Asp Leu Trp Thr Glu Val Lys Ala Gly Ala
                   245                 250                 255
   gat gcc cag cag gac ctg cgc aag gtc tac gag gcc gcc ttc gcc aag      816
   Asp Ala Gln Gln Asp Leu Arg Lys Val Tyr Glu Ala Ala Phe Ala Lys
               260                 265                 270
   ctg cag ccc aag tac ggc cag tgg gtg atc ttc aac cac tgc atg ccc      864
   Leu Gln Pro Lys Tyr Gly Gln Trp Val Ile Phe Asn His Cys Met Pro
           275                 280                 285
   ttc gat gtg acc cgc gcc tat gac gag gca tcg ggc cac gcc gac cca      912
   Phe Asp Val Thr Arg Ala Tyr Asp Glu Ala Ser Gly His Ala Asp Pro
       290                 295                 300
   cgc atc tgg acc gcc gag cgc gac cgc cag atg tgg ctg gcg ctc gaa      960
   Arg Ile Trp Thr Ala Glu Arg Asp Arg Gln Met Trp Leu Ala Leu Glu
   305                 310                 315                 320
   ggc tga                                                              966
   Gly
<210> 14
   <211> 322
   <212>
   <213> Séquence artificielle
   <223> Description de la séquence artificielle: mutant d'HPAC de P. acidovorans
<400> 14
<210> 15
   <211> 3549
   <212> ADN
   <213> Séquence artificielle
<220>
   <223> Description de la séquence artificielle: cassette d'expression
<220>
   <221> promoter
   <222> (1)..(928)
<220>
   <221> CDS
   <222> (965)..(2647)
<220>
   <221> terminator
   <222> (2811)..(3549)
<400> 15
   tgcatgccta ggtcgaggag aaatatgagt cgaggcatgg atacactaag ttcccctgaa 60
   gtgagcatga tctttgatgc tgagatgatt cccagagcaa gatagtttgt gctgcaagtg 120
   acacaattgt aatgaaacca ccactcaacg aatttacttg tggctttgac atgtcgtgtg 180
   ctctgtttgt atttgtgagt gccggttggt aattattttt gttaatgtga ttttaaaacc 240
   tcttatgtaa atagttactt tatctattga agtgtgttct tgtggtctat agtttctcaa 300
   agggaaatta aaatgttgac atcccattta caattgataa cttggtatac acaaactttg 360
   taaatttggt gatatttatg gtcgaaagaa ggcaataccc attgtatgtt ccaatatcaa 420
   tatcaatacg ataacttgat aatactaaca tatgattgtc attgtttttc cagtatcaat 480
   atacattaag ctactacaaa attagtataa atcactatat tataaatctt tttcggttgt 540
   aacttgtaat tcgtgggttt ttaaaataaa agcatgtgaa aattttcaaa taatgtgatg 600
   gcgcaatttt attttccgag ttccaaaata ttgccgcttc attaccctaa tttgtggcgc 660
   cacatgtaaa acaaaagacg attcttagtg gctatcactg ccatcacgcg gatcactaat 720
   atgaaccgtc gattaaaaca gatcgacggt ttatacatca ttttattgta cacacggatc 780
   gatatctcag ccgttagatt taatatgcga tctgattgct caaaaaatag actctccgtc 840
   tttgcctata aaaacaattt cacatctttc tcacccaaat ctactcttaa ccgttcttct 900
   tcttctacag acatcaattt ctctcgactc tagaggatcc aagcttatcg atttcgaacc 960
   cctc atg act tca ctt aca gtg tcc ggc cgg gtg gcg cag gtc ctc agc 1009
   Met Thr Ser Leu Thr Val Ser Gly Arg Val Ala Gln Val Leu Ser
   1 5 10 15
   agc tat gtc agc gat gtg ttc ggt gtg atg ggc aac gga aac gtc tac 1057
   Ser Tyr Val Ser Asp Val Phe Gly Val Met Gly Asn Gly Asn Val Tyr
   20 25 30
   ttc ctg gac gcc gcc gag aag gag ggc ctc cgc ttc acg gcc gta cgc 1105
   Phe Leu Asp Ala Ala Glu Lys Glu Gly Leu Arg Phe Thr Ala Val Arg
   35 40 45
   cat gaa ggt gcc gcc atc gcg gcg gcg gac gcc tac tat cgg gca tcc 1153
   His Glu Gly Ala Ala Ile Ala Ala Ala Asp Ala Tyr Tyr Arg Ala Ser
   50 55 60
   ggg cgc ctg gcg gcg ggg acc acc acc tac ggc ccc ggt tac acc aac 1201
   Gly Arg Leu Ala Ala Gly Thr Thr Thr Tyr Gly Pro Gly Tyr Thr Asn
   65 70 75
   gcc ctg acg gcc ctc gcc gag gcg gtc cag gcg cag atc ccc gtg gtg 1249
   Ala Leu Thr Ala Leu Ala Glu Ala Val Gln Ala Gln Ile Pro Val Val
   80 85 90 95
   ctc gtc acc ggg gac gcc ccg agc agc ggc gcc cgg cct tgg gac gtg 1297
   Leu Val Thr Gly Asp Ala Pro Ser Ser Gly Ala Arg Pro Trp Asp Val
   100 105 110
   gac cag gcc gcg atc gcc gcc ggg ctg ggg gcg gcg acc ttc acg gtc 1345
   Asp Gln Ala Ala Ile Ala Ala Gly Leu Gly Ala Ala Thr Phe Thr Val
   115 120 125
   acc cgt gaa gcc gca ggc tcc atc acg cag gaa gcg gtg gag tac gca    1393
   Thr Arg Glu Ala Ala Gly Ser Ile Thr Gln Glu Ala Val Glu Tyr Ala
           130                 135                 140
   ctt gcc cgg cgg acc gcc gtc gtg atc gcc gtt cca tac gac ctg tcg    1441
   Leu Ala Arg Arg Thr Ala Val Val Ile Ala Val Pro Tyr Asp Leu Ser
       145                 150                 155
   gcc ctt gag gcg gcg gag gaa gat ctt ccc gtg ccg ccg gcg gcc tcg    1489
   Ala Leu Glu Ala Ala Glu Glu Asp Leu Pro Val Pro Pro Ala Ala Ser
   160                 165                 170                 175
   gtt ccg gac gcc atc ggc ggc gga ctc gga cgg gcg gcc gaa gtg cgg    1537
   Val Pro Asp Ala Ile Gly Gly Gly Leu Gly Arg Ala Ala Glu Val Arg
                   180                 185                 190
   gcg gcc gaa ttg ctg gcg ggc gcg aag cgg ccg ctc atc ctt gcc ggc    1585
   Ala Ala Glu Leu Leu Ala Gly Ala Lys Arg Pro Leu Ile Leu Ala Gly
               195                 200                 205
   cgc ggt gcg cac ctc gca gga acc ggc ccc gaa ctc cgg gaa ctc gcc    1633
   Arg Gly Ala His Leu Ala Gly Thr Gly Pro Glu Leu Arg Glu Leu Ala
           210                 215                 220
   gac cgc ctc ggc gcg ctc acg gcc ggc acc gca ctg gcg ctg aac ctg    1681
   Asp Arg Leu Gly Ala Leu Thr Ala Gly Thr Ala Leu Ala Leu Asn Leu
       225                 230                 235
   ctg cag ggc gag ggg tac ctc ggc gtc gcg ggc ggc ttc ggc acg gat    1729
   Leu Gln Gly Glu Gly Tyr Leu Gly Val Ala Gly Gly Phe Gly Thr Asp
   240                 245                 250                 255
   acc gcc gcc ggg ctc atg ggc gag gcg gac gtg gtg ctc gtg gcg gga    1777
   Thr Ala Ala Gly Leu Met Gly Glu Ala Asp Val Val Leu Val Ala Gly
                   260                 265                 270
   gcc agc ctg acc ccc ttc acc atg cgc ttc ggc cac ctg atc ggc ccg    1825
   Ala Ser Leu Thr Pro Phe Thr Met Arg Phe Gly His Leu Ile Gly Pro
               275                 280                 285
   gac gcc acc gtg atc cag atc gac acc gcc atg gag ccg acg gac ccg    1873
   Asp Ala Thr Val Ile Gln Ile Asp Thr Ala Met Glu Pro Thr Asp Pro
           290                 295                 300
   cgg gtg gac ctg ttt gtc agt gcg gac gcg aag gcc gct gcc ggc cgg    1921
   Arg Val Asp Leu Phe Val Ser Ala Asp Ala Lys Ala Ala Ala Gly Arg
       305                 310                 315
   atc ctc cgg ctg ctg gat gac gcc gcc ggg gcc aat gcg tcg aag gcc    1969
   Ile Leu Arg Leu Leu Asp Asp Ala Ala Gly Ala Asn Ala Ser Lys Ala
   320                 325                 330                 335
   tgg cgc gcg gaa gca ctc aag cgt ctg gcc gaa gga ccc tgc cac cac    2017
   Trp Arg Ala Glu Ala Leu Lys Arg Leu Ala Glu Gly Pro Cys His His
                   340                 345                 350
   ccc ggc acc gca gag acc acg gac ggc cgc ctt gac ccc cgg gcg ctt    2065
   Pro Gly Thr Ala Glu Thr Thr Asp Gly Arg Leu Asp Pro Arg Ala Leu
               355                 360                 365
   gct tcg gca ctg gat gcc gtc ctg ccg gaa cgc cgc acc gtg gtc cag    2113
   Ala Ser Ala Leu Asp Ala Val Leu Pro Glu Arg Arg Thr Val Val Gln
           370                 375                 380
   gac ggc ggg cac ttc ctg ggc tgg gca ccc atg tac tgg cgc atc ccc    2161
   Asp Gly Gly His Phe Leu Gly Trp Ala Pro Met Tyr Trp Arg Ile Pro
       385                 390                 395
   cgt cct cag gac ctg gtc atg gtg ggg acc gcg tac cag tcg atc ggg    2209
   Arg Pro Gln Asp Leu Val Met Val Gly Thr Ala Tyr Gln Ser Ile Gly
   400                 405                 410                 415
   ctt ggc ctg gcc agc gcc gtg ggg gcg tcc cgg gcc gtg gac gac ggc    2257
   Leu Gly Leu Ala Ser Ala Val Gly Ala Ser Arg Ala Val Asp Asp Gly
                   420                 425                 430
   aat atc ctg gtg ctg gcg gcg ggc gac ggc gga ttc ctg atg ggc ctg    2305
   Asn Ile Leu Val Leu Ala Ala Gly Asp Gly Gly Phe Leu Met Gly Leu
               435                 440                 445
   tcc gac ctg gaa tcg ctc gtg ggc gcg gcg agc agc gcc gtc gtg gtg    2353
   Ser Asp Leu Glu Ser Leu Val Gly Ala Ala Ser Ser Ala Val Val Val
           450                 455                 460
   atc tac aac gac gcc gcc tac ggg gcc gag atc cat cag tac ggc tca    2401
   Ile Tyr Asn Asp Ala Ala Tyr Gly Ala Glu Ile His Gln Tyr Gly Ser
       465                 470                 475
   cgg ggg ctc acc gaa aag ccc atg ctg atc ccc gaa gtg gac ttc agc    2449
   Arg Gly Leu Thr Glu Lys Pro Met Leu Ile Pro Glu Val Asp Phe Ser
   480                 485                 490                 495
   ggg att gcc cgc gcg atc ggg gcg gaa tcc gca atc atc cgc aag ctg    2497
   Gly Ile Ala Arg Ala Ile Gly Ala Glu Ser Ala Ile Ile Arg Lys Leu
                   500                 505                 510
   tcg gac ctc tcc gcg ctc acg gac tgg atc gag gcc ggc gcc agg gga    2545
   Ser Asp Leu Ser Ala Leu Thr Asp Trp Ile Glu Ala Gly Ala Arg Gly
               515                 520                 525
   acc ttc gtg gcc gac tgc cgc atc acc tca agc gtc cgg gcc ccg tgg    2593
   Thr Phe Val Ala Asp Cys Arg Ile Thr Ser Ser Val Arg Ala Pro Trp
           530                 535                 540
   ctg agc gaa tgg atg agg gcc tcg caa gcg gcg aag gag gcg gtg gcg    2641
   Leu Ser Glu Trp Met Arg Ala Ser Gln Ala Ala Lys Glu Ala Val Ala
       545                 550                 555
   ggc tag ggccggcctc gtcgaaatgc cgccctccaa cccaactcag taccagctca     2697
   Gly
   560
   gggcgttctc agggctggga acgccctgag ctgctactca tttgttcgaa ctcgaggtcg 2757
   acggtatcga taagcttgat atcgaattcc tgcagcccgg gggatccact aggggatccc 2817
   ccgatccgcg tttgtgtttt ctgggtttct cacttaagcg tctgcgtttt acttttgtat 2877
   tgggtttggc gtttagtagt ttgcggtagc gttcttgtta tgtgtaatta cgctttttct 2937
   tcttgcttca gcagtttcgg ttgaaatata aatcgaatca agtttcactt tatcagcgtt 2997
   gttttaaatt ttggcattaa attggtgaaa attgcttcaa ttttgtatct aaatagaaga 3057
   gacaacatga aattcgactt ttgacctcaa atcttcgaac atttatttcc tgatttcacg 3117
   atggatgagg ataacgaaag ggcggttcct atgtccggga aagttcccgt agaagacaat 3177
   gagcaaagct actgaaacgc ggacacgacg tcgcattggt acggatatga gttaaaccga 3237
   ctcaattcct ttattaagac ataaaccgat tttggttaaa gtgtaacagt gagctgatat 3297
   aaaaccgaaa caaaccggta caagtttgat tgagcaactt gatgacaaac ttcagaattt 3357
   tggttattga atgaaaatca tagtctaatc gtaaaaaatg tacagaagaa aagctagagc 3417
   agaacaaaga ttctatattc tggttccaat ttatcatcgc tttaacgtcc ctcagatttg 3477
   atcgggctgc aggaattcgg cctgactgat catttaaaca ctagttctag agcggccgcc 3537
   accgcggtgg ag                                                     3549
<210> 16
   <211> 241
   <212>
   <213> Séquence artificielle
   <223> Description de la séquence artificielle: cassette d'expression
<400> 16
<210> 17
   <211> 2838
   <212> ADN
   <213> Séquence artificielle
<220>
   <223> Description de la séquence artificielle: cassette d'expression
<220>
   <221> promoter
   <222> (1)..(807)
<220>
   <221> CDS
   <222> (847)..(2538)
<220>
   <221> terminator
   <222> (2539)..(2838)
<400> 17
   ggccccccct cgagcccaca gatggttaga gaggcttacg cagcaggtct catcaagacg 60
   atctacccga gcaataatct ccaggaaatc aaataccttc ccaagaaggt taaagatgca 120
   gtcaaaagat tcaggactaa ctgcatcaag aacacagaga aagatatatt tctcaagatc 180
   agaagtacta ttccagtatg gacgattcaa ggcttgcttc acaaaccaag gcaagtaata 240
   gagattggag tctctaaaaa ggtagttccc actgaatcaa aggccatgga gtcaaagatt 300
   caaatagagg acctaacaga actcgccgta aagactggcg aacagttcat acagagtctc 360
   ttacgactca atgacaagaa gaaaatcttc gtcaacatgg tggagcacga cacacttgtc 420
   tactccaaaa atatcaaaga tacagtctca gaagaccaaa gggcaattga gacttttcaa 480
   caaagggtaa tatccggaaa cctcctcgga ttccattgcc cagctatctg tcactttatt 540
   gtgaagatag tggaaaagga aggtggctcc tacaaatgcc atcattgcga taaaggaaag 600
   gccatcgttg aagatgcctc tgccgacagt ggtcccaaag atggaccccc acccacgagg 660
   agcatcgtgg aaaaagaaga cgttccaacc acgtcttcaa agcaagtgga ttgatgtgat 720
   atctccactg acgtaaggga tgacgcacaa tcccactatc cttcgcaaga cccttcctct 780
   atataaggaa gttcatttca tttggagaga acacggggga ctctagagga tcagaggacg 840
   aacaac atg tcc cac ccc gcc atc tcc ctg caa gcg ctg cgc ggc agc    888
          Met Ser His Pro Ala Ile Ser Leu Gln Ala Leu Arg Gly Ser
           1               5                  10
   ggc gca gac ata cag tcc atc cac atc ccc tac gag cgc cat gcc gac   936
   Gly Ala Asp Ile Gln Ser Ile His Ile Pro Tyr Glu Arg His Ala Asp
    15                  20                  25                  30
   cag gac gcc ggt gcg gac acg ccc gcc cgg cat ccc gtc gtc atc gtc   984
   Gln Asp Ala Gly Ala Asp Thr Pro Ala Arg His Pro Val Val Ile Val
                   35                  40                  45
   ggc gcc ggc ccc gtg ggc ctg tcg ctg gcc atc gac ctg gcc cag cgc   1032
   Gly Ala Gly Pro Val Gly Leu Ser Leu Ala Ile Asp Leu Ala Gln Arg
               50                  55                  60
   ggc cag cgc gtg gtg ctg ctg gac aac gac tgc cgg ctg tcc acg ggc   1080
   Gly Gln Arg Val Val Leu Leu Asp Asn Asp Cys Arg Leu Ser Thr Gly
           65                  70                  75
   tcg cgc gcc atc tgc ttt tcc aag cgc acg ctg gag atc tgg gac cgc   1128
   Ser Arg Ala Ile Cys Phe Ser Lys Arg Thr Leu Glu Ile Trp Asp Arg
       80                  85                  90
   ctg ggc gtg ggc cag ccc atg gtg gac aag ggc gtg tcc tgg aac ctg   1176
   Leu Gly Val Gly Gln Pro Met Val Asp Lys Gly Val Ser Trp Asn Leu
   95 100 105 110
   ggc aag gtc ttc ttc aag gac cag ccg ctg tac cgc ttc gac ctg ctg 1224
   Gly Lys Val Phe Phe Lys Asp Gln Pro Leu Tyr Arg Phe Asp Leu Leu
   115 120 125
   ccc gag gac ggc cac gag cgc ccg gcc ttc atc aac ctg cag cag tac 1272
   Pro Glu Asp Gly His Glu Arg Pro Ala Phe Ile Asn Leu Gln Gln Tyr
   130 135 140
   tac gcc gag gcc tat ctg gtc gag cgc gca ctg cag ctg ccg ctg atc 1320
   Tyr Ala Glu Ala Tyr Leu Val Glu Arg Ala Leu Gln Leu Pro Leu Ile
   145 150 155
   gac ctg cgc tgg cac agc aag gtc acg gca ctg gag ccg cag gcc gag 1368
   Asp Leu Arg Trp His Ser Lys Val Thr Ala Leu Glu Pro Gln Ala Glu
   160 165 170
   ggc gcg ctg ctg acc gtg gag acg cct gac ggc agc tac cgc atc gat 1416
   Gly Ala Leu Leu Thr Val Glu Thr Pro Asp Gly Ser Tyr Arg Ile Asp
   175 180 185 190
   gcg caa tgg gtc ctg gcc tgc gat ggc tcg cgc tcg ccg ctg cgc ggc 1464
   Ala Gln Trp Val Leu Ala Cys Asp Gly Ser Arg Ser Pro Leu Arg Gly
   195 200 205
   ctg ctg ggc cag gaa agc cat ggc cgc atc ttc cgc gac cgc ttc ctg 1512
   Leu Leu Gly Gln Glu Ser His Gly Arg Ile Phe Arg Asp Arg Phe Leu
   210 215 220
   atc gcc gac gtg aag atg cac gcc gaa ttc ccc acc gag cgc tgg ttc 1560
   Ile Ala Asp Val Lys Met His Ala Glu Phe Pro Thr Glu Arg Trp Phe
   225 230 235
   tgg ttc gac ccg ccc ttc cac ccg ggc cag agc gtg ctg ctg cac cgc 1608
   Trp Phe Asp Pro Pro Phe His Pro Gly Gln Ser Val Leu Leu His Arg
   240 245 250
   cag ccc gac gat gtc tgg cgc atc gac ttc cag ctg ggc tgg gac gcg 1656
   Gln Pro Asp Asp Val Trp Arg Ile Asp Phe Gln Leu Gly Trp Asp Ala
   255 260 265 270
   gac ccc gag gaa gag aaa aag ccc gag aac atc gtg ccg cgc atc cgc 1704
   Asp Pro Glu Glu Glu Lys Lys Pro Glu Asn Ile Val Pro Arg Ile Arg
   275 280 285
   gcc ctg ctg ggc aag gac gcg ccc ttc gag ctg gaa tgg gcc agc gtc 1752
   Ala Leu Leu Gly Lys Asp Ala Pro Phe Glu Leu Glu Trp Ala Ser Val
   290 295 300
   tac acc ttc gcc tgc ctg cgc atg gac cgc ttc gtc cat ggc cgc gtg 1800
   Tyr Thr Phe Ala Cys Leu Arg Met Asp Arg Phe Val His Gly Arg Val
   305 310 315
   gtc ttt gcg ggc gac agc gcc cac ggc gtc tcg ccg ttt ggc gca cgc 1848
   Val Phe Ala Gly Asp Ser Ala His Gly Val Ser Pro Phe Gly Ala Arg
   320 325 330
   ggc gcc aac agc ggc gtg cag gat gcc gag aac ctg gca tgg aag ctg 1896
   Gly Ala Asn Ser Gly Val Gln Asp Ala Glu Asn Leu Ala Trp Lys Leu
   335 340 345 350
   gac cgc gtg ctg cgc ggc cag gcc gat gcc tcg ctg atc gcc acc tac 1944
   Asp Arg Val Leu Arg Gly Gln Ala Asp Ala Ser Leu Ile Ala Thr Tyr
   355 360 365
   ggc gcc gag cgc gaa tac gcg gcc gac gag aac atc cgc aac tcc acg 1992
   Gly Ala Glu Arg Glu Tyr Ala Ala Asp Glu Asn Ile Arg Asn Ser Thr
               370                 375                 380
   cgc gcc acc gac ttc atc acg ccc aag agc gag atc agc cgc ctg ttt   2040
   Arg Ala Thr Asp Phe Ile Thr Pro Lys Ser Glu Ile Ser Arg Leu Phe
           385                 390                 395
   cgc gac gcc gtg ctg gac ctg gcg cgc gac cat gaa ttc gcg cgc cgc   2088
   Arg Asp Ala Val Leu Asp Leu Ala Arg Asp His Glu Phe Ala Arg Arg
       400                 405                 410
   atc gtc aac agc ggg cgg ctg tcc gtg ccg gcc acg ctg cac ggc tcc   2136
   Ile Val Asn Ser Gly Arg Leu Ser Val Pro Ala Thr Leu His Gly Ser
   415                 420                 425                 430
   gcg ctc aac acg cct gac acc gac acc ttc gac gga acg cag ctg ccc   2184
   Ala Leu Asn Thr Pro Asp Thr Asp Thr Phe Asp Gly Thr Gln Leu Pro
                   435                 440                 445
   ggc gcc gtg ctg gcc gat gcg ccc atg cgc cgg ccc ggc gca gac ggc   2232
   Gly Ala Val Leu Ala Asp Ala Pro Met Arg Arg Pro Gly Ala Asp Gly
               450                 455                 460
   acg gcc tgg ctg ctg cgc gca ctg gga ccg gac ttc acg ctg ctg cac   2280
   Thr Ala Trp Leu Leu Arg Ala Leu Gly Pro Asp Phe Thr Leu Leu His
           465                 470                 475
   ttc gac ccc acg ccc gcc tgg gcg cag gcg ctg ccc ggc gtg ctc aac   2328
   Phe Asp Pro Thr Pro Ala Trp Ala Gln Ala Leu Pro Gly Val Leu Asn
       480                 485                 490
   ctg tcc atc gcg gcc gag ggc gag gcc cat gcg cca gac gcc gac ctc   2376
   Leu Ser Ile Ala Ala Glu Gly Glu Ala His Ala Pro Asp Ala Asp Leu
   495                 500                 505                 510
   atc gat gcg cgc ggc ctg gcg gcc aaa cgc ctg gat gca cgc ccc ggc   2424
   Ile Asp Ala Arg Gly Leu Ala Ala Lys Arg Leu Asp Ala Arg Pro Gly
                   515                 520                 525
   acc agc tac ctg ctg cgg cct gac cag cat gtc tgc gcg cgc tgg cgc   2472
   Thr Ser Tyr Leu Leu Arg Pro Asp Gln His Val Cys Ala Arg Trp Arg
               530                 535                 540
   cgc ccc gac gaa gcc agc gtg cgc gcc gcg ctg caa aga gcc tgc ggc   2520
   Arg Pro Asp Glu Ala Ser Val Arg Ala Ala Leu Gln Arg Ala Cys Gly
           545                 550                 555
   gcc gcc gcc acg gcc tga acctcttaag cttatcgata ccgtcgacga          2568
   Ala Ala Ala Thr Ala
       560
   atttccccga tcgttcaaac atttggcaat aaagtttctt aagattgaat cctgttgccg 2628
   gtcttgcgat gattatcata taatttctgt tgaattacgt taagcatgta ataattaaca 2688
   tgtaatgcat gacgttattt atgagatggg tttttatgat tagagtcccg caattataca 2748
   tttaatacgc gatagaaaac aaaatatagc gcgcaaacta ggataaatta tcgcgcgcgg 2808
   tgtcatctat gttcctaggt cgggaattgc                                  2838
<210> 18
   <211> 284
   <212>
   <213> Séquence artificielle
   <223> Description de la séquence artificielle: cassette d'expression
<400> 18
<210> 19
   <211> 1839
   <212> ADN
   <213> Séquence artificielle
<220>
   <223> Description de la séquence artificielle: cassette d'expression
<220>
   <221> promoter
   <222> (1)..(547)
<220>
   <221> CDS
   <222> (574)..(1539)
<220>
   <221> terminator
   <222> (1540)..(1839)
<400> 19
   ccccctcgag gtcgacggta ttgatcagct tccagaaggt aattatccaa gatgtagcat 60
   caagaatcca atgtttacgg gaaaaactat ggaagtatta tgtgagctca gcaagaagca 120
   gatcaatatg cggcacatat gcaacctatg ttcaaaaatg aagaatgtac agatacaaga 180
   tcctatactg ccagaatacg aagaagaata cgtagaaatt gaaaaagaag aaccaggcga 240
   agaaaagaat cttgaagacg taagcactga cgacaacaat gaaaagaaga agataaggtc 300
   ggtgattgtg aaagagacat agaggacaca tgtaaggtgg aaaatgtaag ggcggaaagt 360
   aaccttatca caaaggaatc ttatccccca ctacttatcc ttttatattt ttccgtgtca 420
   tttttgccct tgagttttcc tatataagga accaagttcg gcatttgtga aaacaagaaa 480
   aaatttggtg taagctattt tctttgaagt actgaggata caacttcaga gaaatttgta 540
   agtttgtaga tctgaattcg atgcaggatg cac atg tcc acc aag acc ttt gcc  594
                                       Met Ser Thr Lys Thr Phe Ala
                                       1               5
   tcc gcc gcc gac ctc gaa atc aag cag gtc agc ttc gac aag ctc tcc  642
   Ser Ala Ala Asp Leu Glu Ile Lys Gln Val Ser Phe Asp Lys Leu Ser
           10                  15                  20
   gag cac gcc tat gcc tac acg gcc gaa ggc gac ccc aac acc ggc atc  690
   Glu His Ala Tyr Ala Tyr Thr Ala Glu Gly Asp Pro Asn Thr Gly Ile
       25                  30                  35
   atc att ggc gac gac gcg gtg atg gtg atc gac acc cag gcc acg ccc  738
   Ile Ile Gly Asp Asp Ala Val Met Val Ile Asp Thr Gln Ala Thr Pro
   40                  45                  50                  55
   gtc atg gcc cag gac gtg atc cgc cgc atc cgt gag gtc acg gac aag  786
   Val Met Ala Gln Asp Val Ile Arg Arg Ile Arg Glu Val Thr Asp Lys
                   60                  65                  70
   ccc atc aag tac gtg acg ctg tcg cac tac cac gcg gtg cgc gtg ctg  834
   Pro Ile Lys Tyr Val Thr Leu Ser His Tyr His Ala Val Arg Val Leu
               75                  80                  85
   ggc gcc tcg gcc ttc ttc gcg gaa ggc gcc gaa cac atc att gcc agc  882
   Gly Ala Ser Ala Phe Phe Ala Glu Gly Ala Glu His Ile Ile Ala Ser
           90                  95                  100
   cag gac acc tac gac ctc atc gtg gag cgc ggc gag cag gac aag gcc  930
   Gln Asp Thr Tyr Asp Leu Ile Val Glu Arg Gly Glu Gln Asp Lys Ala
       105                 110                 115
   agc gag atc ggc cgc ttt ccc cgc ctg ttc cag aac gtg gaa agc gtg  978
   Ser Glu Ile Gly Arg Phe Pro Arg Leu Phe Gln Asn Val Glu Ser Val
   120                 125                 130                 135
   ccc gat ggc atg acc tgg ccc acc ctc acc ttc acc ggc aag atg acg  1026
   Pro Asp Gly Met Thr Trp Pro Thr Leu Thr Phe Thr Gly Lys Met Thr
                   140                 145                 150
   ctg tgg ctg ggc aag ctg gaa gtg cag atc ctg cag ctg ggc cgc ggc     1074
   Leu Trp Leu Gly Lys Leu Glu Val Gln Ile Leu Gln Leu Gly Arg Gly
               155                 160                 165
   cac acc aag ggc gac acc gtg gtc tgg ctg ccc cag gac aag gtg ctg     1122
   His Thr Lys Gly Asp Thr Val Val Trp Leu Pro Gln Asp Lys Val Leu
           170                 175                 180
   ttc agc ggc gac ctg gtg gag ttc ggc gcc acg ccc tat gcg ggc gat     1170
   Phe Ser Gly Asp Leu Val Glu Phe Gly Ala Thr Pro Tyr Ala Gly Asp
       185                 190                 195
   gcc tac ttc cag gac tgg ccg cac acg ctg gac gcc atc gcc gcc ctg     1218
   Ala Tyr Phe Gln Asp Trp Pro His Thr Leu Asp Ala Ile Ala Ala Leu
   200                 205                 210                 215
   cag ccc gaa aag ctc gtg ccc ggc cgg ggc gcc gcg ctg cag acg ccg     1266
   Gln Pro Glu Lys Leu Val Pro Gly Arg Gly Ala Ala Leu Gln Thr Pro
                   220                 225                 230
   gcc gag gtg cag gcc ggc ctg gcc ggc acg cgc gac ttc atc agc gac     1314
   Ala Glu Val Gln Ala Gly Leu Ala Gly Thr Arg Asp Phe Ile Ser Asp
               235                 240                 245
   ctg tgg acc gag gtc aag gcc ggc gcc gat gcc cag cag gac ctg cgc     1362
   Leu Trp Thr Glu Val Lys Ala Gly Ala Asp Ala Gln Gln Asp Leu Arg
           250                 255                 260
   aag gtc tac gag gcc gcc ttc gcc aag ctg cag ccc aag tac ggc cag     1410
   Lys Val Tyr Glu Ala Ala Phe Ala Lys Leu Gln Pro Lys Tyr Gly Gln
       265                 270                 275
   tgg gtg atc ttc aac cac tgc atg ccc ttc gat gtg acc cgc gcc tat     1458
   Trp Val Ile Phe Asn His Cys Met Pro Phe Asp Val Thr Arg Ala Tyr
   280                 285                 290                 295
   gac gag gca tcg ggc cac gcc gac cca cgc atc tgg acc gcc gag cgc     1506
   Asp Glu Ala Ser Gly His Ala Asp Pro Arg Ile Trp Thr Ala Glu Arg
                   300                 305                 310
   gac cgc cag atg tgg ctg gcg ctc gaa ggc tga tgcaagctta tcgataccgt 1559
   Asp Arg Gln Met Trp Leu Ala Leu Glu Gly
               315                 320
   cgacgaattt ccccgatcgt tcaaacattt ggcaataaag tttcttaaga ttgaatcctg 1619
   ttgccggtct tgcgatgatt atcatataat ttctgttgaa ttacgttaag catgtaataa 1679
   ttaacatgta atgcatgacg ttatttatga gatgggtttt tatgattaga gtcccgcaat 1739
   tatacattta atacgcgata gaaaacaaaa tatagcgcgc aaactaggat aaattatcgc 1799
   gcgcggtgtc atctatgtta ctagatcggg aattgcggcc                       1839
<210> 20
   <211> 142
   <212>
   <213> Séquence artificielle
   <223> Description de la séquence artificielle: cassette d'expression
<400> 20
<210> 21
   <211> 4677
   <212> ADN
   <213> Séquence artificielle
<220>
   <223> Description de la séquence artificielle: cassette d'expression
<400> 21
   ccccctcgag gtcgacggta ttgatcagct tccagaaggt aattatccaa gatgtagcat 60
   caagaatcca atgtttacgg gaaaaactat ggaagtatta tgtgagctca gcaagaagca 120
   gatcaatatg cggcacatat gcaacctatg ttcaaaaatg aagaatgtac agatacaaga 180
   tcctatactg ccagaatacg aagaagaata cgtagaaatt gaaaaagaag aaccaggcga 240
   agaaaagaat cttgaagacg taagcactga cgacaacaat gaaaagaaga agataaggtc 300
   ggtgattgtg aaagagacat agaggacaca tgtaaggtgg aaaatgtaag ggcggaaagt 360
   aaccttatca caaaggaatc ttatccccca ctacttatcc ttttatattt ttccgtgtca 420
   tttttgccct tgagttttcc tatataagga accaagttcg gcatttgtga aaacaagaaa 480
   aaatttggtg taagctattt tctttgaagt actgaggata caacttcaga gaaatttgta 540
   agtttgtaga tctgaattcg atgcaggatg cacatgtcca ccaagacctt tgcctccgcc 600
   gccgacctcg aaatcaagca ggtcagcttc gacaagctct ccgagcacgc ctatgcctac 660
   acggccgaag gcgaccccaa caccggcatc atcattggcg acgacgcggt gatggtgatc 720
   gacacccagg ccacgcccgt catggcccag gacgtgatcc gccgcatccg tgaggtcacg 780
   gacaagccca tcaagtacgt gacgctgtcg cactaccacg cggtgcgcgt gctgggcgcc 840
   tcggccttct tcgcggaagg cgccgaacac atcattgcca gccaggacac ctacgacctc 900
   atcgtggagc gcggcgagca ggacaaggcc agcgagatcg gccgctttcc ccgcctgttc 960
   cagaacgtgg aaagcgtgcc cgatggcatg acctggccca ccctcacctt caccggcaag 1020
   atgacgctgt ggctgggcaa gctggaagtg cagatcctgc agctgggccg cggccacacc 1080
   aagggcgaca ccgtggtctg gctgccccag gacaaggtgc tgttcagcgg cgacctggtg 1140
   gagttcggcg ccacgcccta tgcgggcgat gcctacttcc aggactggcc gcacacgctg 1200
   gacgccatcg ccgccctgca gcccgaaaag ctcgtgcccg gccggggcgc cgcgctgcag 1260
   acgccggccg aggtgcaggc cggcctggcc ggcacgcgcg acttcatcag cgacctgtgg 1320
   accgaggtca aggccggcgc cgatgcccag caggacctgc gcaaggtcta cgaggccgcc 1380
   ttcgccaagc tgcagcccaa gtacggccag tgggtgatct tcaaccactg catgcccttc 1440
   gatgtgaccc gcgcctatga cgaggcatcg ggccacgccg acccacgcat ctggaccgcc 1500
   gagcgcgacc gccagatgtg gctggcgctc gaaggctgat gcaagcttat cgataccgtc 1560
   gacgaatttc cccgatcgtt caaacatttg gcaataaagt ttcttaagat tgaatcctgt 1620
   tgccggtctt gcgatgatta tcatataatt tctgttgaat tacgttaagc atgtaataat 1680
   taacatgtaa tgcatgacgt tatttatgag atgggttttt atgattagag tcccgcaatt 1740
   atacatttaa tacgcgatag aaaacaaaat atagcgcgca aactaggata aattatcgcg 1800
   cgcggtgtca tctatgttac tagatcggga attgcggccc cccctcgagc ccacagatgg 1860
   ttagagaggc ttacgcagca ggtctcatca agacgatcta cccgagcaat aatctccagg 1920
   aaatcaaata ccttcccaag aaggttaaag atgcagtcaa aagattcagg actaactgca 1980
   tcaagaacac agagaaagat atatttctca agatcagaag tactattcca gtatggacga 2040
   ttcaaggctt gcttcacaaa ccaaggcaag taatagagat tggagtctct aaaaaggtag 2100
   ttcccactga atcaaaggcc atggagtcaa agattcaaat agaggaccta acagaactcg 2160
   ccgtaaagac tggcgaacag ttcatacaga gtctcttacg actcaatgac aagaagaaaa 2220
   tcttcgtcaa catggtggag cacgacacac ttgtctactc caaaaatatc aaagatacag 2280
   tctcagaaga ccaaagggca attgagactt ttcaacaaag ggtaatatcc ggaaacctcc 2340
   tcggattcca ttgcccagct atctgtcact ttattgtgaa gatagtggaa aaggaaggtg 2400
   gctcctacaa atgccatcat tgcgataaag gaaaggccat cgttgaagat gcctctgccg 2460
   acagtggtcc caaagatgga cccccaccca cgaggagcat cgtggaaaaa gaagacgttc 2520
   caaccacgtc ttcaaagcaa gtggattgat gtgatatctc cactgacgta agggatgacg 2580
   cacaatccca ctatccttcg caagaccctt cctctatata aggaagttca tttcatttgg 2640
   agagaacacg ggggactcta gaggatcaga ggacgaacaa catgtcccac cccgccatct 2700
   ccctgcaagc gctgcgcggc agcggcgcag acatacagtc catccacatc ccctacgagc 2760
   gccatgccga ccaggacgcc ggtgcggaca cgcccgcccg gcatcccgtc gtcatcgtcg 2820
   gcgccggccc cgtgggcctg tcgctggcca tcgacctggc ccagcgcggc cagcgcgtgg 2880
   tgctgctgga caacgactgc cggctgtcca cgggctcgcg cgccatctgc ttttccaagc 2940
   gcacgctgga gatctgggac cgcctgggcg tgggccagcc catggtggac aagggcgtgt 3000
   cctggaacct gggcaaggtc ttcttcaagg accagccgct gtaccgcttc gacctgctgc 3060
   ccgaggacgg ccacgagcgc ccggccttca tcaacctgca gcagtactac gccgaggcct 3120
   atctggtcga gcgcgcactg cagctgccgc tgatcgacct gcgctggcac agcaaggtca 3180
   cggcactgga gccgcaggcc gagggcgcgc tgctgaccgt ggagacgcct gacggcagct 3240
   accgcatcga tgcgcaatgg gtcctggcct gcgatggctc gcgctcgccg ctgcgcggcc 3300
   tgctgggcca ggaaagccat ggccgcatct tccgcgaccg cttcctgatc gccgacgtga 3360
   agatgcacgc cgaattcccc accgagcgct ggttctggtt cgacccgccc ttccacccgg 3420
   gccagagcgt gctgctgcac cgccagcccg acgatgtctg gcgcatcgac ttccagctgg 3480
   gctgggacgc ggaccccgag gaagagaaaa agcccgagaa catcgtgccg cgcatccgcg 3540
   ccctgctggg caaggacgcg cccttcgagc tggaatgggc cagcgtctac accttcgcct 3600
   gcctgcgcat ggaccgcttc gtccatggcc gcgtggtctt tgcgggcgac agcgcccacg 3660
   gcgtctcgcc gtttggcgca cgcggcgcca acagcggcgt gcaggatgcc gagaacctgg 3720
   catggaagct ggaccgcgtg ctgcgcggcc aggccgatgc ctcgctgatc gccacctacg 3780
   gcgccgagcg cgaatacgcg gccgacgaga acatccgcaa ctccacgcgc gccaccgact 3840
   tcatcacgcc caagagcgag atcagccgcc tgtttcgcga cgccgtgctg gacctggcgc 3900
   gcgaccatga attcgcgcgc cgcatcgtca acagcgggcg gctgtccgtg ccggccacgc 3960
   tgcacggctc cgcgctcaac acgcctgaca ccgacacctt cgacggaacg cagctgcccg 4020
   gcgccgtgct ggccgatgcg cccatgcgcc ggcccggcgc agacggcacg gcctggctgc 4080
   tgcgcgcact gggaccggac ttcacgctgc tgcacttcga ccccacgccc gcctgggcgc 4140
   aggcgctgcc cggcgtgctc aacctgtcca tcgcggccga gggcgaggcc catgcgccag 4200
   acgccgacct catcgatgcg cgcggcctgg cggccaaacg cctggatgca cgccccggca 4260
   ccagctacct gctgcggcct gaccagcatg tctgcgcgcg ctggcgccgc cccgacgaag 4320
   ccagcgtgcg cgccgcgctg caaagagcct gcggcgccgc cgccacggcc tgaacctctt 4380
   aagcttatcg ataccgtcga cgaatttccc cgatcgttca aacatttggc aataaagttt 4440
   cttaagattg aatcctgttg ccggtcttgc gatgattatc atataatttc tgttgaatta 4500
   cgttaagcat gtaataatta acatgtaatg catgacgtta tttatgagat gggtttttat 4560
   gattagagtc ccgcaattat acatttaata cgcgatagaa aacaaaatat agcgcgcaaa 4620
   ctaggataaa ttatcgcgcg cggtgtcatc tatgttccta ggtcgggaat tgcggcc 4677
<210> 22
   <211> 8187
   <212> ADN
   <213> Séquence artificielle
<220>
   <223> Description de la séquence artificielle: cassette d'expression
<400> 22
   ccccctcgag gtcgacggta ttgatcagct tccagaaggt aattatccaa gatgtagcat 60
   caagaatcca atgtttacgg gaaaaactat ggaagtatta tgtgagctca gcaagaagca 120
   gatcaatatg cggcacatat gcaacctatg ttcaaaaatg aagaatgtac agatacaaga 180
   tcctatactg ccagaatacg aagaagaata cgtagaaatt gaaaaagaag aaccaggcga 240
   agaaaagaat cttgaagacg taagcactga cgacaacaat gaaaagaaga agataaggtc 300
   ggtgattgtg aaagagacat agaggacaca tgtaaggtgg aaaatgtaag ggcggaaagt 360
   aaccttatca caaaggaatc ttatccccca ctacttatcc ttttatattt ttccgtgtca 420
   tttttgccct tgagttttcc tatataagga accaagttcg gcatttgtga aaacaagaaa 480
   aaatttggtg taagctattt tctttgaagt actgaggata caacttcaga gaaatttgta 540
   agtttgtaga tctgaattcg atgcaggatg cacatgtcca ccaagacctt tgcctccgcc 600
   gccgacctcg aaatcaagca ggtcagcttc gacaagctct ccgagcacgc ctatgcctac 660
   acggccgaag gcgaccccaa caccggcatc atcattggcg acgacgcggt gatggtgatc 720
   gacacccagg ccacgcccgt catggcccag gacgtgatcc gccgcatccg tgaggtcacg 780
   gacaagccca tcaagtacgt gacgctgtcg cactaccacg cggtgcgcgt gctgggcgcc 840
   tcggccttct tcgcggaagg cgccgaacac atcattgcca gccaggacac ctacgacctc 900
   atcgtggagc gcggcgagca ggacaaggcc agcgagatcg gccgctttcc ccgcctgttc 960
   cagaacgtgg aaagcgtgcc cgatggcatg acctggccca ccctcacctt caccggcaag 1020
   atgacgctgt ggctgggcaa gctggaagtg cagatcctgc agctgggccg cggccacacc 1080
   aagggcgaca ccgtggtctg gctgccccag gacaaggtgc tgttcagcgg cgacctggtg 1140
   gagttcggcg ccacgcccta tgcgggcgat gcctacttcc aggactggcc gcacacgctg 1200
   gacgccatcg ccgccctgca gcccgaaaag ctcgtgcccg gccggggcgc cgcgctgcag 1260
   acgccggccg aggtgcaggc cggcctggcc ggcacgcgcg acttcatcag cgacctgtgg 1320
   accgaggtca aggccggcgc cgatgcccag caggacctgc gcaaggtcta cgaggccgcc 1380
   ttcgccaagc tgcagcccaa gtacggccag tgggtgatct tcaaccactg catgcccttc 1440
   gatgtgaccc gcgcctatga cgaggcatcg ggccacgccg acccacgcat ctggaccgcc 1500
   gagcgcgacc gccagatgtg gctggcgctc gaaggctgat gcaagcttat cgataccgtc 1560
   gacgaatttc cccgatcgtt caaacatttg gcaataaagt ttcttaagat tgaatcctgt 1620
   tgccggtctt gcgatgatta tcatataatt tctgttgaat tacgttaagc atgtaataat 1680
   taacatgtaa tgcatgacgt tatttatgag atgggttttt atgattagag tcccgcaatt 1740
   atacatttaa tacgcgatag aaaacaaaat atagcgcgca aactaggata aattatcgcg 1800
   cgcggtgtca tctatgttac tagatcggga attgcggccc cccctcgagc ccacagatgg 1860
   ttagagaggc ttacgcagca ggtctcatca agacgatcta cccgagcaat aatctccagg 1920
   aaatcaaata ccttcccaag aaggttaaag atgcagtcaa aagattcagg actaactgca 1980
   tcaagaacac agagaaagat atatttctca agatcagaag tactattcca gtatggacga 2040
   ttcaaggctt gcttcacaaa ccaaggcaag taatagagat tggagtctct aaaaaggtag 2100
   ttcccactga atcaaaggcc atggagtcaa agattcaaat agaggaccta acagaactcg 2160
   ccgtaaagac tggcgaacag ttcatacaga gtctcttacg actcaatgac aagaagaaaa 2220
   tcttcgtcaa catggtggag cacgacacac ttgtctactc caaaaatatc aaagatacag 2280
   tctcagaaga ccaaagggca attgagactt ttcaacaaag ggtaatatcc ggaaacctcc 2340
   tcggattcca ttgcccagct atctgtcact ttattgtgaa gatagtggaa aaggaaggtg 2400
   gctcctacaa atgccatcat tgcgataaag gaaaggccat cgttgaagat gcctctgccg 2460
   acagtggtcc caaagatgga cccccaccca cgaggagcat cgtggaaaaa gaagacgttc 2520
   caaccacgtc ttcaaagcaa gtggattgat gtgatatctc cactgacgta agggatgacg 2580
   cacaatccca ctatccttcg caagaccctt cctctatata aggaagttca tttcatttgg 2640
   agagaacacg ggggactcta gaggatcaga ggacgaacaa catgtcccac cccgccatct 2700
   ccctgcaagc gctgcgcggc agcggcgcag acatacagtc catccacatc ccctacgagc 2760
   gccatgccga ccaggacgcc ggtgcggaca cgcccgcccg gcatcccgtc gtcatcgtcg 2820
   gcgccggccc cgtgggcctg tcgctggcca tcgacctggc ccagcgcggc cagcgcgtgg 2880
   tgctgctgga caacgactgc cggctgtcca cgggctcgcg cgccatctgc ttttccaagc 2940
   gcacgctgga gatctgggac cgcctgggcg tgggccagcc catggtggac aagggcgtgt 3000
   cctggaacct gggcaaggtc ttcttcaagg accagccgct gtaccgcttc gacctgctgc 3060
   ccgaggacgg ccacgagcgc ccggccttca tcaacctgca gcagtactac gccgaggcct 3120
   atctggtcga gcgcgcactg cagctgccgc tgatcgacct gcgctggcac agcaaggtca 3180
   cggcactgga gccgcaggcc gagggcgcgc tgctgaccgt ggagacgcct gacggcagct 3240
   accgcatcga tgcgcaatgg gtcctggcct gcgatggctc gcgctcgccg ctgcgcggcc 3300
   tgctgggcca ggaaagccat ggccgcatct tccgcgaccg cttcctgatc gccgacgtga 3360
   agatgcacgc cgaattcccc accgagcgct ggttctggtt cgacccgccc ttccacccgg 3420
   gccagagcgt gctgctgcac cgccagcccg acgatgtctg gcgcatcgac ttccagctgg 3480
   gctgggacgc ggaccccgag gaagagaaaa agcccgagaa catcgtgccg cgcatccgcg 3540
   ccctgctggg caaggacgcg cccttcgagc tggaatgggc cagcgtctac accttcgcct 3600
   gcctgcgcat ggaccgcttc gtccatggcc gcgtggtctt tgcgggcgac agcgcccacg 3660
   gcgtctcgcc gtttggcgca cgcggcgcca acagcggcgt gcaggatgcc gagaacctgg 3720
   catggaagct ggaccgcgtg ctgcgcggcc aggccgatgc ctcgctgatc gccacctacg 3780
   gcgccgagcg cgaatacgcg gccgacgaga acatccgcaa ctccacgcgc gccaccgact 3840
   tcatcacgcc caagagcgag atcagccgcc tgtttcgcga cgccgtgctg gacctggcgc 3900
   gcgaccatga attcgcgcgc cgcatcgtca acagcgggcg gctgtccgtg ccggccacgc 3960
   tgcacggctc cgcgctcaac acgcctgaca ccgacacctt cgacggaacg cagctgcccg 4020
   gcgccgtgct ggccgatgcg cccatgcgcc ggcccggcgc agacggcacg gcctggctgc 4080
   tgcgcgcact gggaccggac ttcacgctgc tgcacttcga ccccacgccc gcctgggcgc 4140
   aggcgctgcc cggcgtgctc aacctgtcca tcgcggccga gggcgaggcc catgcgccag 4200
   acgccgacct catcgatgcg cgcggcctgg cggccaaacg cctggatgca cgccccggca 4260
   ccagctacct gctgcggcct gaccagcatg tctgcgcgcg ctggcgccgc cccgacgaag 4320
   ccagcgtgcg cgccgcgctg caaagagcct gcggcgccgc cgccacggcc tgaacctctt 4380
   aagcttatcg ataccgtcga cgaatttccc cgatcgttca aacatttggc aataaagttt 4440
   cttaagattg aatcctgttg ccggtcttgc gatgattatc atataatttc tgttgaatta 4500
   cgttaagcat gtaataatta acatgtaatg catgacgtta tttatgagat gggtttttat 4560
   gattagagtc ccgcaattat acatttaata cgcgatagaa aacaaaatat agcgcgcaaa 4620
   ctaggataaa ttatcgcgcg cggtgtcatc tatgttccta ggtcgaggag aaatatgagt 4680
   cgaggcatgg atacactaag ttcccctgaa gtgagcatga tctttgatgc tgagatgatt 4740
   cccagagcaa gatagtttgt gctgcaagtg acacaattgt aatgaaacca ccactcaacg 4800
   aatttacttg tggctttgac atgtcgtgtg ctctgtttgt atttgtgagt gccggttggt 4860
   aattattttt gttaatgtga ttttaaaacc tcttatgtaa atagttactt tatctattga 4920
   agtgtgttct tgtggtctat agtttctcaa agggaaatta aaatgttgac atcccattta 4980
   caattgataa cttggtatac acaaactttg taaatttggt gatatttatg gtcgaaagaa 5040
   ggcaataccc attgtatgtt ccaatatcaa tatcaatacg ataacttgat aatactaaca 5100
   tatgattgtc attgtttttc cagtatcaat atacattaag ctactacaaa attagtataa 5160
   atcactatat tataaatctt tttcggttgt aacttgtaat tcgtgggttt ttaaaataaa 5220
   agcatgtgaa aattttcaaa taatgtgatg gcgcaatttt attttccgag ttccaaaata 5280
   ttgccgcttc attaccctaa tttgtggcgc cacatgtaaa acaaaagacg attcttagtg 5340
   gctatcactg ccatcacgcg gatcactaat atgaaccgtc gattaaaaca gatcgacggt 5400
   ttatacatca ttttattgta cacacggatc gatatctcag ccgttagatt taatatgcga 5460
   tctgattgct caaaaaatag actctccgtc tttgcctata aaaacaattt cacatctttc 5520
   tcacccaaat ctactcttaa ccgttcttct tcttctacag acatcaattt ctctcgactc 5580
   tagaggatcc aagcttatcg atttcgaacc cctcatgact tcacttacag tgtccggccg 5640
   ggtggcgcag gtcctcagca gctatgtcag cgatgtgttc ggtgtgatgg gcaacggaaa 5700
   cgtctacttc ctggacgccg ccgagaagga gggcctccgc ttcacggccg tacgccatga 5760
   aggtgccgcc atcgcggcgg cggacgccta ctatcgggca tccgggcgcc tggcggcggg 5820
   gaccaccacc tacggccccg gttacaccaa cgccctgacg gccctcgccg aggcggtcca 5880
   ggcgcagatc cccgtggtgc tcgtcaccgg ggacgccccg agcagcggcg cccggccttg 5940
   ggacgtggac caggccgcga tcgccgccgg gctgggggcg gcgaccttca cggtcacccg 6000
   tgaagccgca ggctccatca cgcaggaagc ggtggagtac gcacttgccc ggcggaccgc 6060
   cgtcgtgatc gccgttccat acgacctgtc ggcccttgag gcggcggagg aagatcttcc 6120
   cgtgccgccg gcggcctcgg ttccggacgc catcggcggc ggactcggac gggcggccga 6180
   agtgcgggcg gccgaattgc tggcgggcgc gaagcggccg ctcatccttg ccggccgcgg 6240
   tgcgcacctc gcaggaaccg gccccgaact ccgggaactc gccgaccgcc tcggcgcgct 6300
   cacggccggc accgcactgg cgctgaacct gctgcagggc gaggggtacc tcggcgtcgc 6360
   gggcggcttc ggcacggata ccgccgccgg gctcatgggc gaggcggacg tggtgctcgt 6420
   ggcgggagcc agcctgaccc ccttcaccat gcgcttcggc cacctgatcg gcccggacgc 6480
   caccgtgatc cagatcgaca ccgccatgga gccgacggac ccgcgggtgg acctgtttgt 6540
   cagtgcggac gcgaaggccg ctgccggccg gatcctccgg ctgctggatg acgccgccgg 6600
   ggccaatgcg tcgaaggcct ggcgcgcgga agcactcaag cgtctggccg aaggaccctg 6660
   ccaccacccc ggcaccgcag agaccacgga cggccgcctt gacccccggg cgcttgcttc 6720
   ggcactggat gccgtcctgc cggaacgccg caccgtggtc caggacggcg ggcacttcct 6780
   gggctgggca cccatgtact ggcgcatccc ccgtcctcag gacctggtca tggtggggac 6840
   cgcgtaccag tcgatcgggc ttggcctggc cagcgccgtg ggggcgtccc gggccgtgga 6900
   cgacggcaat atcctggtgc tggcggcggg cgacggcgga ttcctgatgg gcctgtccga 6960
   cctggaatcg ctcgtgggcg cggcgagcag cgccgtcgtg gtgatctaca acgacgccgc 7020
   ctacggggcc gagatccatc agtacggctc acgggggctc accgaaaagc ccatgctgat 7080
   ccccgaagtg gacttcagcg ggattgcccg cgcgatcggg gcggaatccg caatcatccg 7140
   caagctgtcg gacctctccg cgctcacgga ctggatcgag gccggcgcca ggggaacctt 7200
   cgtggccgac tgccgcatca cctcaagcgt ccgggccccg tggctgagcg aatggatgag 7260
   ggcctcgcaa gcggcgaagg aggcggtggc gggctagggc cggcctcgtc gaaatgccgc 7320
   cctccaaccc aactcagtac cagctcaggg cgttctcagg gctgggaacg ccctgagctg 7380
   ctactcattt gttcgaactc gaggtcgacg gtatcgataa gcttgatatc gaattcctgc 7440
   agcccggggg atccactagg ggatcccccg atccgcgttt gtgttttctg ggtttctcac 7500
   ttaagcgtct gcgttttact tttgtattgg gtttggcgtt tagtagtttg cggtagcgtt 7560
   cttgttatgt gtaattacgc tttttcttct tgcttcagca gtttcggttg aaatataaat 7620
   cgaatcaagt ttcactttat cagcgttgtt ttaaattttg gcattaaatt ggtgaaaatt 7680
   gcttcaattt tgtatctaaa tagaagagac aacatgaaat tcgacttttg acctcaaatc 7740
   ttcgaacatt tatttcctga tttcacgatg gatgaggata acgaaagggc ggttcctatg 7800
   tccgggaaag ttcccgtaga agacaatgag caaagctact gaaacgcgga cacgacgtcg 7860
   cattggtacg gatatgagtt aaaccgactc aattccttta ttaagacata aaccgatttt 7920
   ggttaaagtg taacagtgag ctgatataaa accgaaacaa accggtacaa gtttgattga 7980
   gcaacttgat gacaaacttc agaattttgg ttattgaatg aaaatcatag tctaatcgta 8040
   aaaaatgtac agaagaaaag ctagagcaga acaaagattc tatattctgg ttccaattta 8100
   tcatcgcttt aacgtccctc agatttgatc gggctgcagg aattcggcct gactgatcat 8160
   ttaaacacta ggtcgggaat tgcggcc 8187

## Description

La présente invention concerne une nouvelle méthode permettant de rendre les plantes tolérantes aux herbicides, en particulier aux herbicides inhibiteurs d'HPPD, les séquences d'acide nucléique codant pour des enzymes susceptibles d'être employées dans cette méthode, les cassettes d'expression les contenant et les plantes transgéniques comprenant au moins l'une de ces cassettes d'expression.

Les hydroxy-phényl pyruvate dioxygénases sont des enzymes qui catalysent la réaction de transformation du para-hydroxy-phényl-pyruvate (HPP) en homogentisate. Cette réaction a lieu en présence de fer (Fe²⁺) en présence d'oxygène (Crouch N.P. & al., Tetrahedron, 53, 20, 6993-7010, 1997).

On connaît par ailleurs certaines molécules inhibitrices de cette enzyme, qui viennent se fixer à l'enzyme pour inhiber la transformation de l'HPP en homogentisate. Certaines de ces molécules ont trouvé un emploi comme herbicides, dans la mesure où l'inhibition de la réaction dans les plantes conduit à un blanchiment des feuilles des plantes traitées, et à la mort des dites plantes (Pallett K. E. et al. 1997 Pestic. Sci. 50 83-84). De tels herbicides ayant pour cible l'HPPD décrits dans l'état de la technique sont notamment les isoxazoles (EP 418 175, EP 470 856, EP 487 352, EP 527 036, EP 560 482, EP 682 659, US 5 424 276) en particulier l'isoxaflutole, herbicide sélectif du maïs, les dicétonitriles (EP 496 630, EP 496 631), en particulier la 2-cyano-3-cyclopropyl-1-(2-SO₂ CH₃-4-CF₃ phényl) propane-1,3-dione et la 2-cyano-3-cyclopropyl-1-(2-SO₂ CH₃-4-2,3 Cl₂ phényl) propane-1, 3-dione, les tricétones (EP 625 505, EP 625 508, US 5,506,195), en particulier la sulcotrione ou la mésotrione, ou encore les pyrazolinates.

Des essais pour confirmer que l'HPPD est bien la cible des dicétonitriles (DKN) et pour mettre en évidence que l'HPPD est, au moins à certaines doses, la cible unique des dicétonitriles ont été effectués en laboratoire en faisant germer des graines d*'Arabidopsis* sur trois types de milieux en conditions stériles in-vitro :
1 milieu Murashig et Skoog (Murashige, T. and Skoog, F. 1962. A revised medium for a rapid growth and bioassays with tobacco tissue culture. Physiol. Plant. 15, 473-479), expérience contrôle de la germination
2 milieu MS plus DKN à la dose de 1ppm
3 milieu MS plus DKN à la même dose + Homogentisate à la concentration de 5 mM.

Il est très net que sur le milieu 1 la germination se fait normalement, chaque plantule développant deux cotylédons bien verts. Le développement se fait ensuite normalement. Sur le milieu 2, la germination a lieu mais la plantule qui émerge est blanche, les deux cotylédons ne présentant aucune pigmentation. Les plantules meurent ensuite en quelques jours. Sur le milieu 3, la germination se fait normalement, les cotylédons sont bien verts. Les plantules se développent mais très rapidement, la quantité d'homogentisate dans le milieu diminuant, les premiers symptômes de blanchiment apparaissent et la croissance des plantes s'arrêtent, elles finissent par mourir comme dans l'essai effectué sur le milieu n°2.

Ceci permet de confirmer que l'HPPD est bien la cible des DKN in planta et qu'elle semble être la cible unique. Ceci montre aussi que l'homogentisate est transporté du milieu de culture jusqu'au site cellulaire où il est nécessaire pour le bon fonctionnement de la cellule et la survie de la plante.

Pour rendre les plantes tolérantes aux herbicides, on dispose actuellement de trois stratégies, (1) la détoxification de l'herbicide par une enzyme venant transformer l'herbicide, ou son métabolite actif, en produits de dégradation non toxique, comme par exemple les enzymes de tolérance au bromoxynil ou au basta (EP 242 236, EP 337 899) ; (2) la mutation de l'enzyme cible en une enzyme fonctionnelle moins sensible à l'herbicide, ou son métabolite actif, comme par exemple les enzymes de tolérance au glyphosate (EP 293 356, Padgette S. R. & al., J. Biol. Chem., 266, 33, 1991) ; ou (3) la surexpression de l'enzyme sensible, de manière à produire dans la plante des quantités suffisantes d'enzyme cible au regard des constantes cinétiques de cette enzyme vis à vis de l'herbicide de manière à avoir suffisamment d'enzyme fonctionnelle, malgré la présence de son inhibiteur.

C'est cette troisième stratégie qui a été décrite pour obtenir avec succès des plantes tolérantes aux inhibiteurs d'HPPD (WO 96/38567), étant entendu que pour la première fois une stratégie de simple surexpression de l'enzyme cible sensible (non mutée) était employée avec succès pour conférer aux plantes une tolérance à un niveau agronomique à un herbicide. L'identification d'HPPD mutées dans leur partie C-terminal présentant une tolérance améliorée aux inhibiteurs d'HPPD a permis d'obtenir une amélioration de la tolérance des plantes par la mise en oeuvre de la deuxième stratégie (WO 99/24585).

La présente invention concerne une nouvelle méthode permettant de rendre les plantes tolérantes à un herbicide qui met en oeuvre une nouvelle ou quatrième stratégie de tolérance herbicide, cette nouvelle stratégie comprenant le contournement de la voie métabolique inhibée par ledit herbicide. Ce contournement métabolique peut se résumer comme suit :
➢ soit un herbicide « H » actif en inhibant l'activité d'une enzyme « E » qui transforme le substrat « S » en produit « P », ledit produit P et ses métabolites étant essentiels à la vie de la plante,
➢ le contournement métabolique consiste à exprimer dans la plante au moins une nouvelle enzyme « NE » hétérologue insensible à « H » permettant la conversion de « S » en un produit intermédiaire « I », lequel est ensuite transformé en « P » soit par les voies de biosynthèse naturelles de la plante soit par l'expression d'au moins une autre enzyme hétérologue « AE » également insensible à « H »,
➢ le contournement métabolique consistant également à exprimer au moins une autre enzyme hétérologue « AE » insensible à « H » permettant la conversion de « I » en « P », « I » étant soit un intermédiaire naturellement produit par la plante soit obtenu par l'expression d'au moins une nouvelle enzyme hétérologue « NE » insensible à « H » permettant la conversion de « S » en « I ».

La présente invention concerne plus particulièrement une nouvelle méthode permettant de rendre les plantes tolérantes aux inhibiteurs d'HPPD, ladite méthode comprenant le contournement métabolique de l'HPPD.

Aucune voie de contournement métabolique n'a été décrite à ce jour dans les plantes.

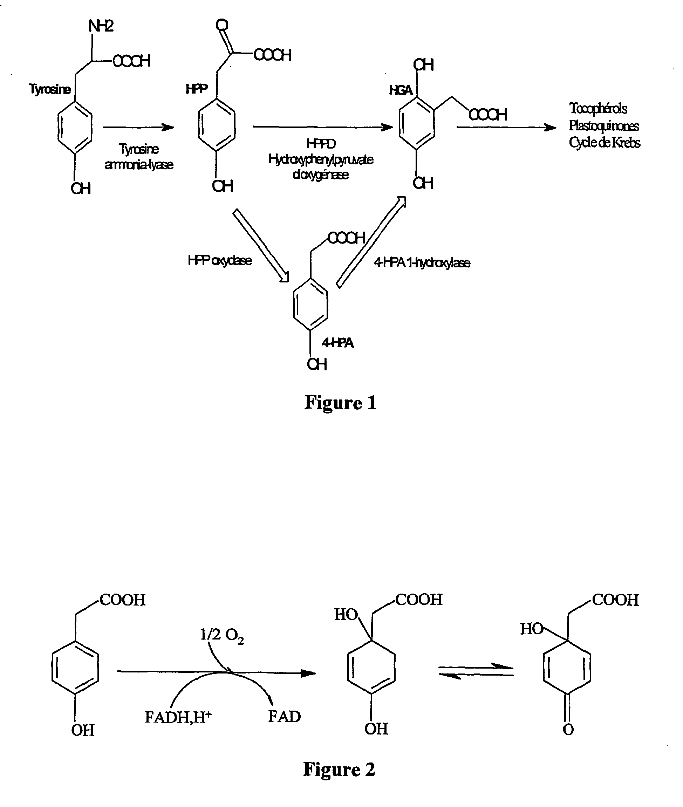

On connaît de la littérature que la conversion de l'HPP en homogentisate peut être obtenue en effectuant d'abord une conversion de l'HPP en acide 4-hydroxyphénylacétique (4-HPA) par un extrait enzymatique présentant une activité HPP oxydase suivie de la conversion du 4-HPA en homogentisate par un extrait enzymatique présentant une activité 4-HPA 1-hydrolase (WO 99/34008). Cette voie de contournement est représentée par la Figure 1.

Une étude bibliographique révèle que les activités enzymatiques nécessaires pour la construction de la voie de contournement de l'HPPD ont été caractérisées sur des extraits bruts bactériens dans les années 1970. Ainsi, les activités HPP oxydase (HPPO, E.C. 1.2.3.-) et 4-HPA 1-hydroxylase (HPAH, E.C. 1.14.13.18.) ont été identifiées respectivement chez *Arthrobacter globiformis* (Blakley, 1977) et chez *Pseudomonas acidovorans* (Hareland *et al.,* 1975). Depuis lors, seule la HPAH a été purifiée par *Suemori et al.* (1996), cependant, ni la séquence protéique ni la séquence nucléique ne sont publiées. Il faut donc identifiés les gènes codant ces activités enzymatiques.

Dans la voie de contournement, la conversion de l'HPP en HGA se fait *via* le 4-HPA. Or, le 4-HPA est un composé rarement identifié chez les plantes. Il est présent chez l'*Astilbe chinensis* (Kindl, 1969), dans les *Plantago* sp. (Swiatek, 1977), dans le pissenlit (*Taraxacum officinale* ; Dey & Harborne, 1997), chez les *Artemisia* (Swiatek *et al.,* 1998), dans le fruit du *Forsythia suspensa* (Liu *et al.,* 1998) et enfin chez l'algue marine *Ulva lactuca* (Flodin *et al.,* 1999). Il y a peu de données sur son origine. Il semble pouvoir provenir dé la tyrosine, du shikimate, de la tyramine. Il n'y a pas davantage d'information sur son devenir et son rôle dans la plante. Kindl (1969) a montré sa dégradation *via* l'acide 3,4-dihydroxyphénylacétique tandis que Flodin *et al.* (1999) ont démontré sa conversion *via* l'acide 4-hydroxymandélique en 2,4,6-tribromophénol qui s'accumule dans l'algue verte *Ulva lactuca.* Gross (1975) suggère que le 4-HPA pourrait être un régulateur de croissance chez certaines plantes supérieures et Abe *et al.* (1974) le considèrent comme un analogue d'auxine chez les algues.

Pour mettre en oeuvre la voie de contournement métabolique de l'HPPD il aura fallu au préalable identifier et isoler les gènes et les séquences d'acide nucléique codant pour la ou les enzymes responsables des deux activités ci-dessus.

L'objet de la présente invention est un procédé pour rendre les plantes tolérantes à un herbicide inhibiteur de l'acide hydroxyphényl pyruvique dioxygénase (HPPD) caractérisé en ce que sont exprimées dans lesdites plantes au moins une enzyme insensible au dit herbicide inhibiteur d'HPPD convertissant l'acide hydroxyphényl-pyruvique (HPP) en acide 4-hydroxyphénylacétique (4-HPA) et une HPAH et une HPAC insensibles au dit herbicide inhibiteur d'HPPD convertissant l' 4-HPA en homogentisate et dont une au moins de ces deux enzymes est hétérologue, et pour lequel l'HPAH comprend une séquence d'acides aminés sélectionnée parmi le groupe constitué de la SEQ ID NO 8 et de ses fragments, et l'HPAC comprend une séquence d'acides aminés sélectionnée parmi le groupe constitué des SEQ ID NO 10, SEQ ID NO 12 et SEQ ID NO 14, et de leurs fragments.

L'objet de la présente invention est également un procédé pour rendre les plantes tolérantes à un herbicide inhibiteur de l'acide hydroxyphényl pyruvique dioxygénase (HPPD) caractérisé en ce que sont exprimées dans lesdites plantes au moins une enzyme insensible au dit herbicide inhibiteur d'HPPD convertissant l'acide hydroxyphényl-pyruvique (HPP) en acide 4-hydroxyphénylacétique (4-HPA) et une HPAH et une HPAC insensibles au dit herbicide inhibiteur d'HPPD convertissant l'4-HPA en homogentisate et dont une au moins de ces deux enzymes est hétérologue, et pour lequel l'HPAH est codée par une séquence d'acide nucléique comprenant une séquence sélectionnée parmi le groupe constitué des séquences codantes des SEQ ID NO 7 et SEQ ID NO 17, de leurs fragments et des séquences capables de s'hybrider de manière sélective dans des conditions de milieu très sévères aux dites SEQ ID NO 7ou 17, et l'HPAC est codée par une séquence d'acide nucléique comprenant une séquence sélectionnée parmi le groupe constitué des séquences codantes des SEQ ID NO 9, SEQ ID NO 11, SEQ ID NO 13, et SEQ ID NO 19, de leurs fragments et des séquences capables de s'hybrider de manière sélective dans des conditions de milieu très sévères aux dites SEQ ID NO 9, 11, 13 ou 19.

Selon un mode particulier de réalisation du procédé selon l'invention, l'enzyme insensible au dit herbicide inhibiteur d'HPPD convertissant l'acide hydroxyphényl-pyruvique (HPP) en acide 4-hydroxyphénylacétique (4-HPA) est une HPP oxydase.

Selon un autre mode particulier de réalisation du procédé selon l'invention, l'enzyme insensible au dit herbicide inhibiteur d'HPPD convertissant l'acide hydroxyphényl-pyruvique (HPP) en acide 4-hydroxyphénylacétique (4-HPA) est une HPP oxydase comprenant la séquence d'acides aminés sélectionnée parmi le groupe constitué des SEQ ID NO 2, SEQ ID NO 4 et SEQ ID NO 6, et de leurs fragments.

Selon un autre mode particulier de réalisation du procédé selon l'invention, l'enzyme insensible au dit herbicide inhibiteur d'HPPD convertissant l'acide hydroxyphényl-pyruvique (HPP) en acide 4-hydroxyphénylacétique (4-HPA) est une HPP oxydase comprenant une séquence sélectionnée parmi le groupe constitué des séquences codantes des SEQ ID NO 1, SEQ ID NO 3, SEQ ID NO 5 et SEQ ID NO15, de leurs fragments et des séquences capables de s'hybrider de manière sélective dans des conditions de milieu très sévères aux dites SEQ ID NO 1, 3, 5, ou 15.

### Définitions selon l'invention

« Séquence d'acide nucléique » : une séquence nucléotidique ou polynucléotide, pouvant être de type ADN ou ARN, de préférence de type ADN, notamment double brin. La séquence d'acide nucléique peut être d'origine naturelle, en particulier ADN génomique ou ADNc, ou encore une séquence synthétique ou semi-synthétique, les acides nucléiques la comprenant ayant été choisis soit pour optimiser les codons d'une séquence codante en fonction de l'organisme hôte dans lequel elle sera exprimée, soit pour introduire ou éliminer un ou plusieurs sites de restriction. Les méthodes de préparation des séquences d'acide nucléique synthétiques ou semi-synthétiques sont bien connues de l'homme du métier.

« Séquence capable de s'hybridiser de manière sélective » : les séquences d'acide nucléique qui s'hybrident avec une séquence d'acide nucléique de référence à un niveau suppérieur au bruit de fond de manière significative. Le bruit de fond peut être lié à l'hybridisation d'autres séquences d'ADN présentes, notamment d'autres ADNc présentes dans une banque d'ADNc. Le niveau du signal généré par l'interaction entre la séquence capable de s'hybridiser de manière sélective et les séquences définies par les séquence ID ci-dessus selon l'invention est généralement 10 fois, de préférence 100 fois plus intense que celui de l'interaction des autres séquences d'ADN générant le bruit de fond. Le niveau d'interaction peut être mesuré par exemple , par marquage de la sonde avec des éléments radioactifs, comme le 32P. L'hybridation sélective est généralement obtenue en employant des conditions de milieu très sévères (par exemple NaCl 0,03 M et citrate de sodium 0,03 M à environ 50°C-60°C). L'hybridation peut bien entendu être effectuée selon les méthodes usuelles de l'état de la technique (notamment Sambrook & al., 1989, Molecular Cloning : A Labratory Manual).

« Homologue d'une séquence d'acide nucléique » : séquence d'acide nucléique présentant une ou plusieurs modifications de séquence par rapport à une séquence d'acide nucléique de référence. Ces modifications peuvent être obtenues selon les techniques usuelles de mutation, ou encore en choisissant les oligonucléotides synthétiques employés dans la préparation de ladite séquence par hybridation. Au regard des multiples combinaisons d'acides nucléiques pouvant conduire à l'expression d'un même acide aminé, les différences entre la séquence de référence selon l'invention et l'homologue correspondant peuvent être importantes. De manière avantageuse, le degré d'homologie sera d'au moins 60 % par rapport à la séquence de référence, de préférence d'au moins 70 %, plus préférentiellement d'au moins 80%, encore plus préférentiellement d'au moins 90 %. Ces modifications sont généralement et de préférence neutres, c'est à dire que pour une séquence codante elles n'affectent pas la séquence primaire de la protéine ou du peptide codé. Elles peuvent toutefois introduire des modifications non silencieuses, ou mutations, qui n'affectent pas la fonction de la séquence d'acide nucléique par rapport à la séquence de référence. Les méthodes de mesure et d'identification des homologies entre les séquences d'acides nucléiques sont bien connues de l'homme du métier. On peut employer par exemple les programmes PILEUP ou BLAST (notamment Altschul & al., 1993, J. Mol. Evol. 36 :290-300 ; Altschul & al., 1990, J. Mol. Biol. 215 :403-10).

« Fragments » : fragment d'une séquence d'acide nucléique ou polypeptidique de référence pour laquelle des parties ont été délétées mais qui conservent la fonction de ladite séquence de référence.

« Hétérologue » : séquence d'acide nucléique différente de la séquence d'acide nucléique ayant la même fonction dans un organisme naturel.Une séquence hétérologue peut consister en une séquence d'acide nucléique modifiée *in situ* dans son environement naturel. Il peut également s'agir d'une séquence d'acide nucléique isolée de son organisme naturel puis réintroduite dans ce même organisme. Il peut également s'agit d'une séquence d'acide nucléique hétérologue par rapport à une autre séquence d'acide nucléique, c'est à dire une séquence associée à une autre séquence, cette association ne se trouvant pas dans la nature. C'est le cas notamment des cassettes d'expression constituées de différentes séquences d'acide nucléique ne se trouvant pas généralement associées dans la nature.

« Homologue d'une séquence protéique » : séquences protéiques dont la séquence primaire est différente de la séquence primaire de la protéine de référence, mais qui remplit la même fonction que cette séquence de référence. Les méthodes de mesure et d'identification des homologies entre polypeptides ou protéines sont également connues de l'homme du métier. On peut employer par exemple le « package » UWGCG et le programme BESTFITT pour calculer les homologies (Deverexu & al., 1984, Nucleic Acid Res. 12, 387-395).

« Cassette d'expression » : séquence d'acide nucléique comprenant différents éléments fonctionnels nécessaires à l'expression d'une séquence codante dans un organisme hôte. Ces éléments fonctionnels comprennent dans le sens de la transcription une séquence de régulation promotrice, également appelée promoteur, liée de manière fonctionnelle à une séquence codante et une séquence de régulation terminatrice, également appelée terminateur ou stop. La cassette d'expression peut également comprendre entre la séquence de régulation promotrice et la séquence codante des éléments de régulations tels que des activateurs de transcription ou « enhancers » et/ou des introns.

« Organisme hôte » : on entend essentiellement selon l'invention les cellules végétales ou les plantes. Pour les vecteurs de clonages, les organismes hôtes peuvent également être des bactéries, des champignons ou des levures.

« Cellule végétale » : cellule issue d'une plante et pouvant constituer des tissus indifférenciés tels que des cals, des tissus différenciés tels que des embryons, des parties de plantes, des plantes ou des semences.

« Plante » : organisme multicellulaire différencié capable de photosynthèse, en particulier monocotylédone ou dicotylédone, plus particulièrement des plantes de culture destinées ou non à l'alimentation animale ou humaine, comme le riz, le maïs, le blé, l'orge, la canne à sucre, le colza, le soja, la betterave, la pomme de terre, le tabac, le coton, le trèfle, le gazon, ou les plantes ornementales comme les pétunias, ou encore les bananiers, la vigne, les framboises, les fraises, les tomates, les salades, etc.

« Séquence de régulation promotrice » : Comme séquence de régulation promotrice dans les plantes, on peut utiliser toute séquence de régulation promotrice d'un gène s'exprimant naturellement dans les plantes en particulier un promoteur s'exprimant notamment dans les feuilles des plantes, comme par exemple des promoteurs dits constitutifs d'origine bactérienne, virale ou végétale ou encore des promoteurs dits lumière dépendants comme celui d'un gène de la petite sous-unité de ribulose- biscarboxylase/oxygénase (RuBisCO) de plante ou tout promoteur convenable connu pouvant être utilisé. Parmi les promoteurs d'origine végétale on citera les promoteurs d'histone tels que décrits dans la demande EP 0 507 698, ou le promoteur d'actine de riz (US 5,641,876). Parmi les promoteurs d'un gène de virus de plante, on citera celui de la mosaïque du choux fleur (CAMV 19S ou 35S), du CsVMV (US ...) ou le promoteur du circovirus (AU 689 311). On peut encore utiliser une séquence de régulation promotrice spécifique de régions ou de tissus particuliers des plantes, et plus particulièrement des promoteurs spécifiques des graines ([22] Datla, R.& al., Biotechnology Ann. Rev. (1997) 3, 269-296), notamment les promoteurs de la napine (EP 255 378), de la phaseoline, de la glutenine, de l'héliantinine (WO 92/17580), de l'albumine (WO 98/45460), de l'oélosine (WO 98/45461), de l'ATS1 ou de l'ATS3 (PCT/US98/06978, déposée le 20 octobre 1998). On peut également employer un promoteur inductible avantageusement choisi parmi les promoteurs de phénylalanine ammoniac lyase (PAL), d'HMG-CoA reductase (HMG), de chitinases, de glucanases, d'inhibiteurs de proteinase (PI), de gènes de la famille PR1, de la nopaline synthase (nos) ou du gène vspB (US 5 670 349, Tableau 3), le promoteur HMG2 (US 5 670 349), le promoteur de la beta-galactosidase (ABG1) de pomme ou le promoteur de l'amino cyclopropane carboxylate syntase (ACC synthase) de pomme (WO 98/45445).

« Activateurs de transcription ("enhancer") » : on citera par exemple l'activateur de transcription du virus de la mosaïque du tabac (VMT) décrit dans la demande WO 87/07644, ou du virus etch du tabac (VET) décrit par Carrington & Freed.

« Introns » : séquences d'acide nucléique non traduites. On citera par exemple l'intron 1 du gène d'histone *d'Arabidopsis* tel que décrit dans la demande de brevet WO 97/04114 pour une expression dans les plantes dicotylédones, le premier intron de l'actine de riz décrit dans la demande de brevet WO 99/34005, ou l'intron adh1 de maïs pour une expression dans les plantes monocotylédones.

« Séquence codante » : séquence d'acide nucléique traduite. Elle comprend une séquence codant pour une protéine ou un peptide d'intérêt, éventuellement fusionnée en 5' ou en 3' avec une séquence codant pour un peptide signal ou d'adressage vers un compartiment cellulaire particulier.

« Peptide signal ou d'adressage » : peptides fusionnés à une protéine ou à un peptide d'intérêt dans leur partie N- ou C-terminale, reconnus par la machinerie cellulaire permettant l'adressage de la protéine ou du peptide d'intérêt vers un compartiment cellulaire particulier. Il s'agit en particulier de peptides de transit chloroplastiques permettant l'adressage de la protéine ou du peptide d'intérêt dans les chloroplastes, ou de peptides signaux vers divers compartiments cellulaires, par exemple la vacuole, les mitochondries, le réticulum endoplastique, l'appareil de golgi, etc. Le rôle de telles séquences protéiques sont notamment décrites dans le numéro 38 de le revue Plant molecular Biology (1998) consacré en grande partie aux transports des protéines dans les différents compartiments de la cellule végétale (Sorting of proteins to vacuoles in plant cells pp 127-144 ; the nuclear pore complex pp145-162 ; protein translocation into and across the chloroplastic enveloppe membranes pp 91-207 ; multiple pathways for the targeting of thylakoid proteins in chloroplasts pp 209-221 ; mitochondrial protein import in plants pp 311-338).

« Peptide de transit chloroplastique » : le peptide de transit chloroplastique est codé par une séquence d'acide nucléique en 5' de la séquence d'acide nucléique codant pour une protéine ou un peptide d'intérêt, de manière à permettre l'expression d'une protéine de fusion peptide de transit/protéine (peptide) d'intérêt. Le peptide de transit permet d'adresser la protéine ou le peptide d'intérêt dans les plastes, plus particulièrement les chloroplastes, la protéine de fusion étant clivée entre le peptide de transit et la protéine ou le peptide d'intérêt au passage de la membrane des plastes. Le peptide de transit peut être simple, comme un peptide de transit d'EPSPS (US 5,188,642) ou un peptide de transit de la petite sous-unité de ribulose-biscarboxylase/oxygénase ( ssu RuBisCO) d'une plante, éventuellement comprenant quelques acides aminés de la partie N-terminale de la ssu RuBisCO mature (EP 189 707) ou encore un peptide de transit multiple comprenant un premier peptide de transit de plante fusionné à une partie de la séquence N-terminale d'une protéine mature à localisation plastidiale, fusionnée à un deuxième peptide de transit de plante tel que décrit dans le brevet EP 508 909, et plus particulièrement le peptide de transit optimisé comprenant un peptide de transit de la ssu RuBisCO de tournesol fusionné à 22 acides aminés de l'extrémité N-terminale de la ssu RuBisCO de maïs fusionnée au peptide de transit de la ssu RuBisCO de maïs tel que décrit avec sa séquence codante dans le brevet EP 508 909.

« Peptide signal » : ces séquences peptidiques sont notamment décrites dans le numéro 38 de le revue Plant molecular Biology (1998) consacré en grande partie aux transports des protéines dans les différents compartiments de la cellule végétale (Sorting of proteins to vacuoles in plant cells pp 127-144 ; the nuclear pore complex pp145-162 ; protein translocation into and across the chloroplastic enveloppe membranes pp 91-207 ; multiple pathways for the targeting of thylakoid proteins in chloroplasts pp 209-221 ; mitochondrial protein import in plants pp 311-338). Des peptides d'adressage vers la vacuole sont largements décrits dans la littérature (Neuhaus J.M. and Rogers J.C Sorting of proteins to vacuoles in plant cellsPlant molecular Biology 38 : 127-144, 1998). De préférence, le peptide vacuolaire est le peptide vacuolaire de la protéine décrite dans J.M. Ferullo et al (Plant Molecular Biology 33 : 625-633, 1997), fusionné à la partie C-terminale de la protéine ou du peptide d'intérêt.

« Séquence de régulation terminatrice » : comprenant également les séquences de polyadénylation, on entend toute séquence fonctionnelle dans les cellules végétales ou les plantes qu'elles soient d'origine bactérienne, comme par exemple le terminateur nos *d'Agrobacterium tumefaciens,* d'origine virale, comme par exemple le terminateur du CaMV 35S, ou encore d'origine végétale, comme par exemple un terminateur d'histone tel que décrit dans la demande En 0 633 317.

« Vecteur » : vecteur de clonage et/ou d'expression pour la transformation d'un organisme hôte contenant au moins une cassette d'expression. Le vecteur comprend outre la cassette d'expression, au moins une origine de réplication. Le vecteur peut être constitué par un plasmide, un cosmide, un bactériophage ou un virus, transformés par l'introduction de la cassette d'expression. De tels vecteurs de transformation en fonction de l'organisme hôte à transformer sont bien connus de l'homme du métier et largement décrits dans la littérature. Pour la transformation des cellules végétales ou des plantes, il s'agira notamment d'un virus qui peut être employé pour la transformation des plantes développées et contenant en outre ses propres éléments de réplication et d'expression. De manière préférentielle, le vecteur de transformation des cellules végétales ou des plantes est un plasmide.

### HPP Oxydase

Le procédé selon l'invention peut mettre en oeuvre une séquence d'acide nucléique codant pour une HPP Oxydase, et le polypeptide correspondant. L'HPP oxydase mise en oeuvre est insensible aux inhibiteurs d'HPPD, en particulier aux isoxazoles comme l'isoxaflutole et leurs dikétonitriles, notamment ceux définis précédemment. L'HPP oxydase est notamment une HPP oxydase d'origine bactérienne, par exemple d'*Arthrobacter,* en particulier d'*Arthrobacter globiformis.* L'HPP oxydase est avantageusement une protéine dont la séquence primaire d'acides aminés est représentée par l'identificateur de séquence n°2 (SEQ ID NO 2) ses séquences homologues et ses fragments.

Des séquences protéiques d'HPP oxydases homologues de la SEQ ID NO 2 sont notamment représentées par les SEQ ID NO 4 et 6, ses séquences homologues et ses fragments.

L'HPP oxydase représentée par la SEQ ID NO 4 correspond à l'HPP oxydase de la SEQ ID NO 2 pour laquelle une Glycine est remplacée par une Alanine.

La présente invention concerne également une séquence d'acide nucléique codant pour une HPP oxydase telle que définie ci-dessus.

De manière préférentielle, la séquence codant pour l'HPP oxydase est une séquence d'ADN, notamment ADN génomique ou ADN-c, en particulier une séquence hétérologue ou isolée.

La séquence codant pour une HPP oxydase selon l'invention est notamment choisie parmi les séquences codantes des séquences d'ADN représentées par les SEQ ID NO 1, 3, 5 ou 15, leurs séquences homologues, leurs fragments et les séquences capable de s'hybrider de manière sélective aux SEQ ID 1, 3, 5 ou 15.

La séquence codante de la SEQ ID NO 5 comprend trois mutations en positions 463, 602 et 1511 par rapport à la SEQ ID NO 1 qui sont silencieuses, c'est à dire n'introduisant aucune modification du polypeptide correspondant.

### 4-HPA 1-hydroxylase

Le procédé selon l'invention met également en oeuvre les moyens nécessaires à l'expression de la 4-HPA 1-hydroxylase. Contrairement à ce qui était attendu de la littérature sur l'activité de certains extraits protéiques, il a été constaté que l'activité 4-HPA 1-hydroxylase dans les bactéries, en particulier *Pseudomonas,* résultait de la somme de l'activité de deux enzymes dénommées ci-après HPAH et HPAC.

### HPAH

L'HPAH permet la conversion de l'HPA en métabolite intermédiaire dénommé ci-après métabolite Z dont la structure reste indéterminée. Il peut être envisagé sérieusement que l'HPAH permet l'hydroxylation du noyau aromatique de l'HPA, le métabolite Z se stabilisant sous forme d'une cétone. Cette hypothèse d'activité enzymatique est représentée sur la figure 2.

Le procédé selon l'invention met donc en oeuvre une séquence d'acide nucléique codant pour une HPAH, et le polypeptide correspondant. L'HPAH mise en oeuvre est insensible aux inhibiteurs d'HPPD, en particulier aux isoxazoles comme l'isoxaflutole et leurs dikétonitriles, notamment ceux définis précédemment. L'HPAH est notamment une HPAH d'origine bactérienne, par exemple de *Pseudomonas,* en particulier de *Pseudomonas acidovorans.* L'HPAH est avantageusement une protéine dont la séquence primaire d'acides aminés est représentée par les identificateurs de séquence n°8 et 18 (SEQ ID NO 8 et SEQ ID NO 18) leurs séquences homologues et leurs fragments.

De manière préférentielle, la séquence codant pour l'HPAH est une séquence d'ADN, notamment ADN génomique ou ADN-c, en particulier une séquence hétérologue ou isolée.

La séquence codant pour une HPAH selon l'invention est notamment choisie parmi les parties codantes des séquences représentées par les SEQ ID NO 7 ou 17, leurs séquences homologues, leurs fragments et les séquences capable de s'hybrider de manière sélective aux SEQ ID NO 7 ou 17.

### HPAC

L'HPAC est la deuxième enzyme permettant la conversion du métabolite Z en en homogentisate.

Le procédé selon l'invention met donc en oeuvre une séquence d'acide nucléique codant pour une HPAC, et le polypeptide correspondant., L'HPAC mis en oeuvre est insensible aux inhibiteurs d'HPPD, en particulier aux isoxazoles comme l'isoxaflutole et leurs dikétonitriles, notamment ceux définis précédemment. L'HPAC est notamment une HPAC d'origine bactérienne, par exemple de *Pseudomonas,* en particulier de *Pseudomonas acidovorans.* L'HPAC est avantageusement une protéine dont la séquence primaire d'acides aminés est représentée par l'identificateur de séquence n° 10 (SEQ ID NO 10) ses séquences homologues et ses fragments.

Des séquences protéiques d'HPAC homologues de la SEQ ID NO 10 sont notamment représentées par les SEQ ID NO 12, 14 et 20, leurs séquences homologues et leurs fragments.

La séquence codant pour une HPAC selon l'invention est notamment choisie parmi les parties codantes des séquences représentées par les SEQ ID NO 9, 11, 13 ou 19, leurs séquences homologues, leurs fragments et les séquences capables de s'hybrider de manière sélective aux SEQ ID NO9, 11, 13 ou 19.

### Cassettes d'expression

Le procédé selon l'invention met en oeuvre des cassettes d'expression dont la séquence codante comprend une séquence d'acide nucléique codant pour une HPAH, pour une HPAC, et éventuellement pour une HPP oxydase, telles que définies ci-dessus.

La séquence codante peut également comprendre en 5' ou en 3' une séquence codant pour un peptide signal ou un peptide de transit. De manière avantageuse, la séquence codante comprend en 5' de la séquence codant pour HPP oxydase, HPAH ou HPAC, une séquence codant pour un peptide de transit d'adressage chloroplastique, en particulier un peptide de transit multiple, plus particulièrement le peptide de transit optimisé.

Le procédé selon l'invention peut donc également mettre en oeuvre une protéine de fusion peptide de transit/HPP oxydase, peptide de transit/HPAH ou peptide de trasit/HPAC, la séquence du peptide de transit étant définies précédemment, en particulier la séquence du peptide de transit optimisé tel que décrit dans la demande de brevet EP 508 909.

De manière préférentielle, la séquence de régulation promotrice est choisie parmi les séquences de régulation promotrice permettant une expression constitutive de la séquence codante. Il s'agira en particulier des séquences des promoteurs du CaMV 35S, du CsVMV, de l'actine de riz ou d'histone.

On peut également choisir d'exprimer les séquences codantes selon l'invention à un niveau d'expression voisin du niveau d'expression du gène que l'on cherche à contourner. On pourra employer dans la cassette d'expression selon l'invention une séquence de régulation promotrice choisie parmi les séquences de régulation promotrices d'HPPD de plantes.

Pour l'expression des trois enzymes HPP oxydase, HPAH et HPAC dans une même plante, on pourra choisir les cassettes d'expression des séquences codantes correspondantes, des séquences de régulation promotrices différentes présentant des profils d'expression différents, par leur force et/ou leur localisation dans les différents organes fonctionnels de la plante.

On pourra choisir des séquences de régulation promotrice permettant un gradient d'expression HPAC>HPAH>HPP oxydase ou inversement.

Pour l'expression de l'HPP oxydase, de l'HPAH et de l'HPAC, la séquence de régulation promotrice est avantageusement choisie parmi le groupe comprenant les promoteurs d'HPPD de plante, d'histone H3 ou H4, notamment *d'Arabidopsis* ou de maïs, en particulier ceux décrits dans la demande de brevet EP 507 698, de SSU de RuBisCO de plante, en particulier de tournesol ou de maïs comme décrit dans la demande de brevet WO 99/25842, du CaMV 35S ou du CsVMV, et leurs combinaisons, en particulier les promoteurs hybrides histone/35S tels que décrits dans les exemples de la demande de brevet EP 507 698. Pour une expression dans les plantes monocotylédones, ces séquences de régulation promotrices seront avantageusement associées avec le premier intron de l'actine de riz.

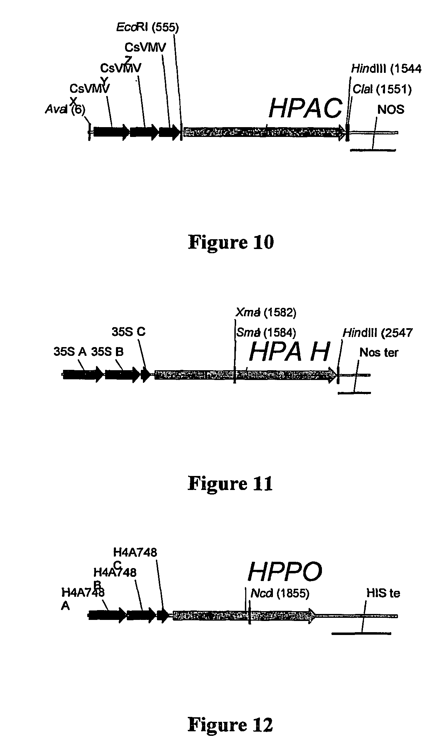

Selon un mode de réalisation de l'invention, la cassette d'expression codant pour une HPP oxydase comprend un promoteur d'histone, une séquence codant pour une HPP oxydase et un terminateur d'histone (Figure 12 ; SEQ ID NO 15).

Selon un autre mode de réalisation de l'invention, la cassette d'expression codant pour une HPAH comprend un promoteur CaMV 35S, une séquence codant pour une HPAH et un terminateur NOS (Figure 11 ; SEQ ID NO 17).

Selon un autre mode de réalisation de l'invention, la cassette d'expression codant pour une HPAC comprend un promoteur CsVMV, une séquence codant pour une HPAC et un terminateur NOS (Figure 10 ; SEQ ID NO 19).

### Vecteurs :

Le procédé selon l'invention peut également mettre en oeuvre un vecteur de clonage et/ou d'expression comprenant des cassettes d'expression telle que définies ci-dessus.

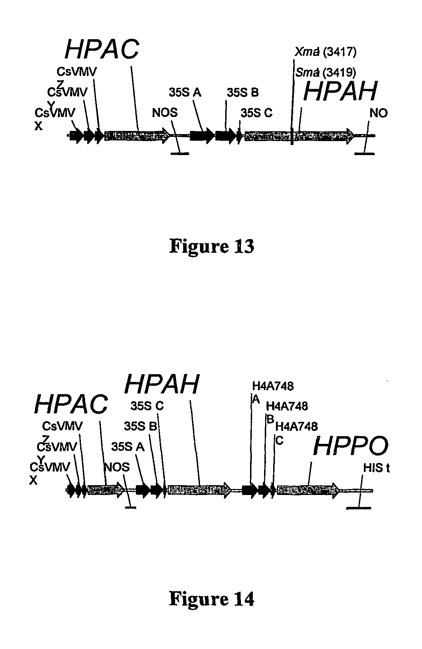

Selon un mode de réalisation particulier de l'invention, le vecteur comprend deux cassettes d'expression comprenant une séquence codante pour une HPAH et pour une HPAC telles que définies précédemment associées deux à deux dans un même vecteur. Un vecteur comprenant une cassette d'expression codant pour l'HPAH et une autre codant pour l'HPAC peut comprendre la combinaison des deux cassettes d'expression définies précédemment (SEQ ID NO 17 et 19). Une telle cassette d'expression est représentée par la Figure 13 et la SEQ ID NO 21.

Selon un autre mode particulier de réalisation de l'invention, le vecteur comprend trois cassettes d'expression selon l'invention, une première cassette d'expression de l'HPP oxydase, une deuxième cassette d'expression de l'HPAH et une troisième cassette d'expression de l'HPAC. Une telle cassette d'expression peut comprendre la combinaison des trois cassettes définies précédement (SEQ ID NO 15, 17 et 19). Un tel vecteur est représenté sur la figure 14 et la SEQ ID NO 22.

Les vecteurs tels que définis ci-dessus peuvent également comprendre des cassettes d'expression d'autres protéines ou peptides d'intérêt.

Lorsque le vecteur comprend plusieurs cassettes d'expression, ces dernières peuvent prendre différentes orientations deux à deux l'une par rapport à l'autre, colinéaires, divergentes ou convergentes.

Les cassettes d'expression d'autres protéines ou peptides d'intérêt comprennent une séquence d'acide nucléique codant pour des protéines ou peptides d'intérêt différents de l'HPP oxydase, de l'HPAH et de l'HPAC définis ci-dessus.

Il peut s'agir de séquences d'un gène codant pour un marqueur de sélection comme d'un gène conférant à la plante transformée de nouvelles propriétés agronomiques, ou d'un gène d'amélioration de la qualité agronomique de la plante transformée.

### Marqueurs de Sélection

Parmi les gènes codant pour des marqueurs de sélection, on peut citer les gènes de résistance aux antibiotiques, les gènes de tolérance aux herbicides (bialaphos, glyphosate ou isoxazoles), des gènes codant pour des enzymes rapporteurs facilement identifiables comme l'enzyme GUS, des gènes codant pour des pigments ou des enzymes régulant la production de pigments dans les cellules transformées. De tels gènes marqueurs de sélection sont notamment décrits dans les demandes de brevet EP 242 236, EP 242 246, GB 2 197 653, WO 91/02071, WO 95/06128, WO 96/38567 ou WO 97/04103. ,

### Gènes d'intérêt

Parmi les gènes conférant de nouvelles propriétés agronomiques aux plantes transformées, on peut citer les gènes conférant une tolérance à certains herbicides, ceux conférant une résistance à certains insectes, ceux conférant une tolérance à certaines maladies, etc. De tels gènes sont notamment décrits dans les demandes de brevet WO 91/02071 et WO 95/06128.

### Tolérance herbicide

La présente invention est particulièrement appropriée pour l'expression de gènes conférant une tolérance à certains herbicides aux cellules végétales et aux plantes monocotylédones transformées. Parmi les gènes conférant une tolérance à certains herbicides, on peut citer le gène Bar conférant une tolérance au bialaphos, le gène codant pour une EPSPS appropriée conférant une résistance aux herbicides ayant l'EPSPS comme cible comme le glyphosate et ses sels (US 4,535,060, US 4,769,061, US 5,094,945, US 4,940,835, US 5,188,642, US 4,971,908, US 5,145,783, US 5,310,667, US 5,312,910, US 5,627,061, US 5,633,435, FR 2 736 926), le gène codant pour la glyphosate oxydoréductase (US 5,463,175), ou encore un gène codant pour une HPPD conférant une tolérance aux herbicides ayant pour cible l'HPPD comme les isoxazoles, notamment l'isoxafutole (FR 95 06800, FR 95 13570), les dicétonitriles (EP 496 630, EP 496 631) ou les tricétones, notamment la sulcotrione (EP 625 505, EP 625 508, US 5,506,195). De tels gènes codant pour une HPPD conférant une tolérance aux herbicides ayant pour cible l'HPPD sont décrits dans la demande de brevet WO 96/38567.

Parmi les gènes codant pour une EPSPS appropriée conférant une résistance aux herbicides ayant l'EPSPS comme cible, on citera plus particulièrement le gène codant pour une EPSPS végétale, en particulier de maïs, présentant deux mutations 102 et 106, décrit dans la demande de brevet FR 2 736 926, dénommé ci-dessous EPSPS double mutant, ou encore le gène codant pour une EPSPS isolée d'Agrobacterium décrit par les séquences ID 2 et ID 3 du brevet US 5,633,435, dénommé ci-dessous CP4.

Parmi les gènes codant pour une HPPD conférant une tolérance aux herbicides ayant pour cible l'HPPD, on citera plus particulièrement l'HPPD de Pseudomonas et celle d'Arabidopsis, décrites dans la demande de brevet WO 96/38567.

Dans les cas des gènes codant pour EPSPS ou HPPD, et plus particulièrement pour les gènes ci-dessus, la séquence codant pour ces enzymes est avantageusement précédée par une séquence codant pour un peptide de transit, en particulier pour le peptide de transit dit peptide de transit optimisé décrit dans les brevets US 5,510,471 ou US 5,633,448.

### Résistance aux Insectes

Parmi les protéines d'intérêt conférant de nouvelles propriétés de résistance aux insectes, on citera plus particulièrement les protéines *Bt* largement décrites dans la littérature et bien connues de l'homme du métier. On citera aussi les protéines extraites de bactéries comme *Photorabdus* (WO 97/17432 & WO 98/08932).

### Résistance aux Maladies

Parmi les protéines ou peptides d'intérêt conférant de nouvelles propriétés de résistance aux maladies on citera notamment les chitinases, les glucanases, l'oxalate oxydase, toutes ces protéines et leurs séquences codantes étant largement décrites dans la littérature, ou encore les peptides antibactériens et/ou antifongiques, en particulier les peptides de moins de 100 acides aminés riches en cystéines comme les thionines ou défensines de plantes, et plus particulièrement les peptides lytiques de toutes origines comprenant un ou plusieurs ponts disulfures entre les cystéines et des régions comprenant des acides aminés basiques, notamment les peptides lytiques suivants : l'androctonine (WO 97/30082 et PCT/FR98/01814, déposée le 18 août 1998) ou la drosomicine (PCT/FR98/01462, déposée le 8 juillet 1998).

Selon un mode particulier de réalisation de l'invention, la protéine ou peptide d'intérêt est choisi parmi les peptides éliciteurs fongiques, en particulier les élicitines (Kamoun & al., 1993 ; Panabières & al., 1995).

### Modification de la qualité

On peut également citer les gènes modifiant la constitution des plantes modifiées, en particulier la teneur et la qualité de certains acides gras essentiels (EP 666 918) ou encore la teneur et la qualité des protéines, en particuliers dans les feuilles et/ou les graines desdites plantes. On citera en particulier les gènes codant pour des protéines enrichies en acides aminés soufrés (Korit, A.A. & al., Eur. J. Biochem. (1991) 195, 329-334 ; WO 98/20133 ; WO 97/41239 ; WO 95/31554 ; WO 94/20828 ; WO 92/14822). Ces protéines enrichies en acides aminés soufrés auront également pour fonction de piéger et stocker la cystéine et/ou la méthionine excédentaire, permettant d'éviter les problèmes éventuels de toxicité liés à une surproduction de ces acides aminés soufrés en les piégeant. On peut citer également des gènes codant pour des peptides riches en acides aminés soufrés et plus particulièrement en cystéines, les dits peptides ayant également une activité antibactérienne et/ou antifongique. On citera plus particulièrement les défensines de plantes, de même que les peptides lytiques de toute origine, et plus particulièrement les peptides lytiques suivants : l'androctonine (WO 97/30082 et PCT/FR98/01814, déposée le 18 août 1998) ou la drosomicine (PCT/FR98/01462, déposée le 8 juillet 1998.

### Cellules végétales et plantes transgéniques

La présente invention concerne également des cellules végétales et des plantes transformées comprenant une première séquence d'acide nucléique codant pour une enzyme insensible à un herbicide inhibiteur d'HPPD convertissant l'HPP en 4-HPA, une seconde séquence d'acide nucléique codant pour une HPAH fonctionnelle insensible au dit herbicide inhibiteur d'HPPD comprenant une séquence d'acides aminés sélectionnée parmi le groupe constitué de la SEQ ID NO 8, et de ses fragments, et une troisième séquence d'acide nucléique codant pour une HPAC fonctionnelle insensible au dit herbicide inhibiteur d'HPPD comprenant la séquence d'acides aminés sélectionnée parmi le groupe constitué de SEQ ID NO 10, SEQ ID NO 12 et SEQ ID NO 14, de leurs fragments, et pour laquelle au moins une des enzymes HPAH ou HPAC est hétérologue.

La présente invention concerne également des cellules végétales et des plantes transformées comprenant une première séquence d'acide nucléique codant pour une enzyme insensible à un herbicide inhibiteur d'HPPD convertissant l'HPP en 4-HPA, une seconde séquence d'acide nucléique codant pour une HPAH fonctionnelle insensible au dit herbicide inhibiteur d'HPPD comprenant une séquence d'acide nucléique sélectionnée parmi le groupe constitué des séquences codantes des SEQ ID NO 7 et SEQ ID NO 17, de leurs fragments et des séquences capables de s'hybrider de manière sélective dans des conditions de milieu très sévères aux dites SEQ ID NO 7 ou 17, et une troisième séquence d'acide nucléique codant pour une HPAC fonctionnelle insensible au dit herbicide inhibiteur d'HPPD comprenant une séquence d'acide nucléique sélectionnée parmi le groupe constitué des séquences codantes des SEQ ID NO 9, SEQ ID NO 11, SEQ ID NO 13 et SEQ ID NO 19 , de leurs fragments et des séquences capables de s'hybrider de manière sélective dans des conditions de milieu très sévères aux dites SEQ ID NO 9, 11, 13 ou 19, et pour laquelle au moins une des enzymes HPAH ou HPAC est hétérologue.

Selon un mode particulier de réalisation de l'invention, l'enzyme insensible à un herbicide inhibiteur d'HPPD convertissant l'HPP en 4-HPA est une HPP oxydase.

Selon un autre mode particulier de réalisation de l'invention, l'HPP oxydase comprend la séquence d'acides aminés sélectionnée parmi le groupe constitué des SEQ ID NO 2, SEQ ID NO 4 et SEQ ID NO 6, et de leurs fragments.

Selon un autre mode particulier de réalisation de l'invention, l'HPP oxydase est codée par une séquence d'acide nucléique comprenant une séquence sélectionnée parmi le groupe constitué des séquences codantes des SEQ ID NO 1, SEQ ID NO 3, SEQ ID NO 5 et SEQ ID NO 15, de leurs fragments et des séquences capables de s'hybrider de manière sélective dans des conditions de milieu très sévères aux dites SEQ ID NO 1, 3, 5, ou 15.

Les cellules végétales ou les plantes selon l'invention telles que définis ci-dessus peuvent également comprendre des cassettes d'expression d'autres protéines ou peptides d'intérêt définies précédemment.

De manière préférentielle, les cassettes d'expression sont intégrées de manière stable dans le génome des cellules végétales ou des plantes. Plus préférentiellement, les plantes selon l'invention sont fertiles, les cassettes d'expression selon l'invention étant transférées à leur descendance.

La présente invention concerne également des graines de plantes transgéniques ci-dessus, caractérisées en ce qu'elles comprennent une cellule végétale selon l'invention.

Les différentes cassettes d'expression dans les plantes transformées selon l'invention peuvent provenir soit de la même plante transformée parente, et dans ce cas la plante est issue d'un seul procédé de transformation/régénération avec les différentes cassettes d'expression contenues dans un même vecteur ou par co-transformation au moyen de plusieurs vecteurs. Elle peut également être obtenue par le croisement de plantes parentes contenant chacune au moins une cassette d'expression selon l'invention.

### Transformation des cellules végétales et des plantes

L'invention a encore pour objet un procédé de transformation des cellules végétales et des plantes par introduction d'au moins une cassette d'expression comprenant une séquence d'acide nucléique codant pour une HPAH fonctionnelle insensible au dit herbicide inhibiteur d'HPPD comprenant la séquence d'acides aminés sélectionnée parmi le groupe constitué de la SEQ ID NO 8 et de ses fragments, ou au moins une séquence d'acide nucléique codant pour une HPAC fonctionnelle insensible au dit herbicide inhibiteur d'HPPD comprenant la séquence d'acides aminés sélectionnée parmi le groupe constitué des SEQ ID NO 10, SEQ ID NO 12 et SEQ ID NO 14, et de leurs fragments.
L'invention a encore pour objet un procédé de transformation des cellules végétales et des plantes par introduction d'au moins une cassette d'expression comprenant une séquence d'acide nucléique codant pour une HPAH fonctionnelle insensible au dit herbicide inhibiteur d'HPPD comprenant la séquence d'acide nucléique sélectionnée parmi le groupe constitué des séquences codantes SEQ ID NO 7 et SEQ ID NO 17, de leurs fragments et des séquences capables de s'hybrider de manière sélective dans des conditions de milieu très sévères aux dites SEQ ID NO 7 ou 17, ou au moins une séquence d'acide nucléique codant pour une HPAC fonctionnelle insensible au dit herbicide inhibiteur d'HPPD comprenant la séquence d'acide nucléique sélectionnée parmi le groupe constitué des séquences codantes SEQ ID NO 9, SEQ ID NO 11, SEQ ID NO 13 et SEQ ID 19, de leurs fragments et des séquences capables de s'hybrider de manière sélective dans des conditions de milieu très sévères aux dites SEQ ID NO 9, 11, 13 ou 19..

Une série de méthodes consiste à bombarder des cellules, des protoplastes ou des tissus avec des particules auxquelles sont accrochées les séquences d'ADN. Une autre série de méthodes consiste à utiliser comme moyen de transfert dans la plante un gène chimère inséré dans un plasmide Ti d'Agrobacterium tumefaciens ou Ri d'Agrobacterium rhizogenes. D'autres méthodes peuvent être utilisées telles que la micro-injection ou l'électroporation, ou encore la précipitation directe au moyen de PEG. L'homme du métier fera le choix de la méthode appropriée en fonction de la nature de l'organisme hôte, en particulier de la cellule végétale ou de la plante.

Lorsque l'on souhaite introduire plusieurs séquences d'acide nucléique ou cassettes d'expression, on peut le faire au moyen d'un seul vecteur selon l'invention comprenant les différentes cassettes d'expression. Elles peuvent également être introduites dans l'organisme hôte par co-transformation au moyen de plusieurs vecteurs, chacun comprenant au moins une cassette d'expression.

D'une manière générale, les plantes transgéniques selon l'invention sont obtenues par transformation de cellules végétales puis régénération d'une plante, de préférence fertile à partir de la cellule transformée. La régénération est obtenue par tout procédé approprié qui dépend de la nature de l'espèce, comme par exemple décrit dans les références ci-dessus. Pour les procédés de transformation des cellules végétales et de régénération des plantes, on citera notamment les brevets et demandes de brevet suivants: US 4,459,355, US 4,536,475, US 5,464,763, US 5,177,010, US 5,187,073, EP 267,159, EP 604 662, EP 672 752, US 4,945,050, US 5,036,006, US 5,100,792, US 5,371,014, US 5,478,744, US 5,179,022, US 5,565,346, US 5,484,956, US 5,508,468, US 5,538,877, US 5,554,798, US 5,489,520, US 5,510,318, US 5,204,253, US 5,405,765, EP 442 174, EP 486 233, EP 486 234, EP 539 563, EP 674 725, WO 91/02071 et WO 95/06128.

### Désherbage sélectif

L'invention a aussi pour objet un procédé de désherbage sélectif de plantes, notamment de cultures, à l'aide d'un inhibiteur de l'HPPD notamment un herbicide définit auparavant, caractérisé en ce qu'on applique cet herbicide sur des plantes transformées selon l'invention, tant en présemis, en prélevée qu'en postlevée de la culture.

La présente invention concerne également un procédé de contrôle des mauvaises herbes dans une surface d'un champ comprenant des graines ou des plantes transformées selon l'invention, lequel procédé comprend l'application dans la dite surface du champ d'une dose toxique pour les dites mauvaises herbes d'un herbicide inhibiteur d'HPPD, sans toutefois affecter de manière substantielle les graines ou plantes transformée selon l'invention.

La présente invention concerne également un procédé de culture des plantes transformées selon l'invention lequel procédé comprend le semis des graines des dites plantes transformées dans une surface d'un champ approprié pour la culture des dites plantes, l'application sur la dite surface du dit champ une dose toxique pour les mauvaises herbes d'un herbicide ayant pour cible l'HPPD défini ci-dessus en cas de présence de mauvaises herbes, sans affecter de manière substantielle les dites graines ou les dites plantes transformées, puis la récolte des plantes cultivées lorsqu'elles arrivent à la maturité souhaitée et éventuellement la séparation des graines des plantes récoltées.

Par « sans affecter de manière substantielle les dites graines ou les dites plantes transformées », on entend selon l'invention que les plantes transformées selon l'invention soumises à une application d'une dose d'herbicide toxique pour les mauvaises herbes, présentent une phytotoxicité légère ou nulle. Par dose toxique pour les mauvaises herbes, on entend selon l'invention une dose d'application de l'herbicide pour laquelle les mauvaises herbes sont tuées. Par phytotoxicité légère on entend selon invention un pourcentage de feuilles blanchies inférieur à 25%, préférentiellement inférieur à 10%, plus préférentiellement inférieur à 5%. Il est entendu également selon la présente invention que l'application de la même dose toxique sur une plante autrement comparable non transformée, c'est à dire ne comprenant pas au moins une cassette d'expression selon l'invention, conduirait à observer sur ladite plante des symptômes de phytotoxicité suppérieurs à ceux observés pour la plante transformée selon l'invention.

Dans les deux procédés ci-dessus, l'application de l'herbicide ayant pour cible l'HPPD peut être faite selon l'invention, tant en présemis, en prélevée qu'en postlevée de la culture.

Par herbicide au sens de la présente invention on entend une matière active herbicide seule ou associée à un additif qui modifie son efficacité comme par exemple un agent augmentant l'activité (synergiste) ou limitant l'activité (en anglais safener). Les herbicides inhibiteurs d'HPPD sont en particulier définis auparavant. Bien entendu, pour leur application pratique, les herbicides ci-dessus sont associée de manière en soi connue aux adjuvants de formulations utilisés habituellement en agrochimie

Lorsque la plante transformée selon l'invention comprend un autre gène de tolérance à un autre herbicide (comme par exemple un gène codant pour une EPSPS mutée ou non conférant à la plante une tolérance au glyphosate), ou lorsque la plante transformée est naturellement insensible à un autre herbicides, le procédé selon l'invention peut comprendre l'application simultanée ou décalée dans le temps d'un inhibiteur d'HPPD en association avec ledit herbicide, par exemple le glyphosate.

Les différents aspects de invention seront mieux compris à l'aide des exemples expérimentaux ci-dessous.

Toutes les méthodes ou opérations décrites ci-dessous dans ces exemples sont données à titre d'exemples et correspondent à un choix, effectué parmi les différentes méthodes disponibles pour parvenir au même résultat. Ce choix n'a aucune incidence sur la qualité du résultat et par conséquent, toute méthode adaptée peut être utilisée par l'homme de l'art pour parvenir au même résultat. La plupart des méthodes d'ingénierie des fragments d'ADN sont décrites dans Coligan *et al.* (1995), Ausubel *et al.* (1995) ; Maniatis *et al.* (1982), Sambrook *et al.*

### Exemple I: IDENTIFICATION DU GENE CODANT L'HPP OXYDASE D'ARTHROBACTER GLOBIFORMIS

L'HPP oxydase (HPPO) convertit l'HPP en 4-HPA par une réaction de décarboxylation. Cette enzyme catalyse donc la première activité enzymatique nécessaire pour la construction de la voie métabolique contournant l'HPPD. L'activité HPP oxydase a été caractérisée dans des extraits bruts de *Rhodococcus erythropolis* S1 (Suemori *et al.,* 1995) ou dans un extrait partiellement purifiée d*'Arthrobacter globiformis* (Blakley, 1977). A notre connaissance, la protéine n'a pas été purifiée. Afin de pouvoir introduire cette activité enzymatique dans la plante, il, est nécessaire d'en identifier le gène. Différentes approches sont envisageables: (1) la mutagenèse insertionnelle et donc l'identification du gène par la perte de l'activité enzymatique, (2) la complémentation fonctionnelle d'un microorganisme en utilisant une banque génomique, (3) la purification de la protéine pour remonter à la séquence nucléique.
Les trois approches furent utilisées. La complémentation fonctionnelle et la mutagenèse insertionnelle seront peu développées, ces techniques n'ayant pas permis d'identifier le gène HPPO.

### I.1 Matériels et Méthodes

### I.1.1- Les conditions de culture

### I.1.1.1- Les milieux riches

Le milieu Luria-Bertani (LB; commercialisé par Bio101) est utilisé pour cultiver les bactéries *(E. coli, P. fluorescens)* lors des expériences de biologie moléculaire. Pour la culture d'*A. globiformis* on préfèrera le milieu Columbia-ANC enrichi avec 5%de sang de mouton (BioMérieux). Ce milieu riche contient deux antibiotiques (acide nalidixique et colimycine) inhibiteurs des germes à Gram négatif. Bien que les trois bactéries poussent sur milieu riche à 37°C, on cultive en général *A. globiformis* et *P. fluorescens* à 29°C.

### I.1.1.2- Le milieu de culture M^{Ag}

Le milieu de culture décrit par Blakley (1977) précipite, il faut donc le filtrer avant utilisation. Nous avons changé progressivement le milieu afin d'atteindre un milieu " minimal " optimal. Les facteurs considérés sont la vitesse de croissance d'*A*. *globiformis* et l'activité enzymatique de l'HPPO. Le milieu retenu (M*^{Ag}*) est un milieu M9 (Maniatis *et al.,* 1982) légèrement modifié: Na₂HPO₄ 12 H₂O (6 g/L); KH₂PO₄ (3 g/L); NH₄Cl (1 g/L); NaCl (0.5 g/L); CaCl₂ (6 mg/L); FeSO₄ 7 H₂O (6 mg/L); extrait de levure (20 mg/L); et enfin le substrat (HPP ou tyrosine ou citrate) à la concentration 1g/L. Le milieu est autoclavé. Avant utilisation, 1 mL de MgSO₄ 1 M stérile est ajouté par litre de milieu.

Ce milieu minimum est aussi utilisé pour cultiver *P. fluorescens.*

### I.1.2- Construction d'une banque génomique d'Arthrobacter globiformis

Il n'existe pas de technique fiable permettant de faire une banque de cDNA bactériens complets. Nous avons donc décidé de créer une banque génomique d'*Arthrobacter globiformis.* Pour la réaliser, nous choisissons le système cosmidique. La banque cosmidique fut réalisée pour les expériences de complémentation fonctionnelle, puis fut utilisée plus tard pour rechercher le ou les cosmides contenant le gène *hpp*O.

### I.1.2.1- Le vecteur cosmidique pLAFR5

### I.1.2.1.1- Description du vecteur

Nous choisissons le vecteur cosmidique conjugatif pLAFR-5 (Keen *et al.,* 1988) qui peut recevoir un insert d'environ 20 kb. Pourvu d'une origine de transfert et d'une origine de réplication à large spectre d'hôte à Gram négatif, il peut être transmis à d'autres genres bactériens par conjugaison tri-parentale ce qui peut être utile pour tester la complémentation fonctionnelle chez différents genres bactériens. Il confère une résistance à la tétracycline.

### I.1.2.1.2- Préparation du vecteur

Le plasmide pLAFR-5 est purifié par un protocole de lyse alcaline (Maniatis *et al.,* 1982), traité à la RNAse puis digéré par *Bam* HI et *Sca* I. La digestion par *Bam* HI permet d'ouvrir le site dans lequel seront "ligués" les inserts d'ADN génomique digéré par Sau3A. La digestion par *Sca* I permet de libérer les sites *cos* qui permettent l'encapsidation. Après extraction phénolique puis chloroformique, l'ADN est précipité à l'éthanol. L'ADN sec est solubilisé dans l'eau. Le vecteur ainsi préparé est conservé à -20°C.

### I.1.2.1- Préparation de l'ADN génomique d'A. globiformis

Une culture de 24 heures (200 mL, 180 rpm, 29°C) réalisée dans le milieu (200 mL) décrit par Blakley (1977) est centrifugée à 3000 g à 4°C pendant 15 minutes. Le culot cellulaire, repris par 10 mL de solution de lyse (TE pH 8 ; 0,5 % SDS; 1 mg protéinase K), est incubé à 37°C au bain-marie avec une agitation douce toutes les 20 minutes. Au bout de 90 minutes, la suspension de cellules lysées est versée dans un tube JA-20 en polypropylène. On ajoute alors 10 mL de phénol/ chloroforme/ isoamylalcool (25/24/1) puis on centrifuge à 6 000 g pendant 15 minutes à 4°C. Le surnageant est alors transféré dans un nouveau tube JA20 auquel on ajoute 1,8 mL d'acétate d'ammonium 10 M et 10 mL d'isopropanol. Après centrifugation à 20 000 g pendant 20 minutes à 4°C, le culot est rincé à l'éthanol 70 %. Le culot sec est repris avec 1 mL TE pH 8 puis transféré dans un tube Eppendorf de 2 mL auquel 10 µL de RNAse (10 mg.mL⁻¹) sont additionnés. Après 30 min à 37°C, 800 µL de phénol/ chloroforme/ isoamylalcool sont rajoutés. Après centrifugation, le surnageant est transféré dans un nouveau tube Eppendorf et extrait avec 0,8 mL de chloroforme. Le surnageant est alors transféré dans un dernier tube Eppendorf auquel 200 µL d'acétate d'ammonium 10 M et 800 µL d'isopropanol sont ajoutés. Après centrifugation, le culot est rincé à l'éthanol 70% puis, une fois sec, repris dans 500 µL d'eau. L'ADN génomique est alors stocké à -20°C.

### I.1.2.3- Digestion ménagée de l'ADN génomique d'A. globiformis

Seuls les cosmides faisant 40-45 kb peuvent être encapsidés. Le vecteur faisant 21,5 kb, les inserts d'ADN génomique d'*A*. *globiformis* doivent avoir une taille comprise entre 19 et 22 kb. Ces fragments sont obtenus en réalisant une digestion ménagée de l'ADN génomique *d'Arthrobacter globiformis.* Pour définir les conditions optimales de la digestion ménagée nous réalisons des digestions de l'ADN génomique d'A. *globiformis* avec des quantités variables d'enzyme de restriction *Sau* 3A. Il apparaît que la meilleure condition de digestion utilise 0,08 unité enzymatique *Sau* 3A pendant 30 minutes à 37°C. L'ADN génomique ainsi digéré présente une taille comprise entre 15 et 22 kb. L'ADN génomique ainsi digéré est extrait au phénol puis au chloroforme et enfin précipité à l'éthanol.

### I.1.2.4- Ligation de l'ADN génomique d'A. globiformis dans le vecteur cosmidique

La réaction de ligation se fait dans un volume final de 10 µL, contenant 500 ng de pLAFR-5 digéré par *Bam* HI et *Sca* I, 650 ng d'ADN génomique digéré par *Sau* 3A, 320 unités de T₄ DNA ligase (N.E.B.) et 5 mM d'ATP. La ligation se déroule à 12°C pendant la nuit (environ 16 heures). Les 5 mM d'ATP permettent d'éviter les ligations entre les extrémités franches (*Sca* I) (Feretti & Sgaramella, 1981) de telle sorte que les dimères de vecteurs n'ayant pas d'insert ne puissent pas s'encapsider dans la tête des phages λ.

### I.1.2.5- Encapsidation des cosmides et amplification de la banque cosmidique

L'encapsidation des cosmides, réalisée en utilisant le kit GIGAPACK II XL (Stratagène) en respectant les instructions du fournisseur, assure une efficacité de transfection supérieure à celles obtenues avec les techniques classiques de transformation. Pour amplifier la banque cosmidique, Keen *et al.* (1988) conseillent d'utiliser les *Escherichia coli* DH-1 et HB101. En effet, lorsque ces souches sont cultivées sur maltose, elles produisent une protéine membranaire qui permet une meilleure fixation du phage et donc une transfection plus efficace des cosmides. La banque, amplifiée en suivant les recommandations de Stratagène, est conservée à -80°C. Pour évaluer la banque cosmidique, l'ADN plasmidique isolé d'une trentaine de clone est digéré par *Apa* I ou *Eco* RI. Les profils de restriction sont observés sur gel d'agarose à 0,8%.

### I.1.3- Purification de l'HPP oxydase

### I.1.3.1- Test colorimétrique de l'activité HPP oxydase

Afin de pouvoir contrôler les étapes de purification, l'activité HPP oxydase est suivie en utilisant le test colorimétrique décrit par Blakley (1977). La réaction enzymatique est stoppée par l'ajout de 2,4 dinitrophénylhydrazine (2,4-DNPH), en solution dans l'HCl 2 M. La 2,4-DNPH réagit avec la fonction cétone en alpha d'une fonction carboxylique (ex : l'HPP). Il se forme ainsi une hydrazone que l'on peut révéler en alcalinisant le milieu. Lorsque l'HPP est convertit en totalité en 4-HPA pendant la réaction enzymatique, l'hydrazone ne peut pas se former, on obtient donc, en milieu basique, la couleur jaune caractéristique de la 2,4-DHPA. Si l'HPP n'est pas entièrement convertit en 4-HPA lors de la réaction enzymatique, la formation d'hydrazone est possible. Ces hydrazones prennent une couleur brune en milieu basique. Une variation de coloration entre ces deux extrêmes est obtenue en fonction de la quantité d'HPP consommé. Les mesures d'absorption sont faites à 445 ou 450 nm. Afin de rendre ce test plus facilement manipulable, nous l'avons adapté au format microplaque à 96 puits. Le mélange réactionnel comprend GSH (900 µM); HPP (135 µM); TPP (1,8 mM); MgCl₂ (4,5 mM); FAD (4 µM); tampon phosphate de potassium (90 mM) pH 7,4. Le mélange est conservé sur glace. Dans chaque puits on dépose 50 µL de la fraction à tester et 150 µL de mélange réactionnel. Après 20 min à 30°C, la réaction enzymatique est stoppée avec 13 µL de solution 2,4-DNPH (0.1% dans HCl 2 M). Laisser réagir 20 min à température ambiante. On révèle la formation d'hydrazone en ajoutant 13 µL de solution NaOH 10 M. Pour réaliser la gamme étalon, des mélanges réactionnels avec des concentrations variables d'HPP sont préparés. Les 50 µL de fraction protéique sont remplacés par 50 µL de tampon d'extraction de la protéine. La courbe étalon est réalisée pour chaque nouvelle solution de 2,4-DNPH (la solution de 2,4-DNPH est stable 6 mois à l'obscurité). L'avantage de ce test est sa rapidité, sa simplicité, mais il a le défaut de mesurer une disparition de substrat et non pas une apparition de produit. En outre, la possibilité d'avoir des faux positifs existe : une activité tyrosine amino-transférase donnera le même résultat que l'activité de l'HPPO. En effet, dans les deux cas, la fonction cétone a disparu. Nous avons donc développé une méthode HPLC rapide et sensible qui permette de confirmer la production de 4-HPA.

### I.1.3.2- Test d'activité analysé par HPLC

Une méthode HPLC a été mise au point en utilisant une petite colonne Sphérisorb ODS2 50 x 4,6 mm et de granulométrie 3 µm. La chromatographie est réalisée en isocratique A: 90%; B: 10% (où tampon A: H₂O 0,1% TFA et tampon B: acétonitrile), débit 0,8 mL.min⁻¹ et l'élution est suivie à 230 nm. Dans ces conditions, il est possible de séparer le 4-HPA, l'HGA, le 3,4-DHPA et HPP en 5 minutes après l'injection. La colonne a été réalisée à façon par Merck.

### I.1.3.3- Purification de la protéine

Lors de la mise au point de ce protocole, un souci de simplicité a été recherché.

### I.1.3.3.1 - Tests préliminaires

Les tests préliminaires ont pour but de déterminer l'influence de composés (NaCl, KCl, propanol-1, éthylène glycol, etc ...) et du pH sur l'activité enzymatique. Les réactions sont réalisées avec des extraits bruts d'*A*. *globiformis* cultivé sur milieu M*^{Ag}* contenant de la tyrosine comme seule source de carbone (M*^{Ag}*-tyrosine). Le composé à tester est ajouté dans le milieu réactionnel. Pour mesurer l'influence du pH sur l'activité enzymatique de HPPO, différents tampons phosphate sont réalisés.

### I.1.3.3.2- Protocole de purification

La souche d*'Arthrobacter globiformis* est étalée sur milieu gélosé LB ou sur milieu gélosé Columbia-ANC. Après 16 heures de culture à 29°C, une colonie est prélevée et ensemencée dans 5 mL de milieu LB, en croissance pendant 8 heures à 29°C, 180 rpm. 50 µL de cette préculture sont alors inoculés dans 1,5 L de milieu M*^{Ag}*-Tyrosine ou M*^{Ag}*-HPP, la culture est alors réalisée à 29°C, 180 rpm, dans des Erlenmeyer à ailettes (Belco). Après 48 heures de culture, les cellules sont collectées par centrifugation à 5 000 g pendant 15 minutes à 4°C. Les cellules sont remises en suspension dans du tampon Tris-HCl 50 mM pH 7,4 puis centrifugées comme précédemment. Le culot est repris dans 2 mL de tampon Tris-HCl 50 mM pH 7,4. Les cellules sont soniquées (Vibra Cell, Sonic Materials INC., Connecticut, USA) pendant 15 minutes, puissance 4, pulse de 30%, dans la glace fondante. Les débris insolubles sont éliminés par une centrifugation de 25 min à 20 000 g, 4°C. Le surnageant est récupéré, il constitue "l'extrait brut". Il peut être congelé dans l'azote liquide puis conservé à -80°C (pendant 6 mois sans perte apparente d'activité). L'extrait brut est chargé, sans dessalage préalable, sur une colonne échangeuse faible d'anions 'EMD/DEAE 650 S' (Merck) équilibrée en tampon phosphate 50 mM pH 7,4. L'élution de l'activité enzymatique est obtenue en appliquant un gradient de concentration de NaCl (en solution dans un tampon phosphate 50 mM pH 7,4. Les fractions contenant l'activité enzymatique sont rassemblées. La solution protéique obtenue est diluée d'un facteur 2,7 avec du tampon phosphate 50 mM pH 7,4. Les protéines sont alors chargées sur une colonne (XK16, Pharmacia) échangeuse forte d'anions 'source Q' (30 mL, Pharmacia) préalablement équilibrée avec un tampon phosphate 50 mM pH 7,4. Les fractions protéiques intéressantes, identifiées par l'activité enzymatique, sont rassemblées puis concentrées sur membrane UVIKON 10 kDa. L'extrait protéique résultant est alors dessalé par la technique de gel-filtration en utilisant une colonne 'PD10' (Pharmacia) équilibrée en tampon phosphate 10 mM pH 7,4 et élué avec ce même tampon. Les protéines sont alors déposées sur une colonne (XK9/15, Pharmacia) d'hydroxyapatite (2 mL; Hydroxyapatite DNA grade Bio-Gel^{®}HTP gel; Bio-Rad) équilibrée avec 10 mM tampon phosphate pH 7,4. On élue l'activité enzymatique en appliquant un gradient phosphate. Les fractions contenant l'activité enzymatique sont rassemblées et concentrées. On conserve les protéines actives lorsque la concentration protéique est supérieure à 1 mg/mL en ajoutant du FAD, GSH et glycérol afin d'obtenir les concentrations finales suivantes : 27 µM FAD, 110 µM GSH, 0,8% glycérol. Les protéines ainsi préparées peuvent être congelées à -80°C pendant au moins 6 mois.

### I.1.3.3.3- Dosage des protéines

Le dosage des protéines se fait selon la méthode de Bradford (1976) en utilisant la γ-globuline pour standard.

### I.1.3.3.4- Coloration des gels de protéines

Les fractions protéiques sont analysées sur gel de polyacrylamide à 10 % selon la méthode de Laemmli (1970). Après migration, les protéines du gel sont colorées soit en utilisant la méthode au Bleu de Coomassie (Chua, 1980) soit en utilisant la méthode au nitrate d'argent (Schoenle *et al.,* 1984).

### I.1.4- Microséquençage protéique de l'extrémité N-terminale et de peptides internes

Le microséquençage de la protéine est réalisé en utilisant la méthode d'Edman (Laursen, 1971). Pour obtenir les meilleurs résultats lors du séquençage, le gel est préparé le jour même.

### I.1.4.1- Préparation du gel d'acrylamide et son électrophorèse

Les gels (8,5%, 10% ou 12%) sont réalisés selon la méthode de Laemmli (1970) en utilisant le système de minigels d'Hoefer^{®}. Les protéines sont diluées au tiers avec une solution 'bleu de dépôt dénaturant' (Tris-HCl 150 mM pH 6,8; SDS 4 %; β-mercaptoéthanol 2% (v/v); glycérol 3,3% (v/v); bleu de Bromophénol 0,03% qsp 10 mL d'eau milliQ). Après avoir été bouillies 5 minutes, les protéines sont chargées sur le gel d'acrylamide. La migration est réalisée à température ambiante en utilisant un tampon de migration dénaturant (Tris base 25 mM; glycine 250 mM; β-mercaptoéthanol 0,014% (v/v); SDS 0,1 %) et en appliquant une intensité de 15 mA par gel.

### I.1.4.2- Préparatifs pour le séquençage de l'extrémité N-terminale

Afin de pouvoir réaliser le séquençage de l'extrémité N-terminale, le gel est transféré sur membrane PVDF (PROBLOTT^{®} - Applied Biosystems) en utilisant la technique de transfert semi-sec. L'électrotransfert des polypeptides se fait en 30 minutes à 300 mA avec l'appareil 'Semy Dry Electroblotter' (Bio-Rad) et dans un milieu à base de CAPS (tampon de transfert: CAPS 10 mM pH 11,0; méthanol 10% (v/v)). Le tampon de transfert ne contient pas de glycine qui risquerait de "polluer" le séquençage. Après le transfert, la membrane est rincée quelques secondes à l'eau milliQ. Elle est alors immergée quelques secondes dans une solution de coloration à base d'amido-schwarz (Aldrich; ref: 19,524-3). La solution est constituée de méthanol 45% (v/v), d'acide acétique 1% (v/v), d'amido-schwarz 0,1% (m/v) et d'eau 63,9% (v/v). Lorsque la bande correspondante à la protéine d'intérêt est visible, la membrane est rincée abondamment à l'eau milliQ puis elle est séchée à l'air. La partie de la membrane contenant la protéine d'intérêt (60 kDa) est découpée et envoyée pour le séquençage.

### I.1.4.3- Préparatifs en vu du séquençage des peptides internes

Pour visualiser les protéines dans le gel, on utilise un protocole de coloration à l'Amido-Schwarz légèrement différent de celui utilisé pour colorer la membrane PVDF. Après migration, le gel est fixé deux fois trente minutes avec une solution constituée de méthanol 50 %, d'acide acétique 10 %, d'eau milliQ 40 %. La coloration est réalisée avec une solution constituée de méthanol 45 %, d'acide acétique 10 %, d'eau 45 %, d'Amido-Schwarz 0,003 % (p/v). Les protéines apparaissent progressivement. Lorsque la coloration est suffisante pour repérer la protéine, le gel est rincé abondamment à l'eau milliQ. La bande d'intérêt est découpée puis deshydratée au speed-vac (Savant). La bande de gel, ayant perdue environ un tiers de sa longueur, est envoyé pour le séquençage. Les peptides internes sont obtenus après digestion de la protéine par l'endoprotéase Lys-C (sequencing grade Boehringer). La protéine dans le gel de polyacrylamide est digérée dans 150 µL de tampon Tris-HCl pH 8,6 (0,1 M) contenant 0,03% de SDS, à 35°C pendant 18 heures en présence de 0,4 µg d'endoprotéase Lys-C. La protéine digérée est injectée sur colonne HPLC DEAE-C18 (diamètre 1 mm); les peptides sont élués en utilisant un gradient d'acétonitrile (de 2 à 75 %) contenant du TFA à 0,1%. L'endoprotéase Lys-C clive spécifiquement les polypeptides du côté carboxylique des lysines.

### I.1.5.1- Validation théorique en utilisant le gène MndD d'Arthrobacter globiformis

Une partie (867 pb) du gène *Mnd*D est amplifié par PCR en utilisant les amorces 'OZ-MndD-S711': ACGTCACCGA AGAGGATGAA AAC et 'OZ-MndD-AS1578': ACGGCCATTT CGGACTTTTC. La PCR est réalisée en utilisant le programme suivant: 95°C 5 min; 25 cycles: 95°C 45 sec, 56°C 45 sec; 72°C 1 min; 72°C 5 min; 4°C en attente. Le mélange réactionnel comprend 200 à 500 µM de dNTP, 20 à 200 ng d'ADN cosmidique ou génomique et 100 pmol de chaque amorce dans un volume final de 50 µL.

### I.1.5.2- Identification par PCR d'une partie du gène codant l'HPP oxydase

La PCR est réalisée en utilisant le kit 'Advantage^{®}-GC Genomic PCR' (Clontech). Ce kit comprend, entre-autres, un adjuvant à base de bétaïne 'GC melt' et un mélange de polymérases thermorésistantes - principalement avec de la *Thermus thermophilus (Tth)* -. L'amplification est réalisée sur l'ADN génomique d*'Arthrobacter globiformis,* en utilisant la programmation suivante : 94°C 5 min; 30 cycles: 94°C 20 sec, 60°C 30 sec, 72°C 3 min; 72°C 6 min; 4°C en attente. Les conditions réactionnelles sont 400 µM dNTP, 50 ng d'ADN génomique, 100 pmol de chaque amorce, 'GC melt' 1X, pour un volume réactionnel de 50 µL. Dans ces conditions, nous amplifions une bande de 937 pb que nous dénommons Z2.

L'amplification par PCR peut être aussi réalisée en utilisant la *Tth* d'Epicentre ou la *Tbr* (*Thermus brockianus -* Finnzyme). La *Tbr* est la seule polymérase thermorésistante testée à pouvoir réaliser la PCR sans additifs (DMSO, glycérol, bétaïne) ; c'est en outre une enzyme de haute fidélité.

### I.1.6- Criblage de la banque cosmidique

Le criblage de la banque cosmidique est réalisé en utilisant la technique des sondes froides marquées à la dioxygénine (Boehringer Mannheim, 1995).

### I.1.6.1- Préparation de la sonde Z2-Dig

Le marquage de la sonde à la digoxygénine est faite par PCR dans un volume final de 50 µL, dans les conditions définies au paragraphe II.5.2, sauf pour le mélange de dNTP constitué par : dUTP-Dig 90 µM; dTTP 135 µM; dATP 225 µM; dCTP 225 µM; dGTP 225 µM. On quantifie la sonde amplifiée en déposant 3µL de la réaction sur un gel agarose à 0,8%. Il apparaît un léger bruit de fond, c'est à dire que la PCR n'est pas suffisamment spécifique. Afin d'éviter tous problèmes ultérieurs, la totalité de la PCR est déposée sur gel et la bande d'intérêt est extraite en utilisant le kit Qiaex II (Qiagen).

### I.1.6.2- Transfert de la banque cosmidique sur membrane Hybond N

Le stock glycérol de la banque cosmidique réalisée dans *E*. *coli* HB101 est utilisé pour inoculer 2 mL de milieu LBT¹⁵. Après 8 heures de croissance la DO₆₀₀ est estimée; des dilutions en cascade sont réalisées afin d'étaler environ 1000 clones par boîte (144 cm²). Après 16 heures de croissance à 37°C, les bactéries sont transférées sur des membranes Hybond N (Amersham) et lysées en suivant les recommandations de Boehringer Mannheim (1995). L'ADN libéré est fixé à la membrane par exposition aux U.V. (120 mJ délivrés en 45 sec - Stratalinker ; Stratagène). Les membranes sont débarassées des débris cellulaires en réalisant le traitement à la protéinase K comme préconisé par Boehringer Mannheim (1995).

### I.1.6.3- Préhybridation - hybridation - détection

Les étapes de préhybridation et hybridation se font dans un sac disposé sur un plateau à bascule, en utilisant la technique d'hybridation avec la sonde marquée à la digoxygénine (Boehringer Mannheim, 1995). La préhybridation (5x SSC; 0,5% SDS; 0,1% N-laurylsarcosine; 1% agents bloquants (Boehringer Mannheim, ref: 1096 176); 100 µg.mL⁻¹ sperme de saumon soniqué et dénaturé) est réalisée pendant 4 heures à 65°C. L'hybridation de la membrane est faite pendant la nuit à 68°C (milieu préhybridation frais contenant 20 ng.mL⁻¹ de sonde marquée à la digoxygénine et dénaturée pendant 5 min à 100°C). Le lendemain, l'excès de sonde et les hybridations aspécifiques sont éliminées par quatre lavages avec le tampon A (0,5x SSC ; 0,1% SDS, 65°C). Les membranes sont alors équilibrées pendant 5 min à température ambiante dans le tampon B (acide malique 138 mM, NaCl 142 mM, ajusté à pH 7,5 avec des pastilles de soude, 0,3% tween 20). Puis elles sont saturées par des agents bloquants (Boehringer Mannheim) durant 30 minutes avant d'être hybridées avec l'anticorps Anti-Digoxigénine couplé à la phosphatase alcaline ('Anti-Digoxigénine-AP, Fab fragments' ; Boehringer Mannheim) dilué au 1/10000 dans une solution fraîche d'agents bloquants. Après 30 minutes, les membranes sont rincées deux fois 15 minutes dans du tampon B, puis équilibrées 5 minutes dans le tampon réactionnel de la phosphatase alcaline (Tris 0,1 M; NaCl 0,1 M; MgCl₂ 0,05 M pH 9,5). Les membranes sont recouvertes avec 1 mL de CSPD prêt à l'emploi puis incubées 15 min à 37°C. Cette étape à 37°C permet une activation rapide de la phosphatase alcaline couplée à l'anticorps. On révèle les membranes en exposant des Hyperfilm^{®} ECL (Amersham) pendant 1 à 15 minutes.

### I.1.6.4- Analyse des cosmides positifs par Southern et PCR

Les cosmides identifiés lors de l'hybridation sur membrane sont confirmés par PCR et par la technique de Southern. Dans ce cas, l'ADN cosmidique, purifié par lyse alcaline (Maniatis *et al*., 1982), est digéré par des enzymes de restriction puis séparé sur gel d'agarose à 0,8 %. Les gels sont transférés sur membrane Hybond N⁺ (Amersham) par la technique de Southern en 20x SSC (Ausubel *et al.,* 1995). Après transfert, la membrane est rincée au 2x SSC, puis l'ADN est fixé à la membrane grâce aux U.V. (120 mJ délivrés en 45 sec - Stratalinker ; Stratagène). La membrane est alors révélée en utilisant la technique de sonde froide décrite précédemment.

### I.1.7- Vecteurs de clonage et bactéries hôtes

Les séquences d'ADN amplifiées par PCR sont généralement clonées dans le plasmide pGEMT-easy (Proméga) qui permet un criblage par le technique "bleu-blanc". Pour la surexpression, on utilise le plasmide pKK223-3 (Pharmacia) qui place le gène sous la dépendance d'un promoteur *tac*. Les clonages sont généralement réalisés en utilisant *E. coli* DH5α (New England Biolabs) ou *E*. *coli* XL1 Blue (Stratagène). Pour la surexpression on préférera *E*. *coli* BL21(DE3).

### I.1.8- Activité enzymatique de l'acétolactate synthase (ALS)

L'activité acétolactate synthase (ALS) est mesurée en utilisant la méthode colorimétrique décrite par Chang et Duggleby (1997). Les réactions sont conduites en microplaques avec un volume total de 250 µL. Pour chaque réaction, 25 µL d'enzyme sont incubées 30 min à 37°C dans 225 µL de milieu réactionnel constitué de KPi 50 mM pH 7,0; pyruvate de sodium 50 mM ; TPP 1 mM ; MgCl₂ 10 mM ; FAD 10 µM. La réaction est arrêtée par ajout de 25 µL d'H₂SO₄ 10 %. Les microplaques alors sont incubées à 60°C pendant 15 min. Puis on ajoute 250 µL de créatine 0,5 % et 250 µL d'α-naphtol à 5 % dans du NaOH 4 M (la solution d'α-naphtol doit être préparée moins de 10 min avant usage). La microplaque est alors incubée 15 minutes à 60°C puis 15 minutes à température ambiante. Une couleur rouge cerise apparaît. La lecture est réalisée à 525 nm (ε_{M}= 22 700 M⁻¹ cm⁻¹).

### I.2- Résultats - Discussion

L'HPP oxydase est la première activité enzymatique que nous souhaitons introduire dans la plante dans le cadre de la création de la voie métabolique contournant l'HPPD. Afin de pouvoir identifier le gène codant l'activité HPP oxydase différentes approches furent développées: (1) la mutagenèse insertionnelle et donc l'identification du gène par la perte de l'activité enzymatique, (2) la complémentation fonctionnelle d'un microorganisme en utilisant une banque génomique, (3) la purification de la protéine pour remonter à la séquence nucléique. C'est la troisième voie qui a été préférée.

### I.2.1- Purification de l'HPPO

### 1.2.1.1- Optimisation des conditions de culture

Avant de commencer à purifier la protéine, il est utile de déterminer quels sont les conditions de culture qui permettent son expression dans la bactérie. Les résultats d'optimisation des conditions de culture montrent que l'activité HPP oxydase n'est pas détectable lorsque la croissance d'*A*. *globiformis* est faite au dépend d'une source de carbone telle que le succinate, le fumarate ou le glucose. Par contre l'activité HPP oxydase est détectée lorsqu' *A*. *globiformis* est cultivé en utilisant l'HPP, la tyrosine ou la phénylalanine comme seule source de carbone. Si l'on augmente la quantité d'extrait de levure (par exemple 200 mg.L⁻¹ au lieu de 20 mg.L⁻¹) on observe une diminution de l'activité enzymatique produite. Sur la base de ces observations, le milieu M^{Ag} est défini. Enfin, on observe qu'une culture à forte densité (en début de phase stationnaire ; DO₆₀₀ ∼ 1) présente une activité enzymatique HPP oxydase plus faible que dans le cas d'une culture en phase exponentielle de croissance (DO₆₀₀ ∼ 0,4).

### 1.2.1.2- Tests préliminaires

Nous venons de définir le milieu optimal pour la production de l'HPPO, nous allons maintenant rechercher les conditions qui n'altèrent pas la stabilité de l'activité HPP oxydase lors des processus de purification. Pour les chromatographies impliquant les résines échangeuses d'anions et les chromatographies en fonction du pH, il est important de connaître la sensibilité de l'enzyme au pH et aux sels. Nous observons que le pH optimum est compris entre pH 7,0 et 7,8 ainsi que l'avait déjà démontré Blakley (1977). L'enzyme semble peu sensible aux sels (NaCl et KCl) puisqu'il faut des concentrations supérieures à 750 mM pour observer une baisse de l'activité enzymatique. Nous connaissons maintenant les conditions permettant une bonne expression de l'activité enzymatique et nous avons déterminé la sensibilité de l'activité HPP oxydase à des facteurs pouvant intervenir lors de la purification. La purification de l'HPPO peut donc commencer.

### 1.2.2.3- Purification de l'HPPO

Pour purifier l'HPPO, on applique le protocole décrit précédemment. L'activité enzymatique est éluée de la DEAE EMD 650S avec 150 à 200 mM de NaCl en solution dans un tampon phosphate 50 mM pH 7,4. Les fractions contenant l'activité enzymatique sont rassemblées et conservées pendant la nuit à 4°C. En effet la congélation à cette étape entraîne une perte d'activité. Les protéines sont ensuite chargées sur une résine Source Q. L'activité enzymatique est alors éluée avec une concentration en NaCl comprises entre 150 et 200 mM en solution dans un tampon phosphate 50 mM pH 7,4. Les fractions contenant l'activité enzymatique sont rassemblées puis concentrées sur membrane UVIKON 10 kDa, et conservées à 4°C pendant la nuit. Enfin l'HPPO est purifiée lors d'une troisième étape en appliquant un gradient phosphate sur colonne d'hydroxyapatite. L'activité est éluée avec une concentration de phosphate voisine de 30 mM. Les fractions contenant l'activité enzymatique HPP oxydase, en sortie de colonne d'hydroxyapatite, sont alors analysées sur un gel SDS-PAGE à 8,5% coloré au nitrate d'argent. Le gel présente l'évolution de deux bandes protéiques. Par comparaison entre le profil d'activité enzymatique et le profil d'élution des protéines, nous considérons que l'HPPO correspond à la protéine de plus haut poids moléculaire (environ 60 kDa). Dans l'essai présenté, la purification est initiée avec 1,5 g de protéines solubles extraites d'*A*. *globiformis* et nous avons récupéré 150 µg d'un mélange de protéines (dont environ 70 µg d'HPPO). Le facteur de purification en terme d'activité spécifique n'a pas été déterminé. En effet, nous utilisons des conditions de réaction totale pour suivre l'élution de l'activité enzymatique. En outre, la problématique était davantage l'identification de la protéine que la mise au point d'un protocole de purification. L'analyse HPLC, des réactions faites au sortir de chaque étape de purification, montre l'apparition d'un produit qui présente le même temps de rétention que le standard 4-HPA (SIGMA). Quarante picomoles de la protéine HPPO (60 kDa) sont transférées sur une membrane PVDF et sont envoyées pour le séquençage en même temps que 40 pmol de la protéine incluse dans le gel d'acrylamide. Les protéines transférées sur membranes servent à déterminer la séquence N-terminale tandis que les protéines incluses dans le gel sont utilisées pour déterminer la séquence de peptides internes.

### 1.2.2.4- Résultats de séquençage de l'HPPO

Peu de peptides internes sont obtenus en sortie d'HPLC après digestion de l'HPPO par l'endoprotéase Lys-C. Ce résultat suggère que la protéine contient peu de lysine, en effet l'endopeptidase Lys-C coupe après les lysines. Si la lysine est peu fréquente, la digestion par l'endopeptidase K génère des fragments peptidiques longs qui restent adsorbés dans la colonne et ne peuvent pas être élués, même en utilisant des conditions très hydrophobes. En se basant sur la forme des pics chromatographiques ainsi que sur la quantité apparente, on sélectionne puis on séquence trois peptides. Leur dénomination est fonction de leur ordre de sortie de la colonne HPLC: peptide N°4, peptide N°6, peptide N° 11. Leur séquence est respectivement: (A) WWAEALK, AAAGRILRLL DDAAGANASK, XDNRFTAVDF XT (où X est un acide aminé non déterminé). La séquence des 30 premiers acides aminés N-terminaux est obtenue avec un rendement initial de 40 %: TSLTVSGRVA QVLSSYVSD VFGVMGNGNV Y. L'acide aminé (méthionine ou valine) correspondant au codon initiation (ATG ou GTG) n'est pas retrouvé. Le rendement initial obtenu (15 pmol équivalent BSA), comparé avec celui obtenu pour les peptides internes (30 à 35 pmol équivalent BSA), suggère qu'une partie des protéines étaient bloquées en N-terminal. La séquence N-terminale et les séquences internes obtenues ne présentent aucune homologie dans les bases de données. En nous basant sur les séquences peptidiques obtenues, des oligonucléotides dégénérés sont synthétisés afin d'identifier le gène *HPPO* par PCR.

### I.2.3- Validation des techniques PCR et identification d'une partie du gène hppO

### I.2.3.1- Validation des techniques PCR

La teneur en base guanine et cytosine (GC %) de la majorité des ADN génomiques des *Arthrobacter sp.* est comprise entre 59 et 66 %, cependant il est de 67 à 69 % pour *A. agilis* (anciennement *Micrococcus agilis*) (Koch *et al.,* 1995), de 70 % pour *A. atrocyaneus* (Jones *et al.,* 1991) et de 73 % pour un *Arthrobacter sp.* identifié dans les glaces arctiques (Junge *et al.,* 1998). Ces fortes teneurs en guanine et cytosine peuvent rendre plus difficile la mise en oeuvre de la PCR. Pour cette raison que nous avons validé nos méthodes PCR (ADN génomique, polymérases, ...) en utilisant le gène codant la *'Manganese dependent Dioxygenase' (MndD)* d'*Arthrobacter globiformis* (Boldt *et al.,* 1995). Cette enzyme de la voie de dégradation de l'HPP catalyse l'ouverture du cycle aromatique du 3,4-dihydroxyphénylacétate. Pour l'amplification contrôle du gène MndD, nous avons testé des polymérases thermorésistantes de *thermophilus aquaticus* (Taq) commercialisées par différents fournisseurs (Perkin Elmer, ATGC, Appligène, Qiagen, Sigma). Dans tous les cas, l'amplification du gène MndD est obtenue. Cependant, dans des conditions équivalente, en utilisant les amorces dégénérées codant les peptides de l'HPPO, l'amplification du gène *hpp*O n'est pas obtenue même en utilisant des additifs (DMSO, glycérol).

### 1.2.3.2- Identification par PCR de la partie N-terminale du gène hppO

Nous amplifions de manière spécifique une séquence d'ADN de 936 pb qui pourrait correspondre à la partie N-terminale du gène *hpp*O. L'amplification est obtenue en utilisant d'une part les amorces dégénérées Ox3: TTNGCNCCNG CNGCRTCRTC et OZ10N: GAYGTNTTYG GNGTNATGGG NAAYGG correspondant respectivement à une partie du peptide N°6 et à une partie de la séquence peptidique N-terminale et d'autre part le kit 'Advantage GC Genomic PCR' (Clontech). Le kit de Clontech est conçu pour réaliser des PCR sur des génomes riches en bases GC. Il contient un mélange de polymérases thermorésistantes (dont la *Tth*) et un additif à base de bétaïne. La *Tth* est une polymérase thermorésistante purifiée à partir de *Thermus thermophilus.* La dégénérescence de chaque amorce est de 1024; c'est à dire qu'une amorce sur 1024 présente la séquence nucléique exacte du gène recherché. La dégénérescence provient du fait qu'un acide aminé peut être codé par plusieurs codons, pour exemple, l'alanine est codée par quatre codons (GCA, GCC, GCG, CGT). Le code de dégénérescence utilisé pour les amorces est défini comme suit : N = A ou T ou G ou C ; R = A ou G ; Y = T ou C. Les températures théoriques d'hybridation sont respectivement de 55,4°C et 57,6°C. Malgré une température d'hybridation de 60°C utilisée lors de la PCR, l'amorce OX3 seule permet des amplifications non spécifiques. Nous avons amplifié par PCR un fragment d'ADN de 936 pb de manière spécifique en utilisant deux amorces dégénérées. Nous devons nous assurer que cet ADN amplifié correspond bien au gène *hpp*O recherché.

### I.2.4- Caractéristique du fragment d'ADN de 936 pb

Le fragment d'ADN de 936 pb, amplifiée par PCR, est purifié sur gel d'agarose. Il est alors cloné dans pGEM-T easy, selon les instructions du fournisseur, puis séquencé. Lorsque l'on traduit la séquence nucléique obtenue, on observe qu'elle code aux deux extrémités pour la totalité du peptide N°6 et pour une bonne partie de la séquence N-terminale. Nous sommes donc sûr d'avoir amplifié une partie du gène codant la protéine purifiée et microséquencée, l'HPPO. La séquence nucléique contient 73% de bases guanine (G) et cytosine (C) on note en outre la possible formation de structures secondaires dites "en épingles à cheveux" *(stem-loop)* dans les 250 premières bases de l'ARN messager. Cette haute teneur en bases G et C ainsi que l'existence de ces structures secondaires peuvent expliquer en partie les difficultés rencontrées pour parvenir à l'amplification par PCR d'une partie de ce gène. La séquence nucléique de 936 pb ainsi que la séquence protéique correspondante ne présentent pas d'homologies avec les séquences enregistrées dans les bases de données. Nous possédons maintenant une séquence de 936 pb, orientée de N-terminal vers le peptide interne N°6. La protéine faisant environ 60 kDa, nous recherchons un gène d'environ 1650 pb. Il reste donc à identifier environ 700 pb. Pour cela nous allons cribler la banque génomique d'*A. globiformis* réalisée dans le cosmide pLAFR5 et amplifiée dans E. *coli* HB101.

### I.2.5- Criblage de la banque cosmidique d'A. globiformis

La banque génomique réalisée est transférée sur membrane, puis criblée en utilisant, comme sonde, le fragment d'ADN de 936 pb marqué à la digoxygénine. Le protocole standard est adapté pour un ADN "classique" (60% AT), tandis que le fragment de 936 pb présente une proportion estimée de 23% AT. Si nous gardons le même rapport dUTP-Dig/dTTP que dans le cas d'un ADN classique nous obtenons une sonde faiblement marquée donc une détection moins sensible. Nous avons donc optimisé la proportion dUTP-Dig/dTTP nécessaire pour le marquage de la sonde (paragraphe II.7.1). Le criblage de la banque génomique a permis d'identifier quatre cosmides (Cos1A, Cos2A, Cos4A, Cos17A1) ayant des profils de restriction différents. En comparant les résultats d'hybridation de Southern obtenus à partir des cosmides avec ceux obtenus à partir de l'ADN génomique *d'Arthrobacter globiformis,* nous sélectionnons le cosmide 2A. La figure N°14 illustre la démarche utilisée en prenant pour exemple la digestion des cosmides par l'enzyme de restriction *Not* I. Nous observons tout d'abord que le vecteur cosmidique pLAFR5, digéré par *Not* I, n'hybride pas avec la sonde Z2-Dig. Par contre, nous observons que le cosmide 1A présente une seule bande d'hybridation à 2,3 kb alors que les cosmides 2A, 4A et 17A présentent deux bandes d'hybridation à 4,3 et 2,3 kb. Or la digestion du génome d'*A. globiformis* par *Not* I produit deux bandes de 4,3 et 2,3 kb; de fait nous considérons que le cosmide 1A ne contient pas toute l'information que nous recherchons. En nous basant sur d'autres restrictions et en utilisant une démarche équivalente nous éliminons les cosmides 4A et 17A. Le Cosmide 2A est alors séquencé sur une distance d'environ 3 kb de part et d'autre du site *Not* I identifié au milieu de la sonde Z2-Dig. Les résultats d'hybridation de l'ADN génomique montrent en outre que le gène est présent à une seule copie. Nous avons identifié le cosmide 2A que nous avons fait séquencer sur 6,2 kb. Nous allons maintenant pouvoir analyser cette séquence d'ADN issue de génome d'*Arthrobacter globiformis.*

### I.2.6- Analyse globale de 6,2 kb d'ADN génomique d'Arthrobacter globiformis.

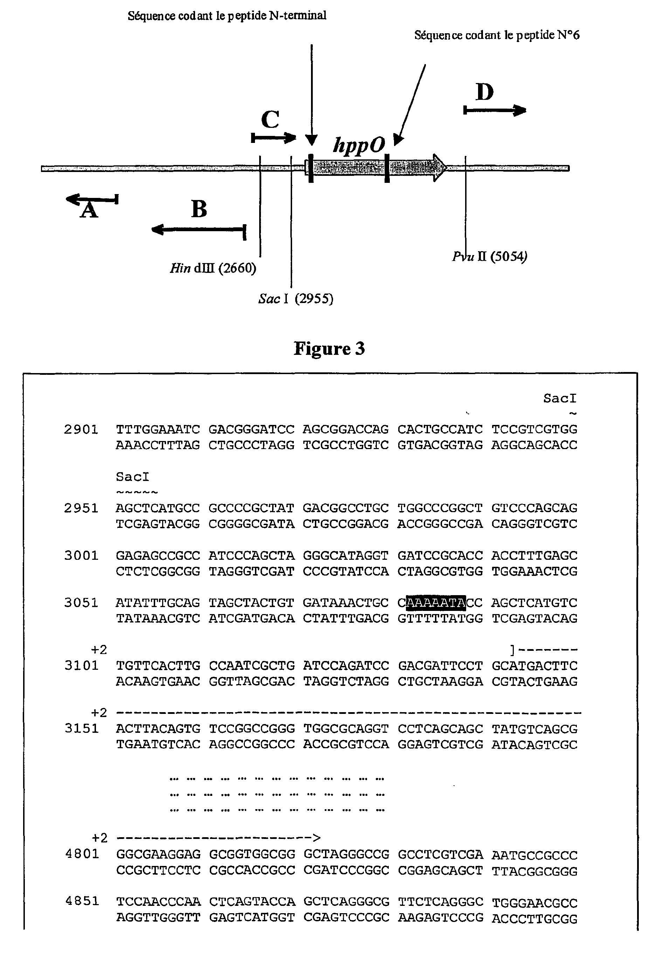

En utilisant le logiciel Vector Nti, la position des gènes potentiels est définie à partir de la séquence nucléique de 6255 pb obtenue en séquençant le cosmide 2A. Nous retrouvons la séquence de 936 pb, identifiée par PCR, comme faisant partie d'un gène potentiel. Ce gène potentiel correspond donc vraisemblablement au gène *hpp*O. Quatre autres gènes (A, B, C, D) sont potentiellement identifiés (Figure 3) en effectuant une recherche par homologie en utilisant l'algorithme BLASTX. Le gène A coderait un transporteur d'acide aminés, le gène B coderait une histidinol-phosphate aminotransférase cependant de précédents travaux montrent que cette enzyme possède l'activité tyrosine aminotransférase chez la bactérie à Gram positif *Bacillus subtilis* (Nester & Montoya, 1976), le gène **C** coderait un régulateur de transcription, tandis que le gène **D** coderait un régulateur d'opéron.

### I.2.7- Analyse du gène hppO

### I.2.7.1- Description générale

Sur la séquence obtenue de 6256 pb, le gène *hpp*O (en vert) est délimité en 5' par le codon d'initiation ATG en position 3143 et en 3' par le codon stop TAG (en rouge) en position 4823. Le gène présente donc une longueur réelle de 1680 pb. Il présente une forte teneur en bases G et C (71,4 % GC). La recherche d'homologies au niveau des séquences nucléiques (BLASTN), ne permet aucune identification. Afin de mieux caractériser le gène, nous recherchons les éléments spécifiques de la transcription et de la traduction.

### 1.2.7.2- Eléments caractérisant la transcription et la traduction du gène hppO

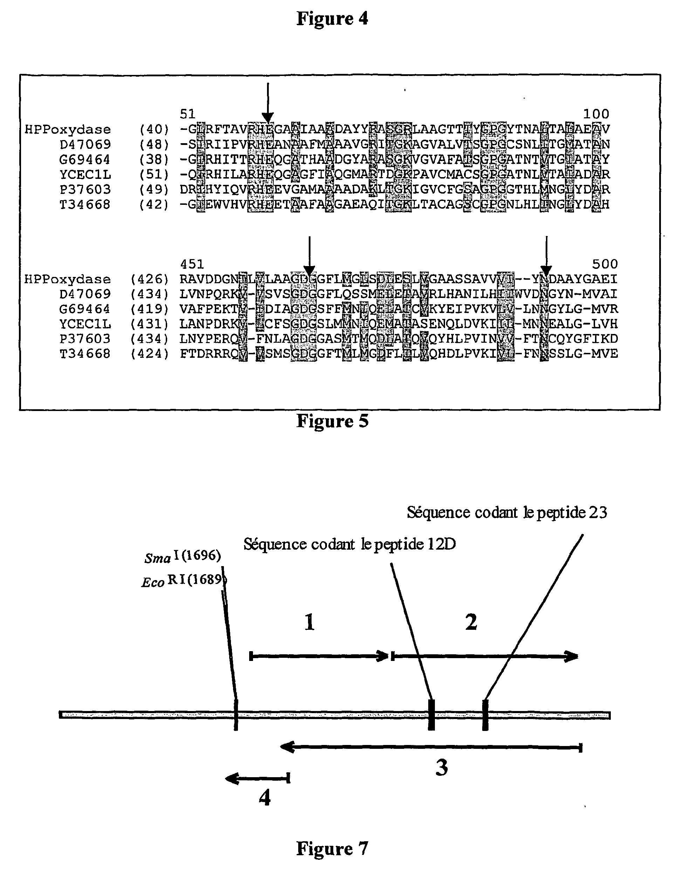

Nous identifions les potentielles boîtes promotrices de la transcription (Figure 4). La boîte « - 10 », dite « boîte de Pribnow », est située entre les positions 3082 à 3088 (AAAAATA) et la boîte « -35 » est située en position 3055 à 3059 (TTGCA). Les boîtes ainsi définies sont légèrement différentes des séquences canoniques (respectivement TATAAT et TTGACA ; Singer & Berg, 1992). Cela peut refléter une interaction faible avec les facteurs permettant la transcription constitutive ou bien la nécessaire interaction avec des facteurs de transcription différents. L'adénine en position 3096 pourrait être la base d'initiation de la transcription. Enfin nous identifions entre les positions 3068 à 3072 (TGTGA) une séquence correspondant au site d'attachement de la protéine CAP (*catabolic gene activator protein*). Le fait de retrouver ce site de fixation de la protéine CAP va dans le sens des résultats obtenus lors de l'optimisation des conditions de culture. En conclusion la transcription du gène *hpp*O est vraisemblablement sous le contrôle d'un promoteur faible, notamment régulé par le glucose. La séquence de Shine-Dalgarno (Singer & Berg, 1992) permet la fixation de la petite sous unité ribosomique. Elle est identifiée (GACGAT; en position 3131 à 3136) 12 bases en amont du codon d'initiation (ATG) de la traduction, par analogie avec la séquence consensus AGGA. On observe en outre que la partie 5' terminale (environ 250 bases) de l'ARN messager est capable de se structurer en épingle à cheveux *(stem-loop).* Or la structure secondaire de la région de l'ARNm qui avoisine l'ATG initiateur influence l'étape d'initiation de la traduction. Ainsi l'initiation est nulle ou peu efficace lorsque l'ATG initiateur ou la séquence Shine-Dalgarno sont impliqués dans un appariement intramoléculaire. On peut donc se poser la question d'un éventuel rôle régulateur de la traduction des structures en épingle à cheveux observées.

### 1.2.7.3- Expression de l'HPPO sous la dépendance du promoteur tac

La surexpression de l'HPPO est intéressante pour définir les caractéristiques cinétiques, pour permettre la production d'anticorps, mais aussi en vu de l'analyse structurale. Le gène est cloné dans un vecteur pKK223-3 en deux étapes. Le gène, amplifié par PCR dans les conditions définies pour l'identification du gène *hpp*O et en utilisant les amorces HPP-N-sens (CATGACTTCA CTTACAGTGT CC) et HPP-C-term (CAAACTGAGT AGCAGCTCAG G), est cloné dans le vecteur pGEMT-easy. On sélectionne le clone présentant le gène *hpp*O en antisens du promoteur *lac.* On le digère alors par *Eco* RI. Ce faisant on récupère le gène *hpp*O que l'on insert dans le vecteur pKK223-3 digéré par *Eco* RI. Le clone pKK3-2, présentant le gène *hpp*O sous le contrôle du promoteur *tac,* est retenu. Lorsque l'on induit l'expression du clone pKK3-2 par l'ajout d'IPTG, on peut détecter une activité HPP oxydase. Cependant la protéine surexprimée (57,4 kDa) n'est pas décelable dans un extrait brut séparé sur gel acrylamide dénaturant. Il reste donc à améliorer le protocole de surexpression. Nous envisageons en outre de cloner l'HPPO en fusion avec une séquence Tag, (GST, poly-histidine, protéine A....) afin de faciliter la purification de la protéine surexprimée. Nous venons définitivement de montrer que le gène identifié codait une activité HPP oxydase. Cependant, en réalisant des recherches d'homologie au niveau des séquences protéiques (BLASTX ou BLASTP), nous observons que la protéine HPPO présente jusqu'à 25% d'identité avec des acétolactate synthases (ALS), des pyruvate oxydases (POX) et des pyruvate deshydrogénases (PDH). Il est ainsi possible d'identifier des motifs très conservés tel ceux concernant la fixation du cofacteur TPP (Figure 5). En outre le profil d'hydrophobicité de l'HPPO est très proche de celui obtenu pour des ALS (non montré). Afin d'être sûr que le gène identifié code réellement l'HPPO et non pas une ALS, une POX ou une PDH ayant une activité annexe de type HPP oxydase, nous décidons de tester l'HPPO pour une éventuelle activité annexe.

### I.2.8- HPPO versus ALS

Les recherches d'homologies protéique montrent que l'HFPO présente jusqu'à 25% d'identité avec des ALS. Ce résultat, bien que surprenant au premier abord, présente une certaine logique. En effet ces deux enzymes utilisent le FAD et le TPP comme cofacteurs réactionnels. Elles réalisent toutes deux une décarboxylation. Par ailleurs, l'un des substrats de l'ALS est le pyruvate, or notre substrat un pyruvate β substitué : l'hydroxyphénylpyruvate. Il est donc possible que la structure du site actif soit voisine et que par conséquence ces protéines partagent des activités enzymatiques communes. Nous avons utilisé la grande sous-unité recombinante et purifiée des ALS d'*Arabidopsis thaliana* (Chang & Duggleby, 1997) et de *E. coli* (Hill & Duggleby, 1998) pour servir de contrôle positif dans nos expériences réalisées pour rechercher une activité ALS chez l'HPPO. Les résultats obtenus montrent que l'HPPO ne présente pas d'activité ALS. Nous montrons aussi à cette occasion que les deux ALS testées n'ont pas d'activité HPP oxydase. Enfin nous observons que l'HPPO n'est pas inhibé par 115 ppm d'imazapyr (inhibiteur d'ALS, Cyanamid). Ces résultats montrent que bien qu'en dépit de points communs (séquence protéique et hydrophobicité) les ALS et l'HPPO sont des enzymes bien distinctes, n'ayant pas d'activités enzymatiques secondaires.

### Exemple 2 IDENTIFICATION DES GENES CODANT LA 4-HPA 1-HYDROXYLASE

La 4-HPA 1-hydroxylase (HPAH) convertit le 4-HPA en HGA par une réaction d'hydroxylation accompagnée par un déplacement de la chaîne acétyle. Son activité a été caractérisée sur extraits bruts de *Rhodococcus erythropolis* S1 (Suemori *et al.,* 1995) ou sur extrait partiellement purifiés de *P. acidovorans* (Hareland, 1975). Elle a été purifiée par Suemori *et al.* (1996) cependant les séquences protéique et génétique ne sont pas publiées. Afin de pouvoir introduire cette activité enzymatique dans la plante, il est nécessaire d'identifier le gène.

Différentes approches sont envisageables: (1) la complémentation phénotypique et/ou fonctionnelle en utilisant une banque génomique, (2) la mutagenèse insertionnelle et donc l'identification du gène par la perte de l'activité enzymatique, (3) la purification de la protéine pour remonter à la séquence nucléique. Nous avons choisi de développer ces trois approches avec *Pseudomonas acidovorans* car il y a de nombreux outils de biologie moléculaire dont l'efficacité a été démontrée sur différentes espèces et souches de *Pseudomonas.* A titre d'exemples nous pouvons citer le transposon mini-Tn5 (De Lorenzo *et al.,* 1990), les vecteurs large spectre d'hôte tels pBBRIMCS (Kovach *et al.,* 1994, 1995; D'Souza *et al.,* 2000), les techniques de transfert par conjugaison. Le transposon mini-Tn5 peut être utilisé soit pour perturber un gène (de Lorenzo *et al.,* 1990 ; Fedi *et al.,* 1996 ; Campos-Garcia *et al.,* 2000) soit pour introduire un gène dans le génome bactérien (Prieto *et al.,* 1999). Nous avons commencé par l'approche par la complémentation phénotypique car c'est ce qui paraissait le plus rapide et le plus simple. Cette approche a été suivie par les deux autres simultanément. Cependant, nous n'aborderons pas ici l'approche par mutagenèse insertionnelle, cette voie n'ayant pas été exploitée par la suite.

### II.1- Matériels et Méthodes

### II.1.1- Construction d'une banque génomique de P. acidovorans dans E. coli

Pour construire la banque nous utilisons le cosmide pLAFR5 et l'ADN génomique de *P. acidovorans.* Nous utilisons la souche hôte *E. coli* HB101.

### II.1.2- Purification de la 4-HPA 1-hydroxylase

### II.1.2.1 - Test d'activité spectrophotométrique

Dans la réaction catalysée par la 4-HPA 1-hydroxylase, décrite par Hareland *et al.* (1975), il y a consommation d'oxygène moléculaire et de NADH,H⁺. Nous avons choisi de mesurer l'activité enzymatique en suivant l'oxydation du NADH,H⁺ en NAD⁺. Le milieu réactionnel comprend: NADH,H⁺ 300 µM; FAD 6,7 µM; KPi 100 mM; DTT 1 mM; 10 à 50 µg de protéines. La réaction est déclenchée par l'ajout du substrat: 4-HPA 1 mM. La réaction est suivie à 340 nm ou à 292 nm pendant 2 à 10 min. En effet, la consommation du NADH,H⁺ se traduit par une diminution de l'absorbance à 340 nm tandis que la production d'homogentisate se traduit par une augmentation de l'absorbance à 292 nm. Le test spectrophotométrique est très rapide, il est utilisé en routine pour suivre l'élution des protéines lors des étapes de purification.

### II.1.2.1- Test d'activité HPLC

L'analyse des réactions enzymatiques par HPLC permet de confirmer la production d'HGA (temps de rétention, spectre UV). Le test enzymatique est réalisé dans les mêmes conditions que ci-dessus. Cependant la réaction est arrêtée par ajout d'un tiers de volume d'acide perchlorique 20%. Les réactions sont alors analysées par HPLC en élution isocratique avec 90% de phase A et 10 % de phase B ou 92% de phase A et 8% de phase B. La phase A est de l'eau milli Q contenant 0,1% d'acide trifluoroacétique (TFA) et la phase B correspond à de l'acétonitrile. Dans l'élution en isocratique 90% - 10%, l'HGA est élué en 1,2 min alors qu'en système isocratique 92% - 8% il est élué en 1,4 min. L'élution est enregistrée généralement à 230 nm. Van den Tweel *et al.* (1986) ont utilisé le 2,2'-bipyridyl (inhibiteur de protéine à fer non hémique) pour inhiber l'homogentisate dioxygénase et ainsi permettre l'accumulation de l'HGA. Pour cette raison, nous ajoutons dans certains milieu réactionnel 2 mM de 2,2-bipyridyl. Dans ces conditions chromatographiques, il est possible d'identifier le 4-HPA et HGA. La chaîne HPLC est constituée d'une HPLC Alliance 2690 (Waters) et d'un détecteur à barette diode 996(Waters) .

### II.1.2.3- Purification de la protéine HPAH

*Pseudomonas acidovorans* est cultivé 48 heures sur milieu M63 contenant du 4-HPA comme seule source de carbone, à 29°C, 220 rpm. Les bactéries sont centrifugées à 3 000 g pendant 15 min à 6°C (Beckmann J2/21 M/E centrifuge). Le culot bactérien est repris dans le tampon de sonication (KPi 0,1 M pH 7,2; MgSO₄ 1mM; DTT 1mM; benzamidine hydrochloride 1 mM; acide caproïque 5 mM). La benzamidine hydrochloride et l'acide caproïque sont des inhibiteurs de protéases. La sonication est réalisée pendant 9 minutes en sonicant toutes les quarante secondes pendant vingt secondes à la puissance 5 (Vibra Cell, Sonic Materials INC., Connecticut, USA). Durant la sonication, l'échantillon est maintenu à la température de la glace fondante. L'extrait soniqué est centrifugé à 15 000 g pendant 15 min à 4°C. Le surnageant récupéré est précipité avec 1 % de sulfate de streptomycine. Le précipité est éliminé par centrifugation à 15 000 g pendant 15 min à 4°C. Le surnageant est dessalé sur colonne PD10 (Pharmacia) puis chargé sur colonne DEAE/EMD 650 S équilibrée en tampon A (KPi 20 mM pH 7,2, glycérol 10 %, MgSO₄ 1 mM, DTT 1 mM). L'élution se fait en utilisant un tampon B (tampon A; KCl 1 M; 100 µM FAD). L'activité 4-HPA 1-hydroxylase est éluée pour une concentration en KCl voisine de 150 mM. Les fractions actives, concentrées sur membrane UVIKON 10 kDa puis dessalées sur colonne PD10, sont alors déposées sur une colonne d'affinité Red (Red 120 Agarose type 3000 CL, SIGMA Ref R-0503) équilibrée en tampon A (ci dessus). L'élution est réalisée en deux étapes. La première est un lavage de la colonne Red en utilisant le tampon A enrichi avec du FAD 50 µM final. La deuxième permet l'élution de la protéine; pour cela le tampon A est enrichi en FAD (3 mM) et en NADH,H⁺ (10 mM). Les fractions, contenant la protéine d'intérêt, sont rassemblées, concentrées et congelées à -80°C.

### II.1.3- Microséquençage protéique de l'extrémité N-terminale et de peptides internes

Le même protocole que celui décrit dans le cas de l'HPP oxydase a été utilisé pour réaliser le séquençage de la protéine purifiée. Cependant, pour produire les peptides internes, la protéine a été digérée à la trypsine au lieu de l'endopeptidase Lys-C. La trypsine coupe après les arginines et les lysines. La digestion par la trypsine conduit généralement à l'obtention de fragments plus petits que ceux obtenus lors d'une digestion par l'endopeptidase Lys-C. Afin de pouvoir séquencer avec précision les peptides récupérés, il est parfois nécessaire de repurifier par HPLC les peptides récupérés.

### II.1.4- Identification d'une partie du gène codant l'HPAH par PCR dégénérée

Pour la synthèse des amorces dégénérées on utilise le code de dégénérescence présenté en page 43. La PCR est réalisée dans un volume final de 50 µL, dans des tubes de 200 µL. La solution réactionnelle contient le tampon Perkin Elmer, 250 µM dNTP, 50 ng d'ADN génomique de *P. acidovorans,* 2 unités enzymatiques d'AmpliTaq (Perkin Elmer). La réaction est réalisée en utilisant un thermocycleur "Hybaid Touchdown": 3 min à 94°C, puis quarante cinq cycles : 30 sec à 94°C, 1 min à 50°C, 1 min 30 sec à 72°C, suivi d'une élongation finale de 5 min à 72°C avant de revenir à 4°C. La PCR est évaluée après dépôt de 10 µL sur gel 1% agarose. Dans ces conditions une bande de 536 pb est identifiée.

### II.1.5- Criblage de la banque cosmidique de P. acidovorans

Etalement de la banque cosmidique sur milieu LBT¹⁵ et croissance pendant 16 h00 à 37°C. Les boîtes sont alors transférées à 4°C. Au bout d'une heure, les colonies sont transférées sur membranes Hybond N (Amersham) selon la méthode de Grunstein & Hogness (1975). Les membranes sont hybridées en utilisant le fragment PCR de 536 pb précédemment identifié et purifié. La détection est réalisée en ³²P. La sonde est marquée en utilisant le kit « DNA Ready to Go » (Pharmacia). La pré-hybridation, l'hybridation et les lavages sont réalisés en ampoules. Les membranes sont pré-hydridées dans une solution composée de SSC 5x, Denhardt 6%, SDS 0,5% pendant 4 heures à 68°C. L'hybridation est réalisée pendant 16 heures à 68°C. Les lavages sont effectués à 65°C en SSC 2x, SDS 0,1%. Les membranes sont révélées en exposant des films Kodak ou Amersham.

### II.1.6- Milieux de croissance de P. putida

*Pseudomonas putida* est cultivé sur milieu riche de Luria-Bertani (LB) ou 2YT contenant 100 µg.mL⁻¹ de rifampicine. En fonction des besoins d'autres antibiotiques sont ajoutés (exemple : tétracycline à 15 µg.mL⁻¹). Le milieu minimum M63 contenant 1,5 g.L⁻¹ de 4-HPA comme seule source de carbone est utilisé pour tester la complémentation fonctionnelle. Dans ce cas les antibiotiques sont omis. Toutes les cultures sont réalisées à 29°C.

### II.1.7- Transformation de P.putida par électroporation

1 litre de milieu LB Rifampicine (100 µg.mL⁻¹) est inoculé avec une culture de *P. putida* mise en croissance à 29°C pendant environ 16 heures en agitation à 180 rpm. Lorsque la DO₆₀₀ₙₘ est voisine de 1,2, les cellules sont collectées par centrifugation pendant 15 min à 3 000 g, 4°C. Le milieu de culture est éliminé et les cellules sont reprises avec 400 mL de glycérol 10% à 4°C. Les cellules sont centrifugées une nouvelle fois à 3000 g, 20 min, 4°C. Deux nouvelles étapes de lavage sont effectuées avec respectivement 200 puis 100 mL de glycérol 10%, 4°C. Enfin, les bactéries sont reprises par 3 à 10 mL de glycérol 10% puis réparties en aliquotes de 100 µL immédiatement congelées dans l'azote liquide. Les bactéries ainsi préparées se conservent au moins six mois à - 80°C. Lors de la préparation, on observe une perte de bactéries due à la lyse. L'ADN cosmidique (Tet^{R}) est introduit par électroporation dans les *P. putida* (Rif^{R}). L'électroporation (Bio-Rad Gene Pulser^{™}) de 80 ng d'ADN cosmidique dans 100 µL *P. putida* électrocompétentes se fait en cuvette d'électroporation de 2 mm sous une tension de 0,9 volt avec une résistance de l'électroporateur de 200 Ω. Dans ces conditions, la constante de temps τ est d'environ 4,5 msec. Après le choc électrique, les cellules sont reprises avec 900 µL de LB et mise en culture pendant 1h30 à 29°C, 180 rpm. Les *P. putida* transformées sont sélectionnées sur milieu gélosé LB Rif¹⁰⁰ Tet¹⁵.

### II.1.8- Modification du vecteur large spectre d'hôte pBBR1MCS-Gm^{R}

Nous avons utilisé les vecteurs à large spectre d'hôte à Gram négatif de la série des pBBR1MCS (Kovach *et al.,* 1994, 1995). Ces plasmides, qui possèdent une origine de réplication de *Bordetella bronchiseptica,* se répliquent à environ 20-30 copies par cellule chez E. *coli.* Ils contiennent deux sites *Not* I. Afin de faciliter les clonages ultérieurs on supprime le site *Not* I présent hors du multi-site de clonage (MCS) sur le plasmide pBBR1MCS-Gm^{R}. Pour cela, le plasmide est coupé par *Sfi* 1 (50°C) puis traité à la T4 DNA polymérase afin d'obtenir des bouts francs. Le plasmide est religué sur lui même (T4 DNA Ligase - New England Biolabs). Après ligation (16 heures, 16°C), une digestion par *Sfi* I est réalisée afin d'éliminer les éventuels plasmides " sauvages ", puis on électropore E. *coli* DH5α. L'ADN plasmidique est isolé des clones sélectionnés sur milieu LB Gm²⁰. Les ADN plasmidiques sont caractérisés par deux digestions: *Not* I et *Not* I/*Bgl* II. Un clone est retenu: pBBR1MCS-Gm-Not-U.

### II.1.9- Sous-clonage du Ccos8 dans pBBR1MCS-Gm-U

Le cosmide Ccos8 est restreint par *Not* I puis déposé sur gel agarose. Après migration, 6 bandes d'ADN sont visualisées : 1,7 - 3 - 4 - 5 - 8 - 10 kbp. Les bandes sont purifiées par Quiaex II. Par ailleurs, pBBRIMCS-Gm-Not-U est restreint par *Not* I, déphosphorylé en utilisant la phosphatase alcaline de crevette (S.A.P.; shrimp alkaline phosphatase). Les différentes bandes sont alors liées (T4 DNA ligase, 16 heures, 16°C) dans le vecteur en utilisant des rapports " insert/vecteur " variables. Les produits de ligation sont transformés dans E. *coli* DH5α.

### II.1.10- Conjugaison tri-parentale entre E. coli et P. putida

Afin de transférer les différents sous-clones de Ccos8 (Gm^{R}) de *E. coli* DH5α vers *P. putida* (Rif^{R}), on opère par conjugaison tri-parentale sur filtre en utilisant le protocole décrit par De Lorenzo *et al.* (1990). Les bactéries récupérées sont étalées sur LB Rif¹⁰⁰Gm²⁰ et sur M63 ayant le 4-HPA comme seule source de carbone.

### II.1.11- Elimination du plasmide p5kbC

Pour éliminer rapidement le plasmide p5kbC de *P. putida,* nous utilisons la stratégie des origines de réplication incompatibles et nous forçons la perte du p5kbC à l'aide d'antibiotiques. On transforme *P. putida* (Rif¹⁰⁰) complémenté par le plamide p5kbC (Gm^{R}) avec pBBR1MCS Kn^{R}. Les clones obtenus (Rif¹⁰⁰ Gm^{R} Kn^{R}) sont vérifiés pour leur activité de complémentation. Les clones sont alors cultivés sur deux milieux : LB Rif¹⁰⁰ Kn¹⁵⁰ Gm²⁰ et LB Rif¹⁰⁰ Kn¹⁵⁰. Ce faisant nous maintenons la pression de sélection pour p5kbC et pBBR1MCS Kn^{R} ou bien seulement pour pBBR1MCS Kn^{R}. Les croissances sont réalisées à 29°C. Le repiquage est réalisé tous les trois jours. Au huitième repiquage, lès colonies sont repiquées sur 4 milieux différents (M63, M63 + 4-HPA, LB Rif¹⁰⁰ Kn¹⁵⁰ Gm²⁰ et LB Rif¹⁰⁰ Kn¹⁵⁰) quelle que soit la boîte d'origine. L'état de croissance est alors relevé au bout de 2 et 7 jours.

### II.1.12- Identification des protéines participant à l'activité enzymatique.

### II.1.12.1- Préparation d'extraits bruts de P. putida

Deux clones *P. putida* sont cultivés sur LB Gm²⁰ pendant 24 heures. Le premier comporte le plasmide pBBRIMCS-Gm-Not-U tandis que le second contient le plasmide de complémentation p5kbC. Après sonication dans un tampon (KPi 0.1 M; MgSO4 1mM; DTT 1mM; benzamidine hydrochloride 1 mM; acide caproïque 5 mM), puis centrifugation à 20 000 g pendant 10 min à 4°C, le surnageant est testé pour son activité 4-HPA 1-hydroxylase en utilisant les deux méthodes de mesure de l'activité enzymatique. Les extraits bruts sont, en outre, analysés par SDS-PAGE à 10%.

### II1.12.2- Transfert sur membrane, Séquençage N-terminal

Le séquençage est réalisé comme en exemple I.

### II.1.12.3- Gel Filtration S75

L'éluat (5 mL) est concentrée d'un facteur 10 en utilisant un Macrosep^{™} 10 K (Pall Filtron) pendant deux heures à 4°C. Les 500 µL concentrés sont injectés sur une colonne de gel filtration Superdex^{™} 75 prep grade (HiLoad 16/60, Pharmacia) préalablement équilibrée avec 700 mL de tampon (KPi 0,02 M pH 7,2; glycérol 10%; MgSO₄ 1 mM ; DTT 1 mM ; 4°C) à un débit de 0,7 mL.min⁻¹. La chromatographie est réalisée à 4°C avec un débit de 1 mL.min⁻¹. Les fractions sont collectées toutes les minutes et conservées à 4°C.

### II1.12.4- Construction de pBBR1MCS FT12Δ1

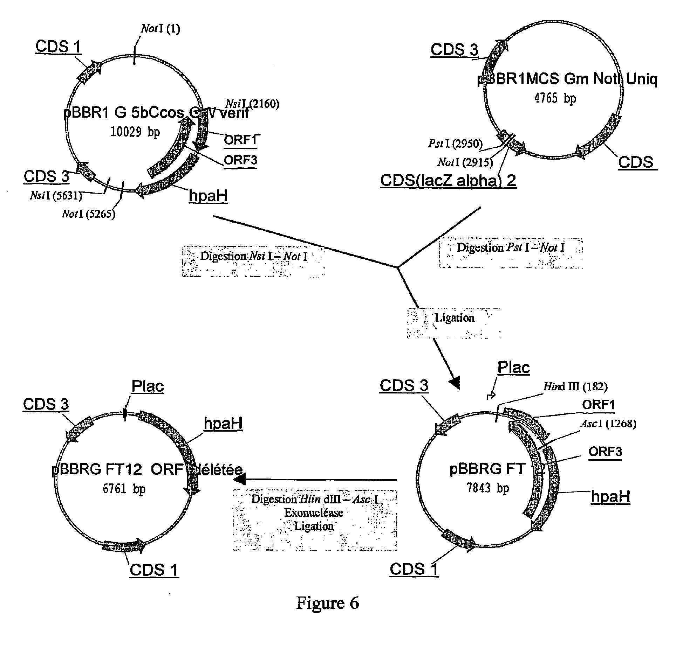

Pour construire le plasmide pBBR1MCS FT12Δ1 on utilise une stratégie de clonage en deux étapes. Le plasmide p5kbC est digéré par *Nsi* I et *Not* I. L'insert obtenu, codant les gènes 1, *hpa*H et **3**, est alors cloné dans pBBRIMCS-Gm-Not-U digéré *Pst* I et *Not* I. Le clone résultant, dénommé pBBR1MCS FT12, est restreint par *Hind* III et *Asc* I, puis rendu bout-francs et enfin religué. Ce faisant, les gènes **1** et **3** sont détruits et le gène *hpa*H se trouve sous la dépendance du promoteur *lac* du vecteur originel. Nous obtenons ainsi le plasmide pBBR1MCS FT12Δ1 (Figure 6).

### II.1.12.5- Construction de pL11ac2

Le laboratoire possède un plasmide dénommé "Clone L". Cette construction correspond au clonage du promoteur et du gène de l'HPPD de *P. fluorescens* dans le vecteur pBBR1MCS-Kn^{R}. Le promoteur du gène HPPD est fonctionnel chez *P. putida* et chez *E. coli.* Le plasmide "Clone L" est digéré par *Bam* HI et *Hin* dIII ce qui permet de récupérer l'insert contenant le promoteur et le gène HPPD de *P. fluorescens.* Cet insert est alors ligué dans le vecteur pBBR1MCS-Gm^{R} digéré par *Bam* HI et *Hin* dIII. Le clone résultant est dénommé pBBRG-L-HPPD. Le plasmide obtenu, digéré par *Nco* I pour éliminer le gène codant l'HPPD, est ligué avec le gène *hpa*C amplifié par PCR et digéré par *Aƒl* III. La construction obtenue est appelée pBBRG-L-ORF1. Pour l'amplification du gène *hpa*C par PCR, on utilise des amorces qui permettent d'introduire un site *Aƒl* III en début et en fin de gène (le site *Aƒl* III est compatible avec le site *Not* I). Les amorces utilisées sont : en 5' du gène : GCAGGATGCA CATGTCCACC AAGAC et en 3' du gène : CGGACGCCGA CATGTATCAG CCTTC. La PCR est réalisée en utilisant 1 unité de KlenTaq polymérase (Sigma), 250 µM de dNTP, 200 nM de chaque amorce et 50 ng du plasmide p5kbC. Le programme de PCR est défini comme suit sur Perkin Elmer 9600 : 3 min à 95°C ; puis 20 cycles : 94°C pendant 1 min, 60°C pendant 30 sec, 68°C pendant 3 min; enfin une dernière étape de 10 min à 68°C est réalisée. Le plasmide pBBR1MCS FT12Δ1 précédemment obtenu est restreint par *Ssp* I et *Not* I. Le site *Not* I est rendu franc par traitement avec la *Pƒu.* Le fragment récupéré (2468 pb), contenant le gène *hpa*H sous la dépendance du promoteur *lac,* est ligué dans pBBRG-L-ORF1 digéré par *Ssp* I. Le clone présentant les gènes *hpa*C et *hpa*H en antisens est retenu, il est nommé pL1lac2. Tous ces clonages sont réalisée dans E. *coli* DH5α.

### II.2- Résultats

Différentes approches sont envisageables pour identifier le gène codant l'activité 4-HPA 1-hydroxylase de *P. acidovorans.* Nous décidons dans un premier temps d'utiliser une approche par coloration phénotypique. Cette approche paraît simple et rapide. En effet, nous possédons au laboratoire un outil de criblage phénotypique pour détecter la production d'HGA. Or l'enzyme que nous recherchons convertit le 4-HPA en HGA.

### II.2.1- Approche par coloration phénotypique

Nous avons observé au laboratoire qu'*E*. *coli* K12 ne peut pas croître en utilisant la tyrosine ou le 4-HPA comme seule source de carbone. Nous savons d'autre part qu'*E*. *coli* K12 possède l'activité tyrosine aminotransférase qui permet la synthèse de tyrosine à partir d'HPP. Cette activité enzymatique est réversible, la cellule peut donc produire de l'HPP à partir de la tyrosine. Si le milieu riche de culture est enrichi en tyrosine (1 g.L⁻¹), la tyrosine est importé dans les bactéries qui l'accumulent puis la transforme en HPP, selon la constante d'équilibre de la réaction de conversion entre HPP et tyrosine. Au laboratoire, nous avons déjà observé que si nous introduisons l'HPPD de *P. fluorescens* dans *E. coli* K12 alors l'HPP produit lors de la désamination de la tyrosine, est transformé en homogentisate (HGA). La réaction catalysée par l'HPPD étant irréversible, l'HGA s'accumule dans la cellule où il s'oxyde puis polymérise spontanément pour former un pigment ochronotique présentant une coloration brune. Nous avons donc là un moyen de détecter la production d'HGA. La 4-HPA 1-hydroxylase recherchée convertit le 4-HPA en HGA. On étale donc les *E*. *coli* HB101 contenant la banque génomique de *Pseudomonas acidovorans* sur milieu gélosé 2YT enrichi en 4-HPA. Après deux jours, deux colonies brunissent : elles produisent donc de l'homogentisate. Cependant, les activités enzymatiques réalisées sur les extraits bruts de ces deux clones révèlent une activité enzymatique de type HPPD alors que l'activité 4-HPA 1-hydroxylase recherchée est discrète, voire inexistante. A priori cette approche a permis de sélectionner les clones dont le cosmide contient le gène codant une HPPD de *P. acidovorans* et non pas la 4-HPA 1-hydroxylase. Lors de l'étude préliminaire *in vitro* sur les extraits bruts de *P. acidovorans,* l'activité HPPD n'a pas été identifiée. On peut supposer que l'activité HPPD de *P. acidovorans* s'exprimerait lorsque la bactérie est cultivée sur milieu riche tandis que l'activité 4-HPA 1-hydroxylase s'exprimerait lorsque le 4-HPA est la seule source de carbone. Cette approche ne permettant pas l'identification de la 4-HPA 1-hydroxylase nous décidons de purifier l'enzyme. Une fois la protéine identifiée, il sera possible de remonter au gène correspondant.

### II.2.2- Purification de la 4-HPA 1-hydroxylase

Pour suivre la purification de la protéine, nous utilisons le dosage de son activité NADH,H⁺ oxydase dépendante du 4-HPA. Nous purifions ainsi la protéine à quasi homogénéité en appliquant le protocole de purification décrit précédemment. Le facteur d'enrichissement de l'activité spécifique NADH,H⁺ oxydase est généralement compris entre 50 et 100 selon les préparations. Sur SDS-PAGE, la protéine présente un poids moléculaire apparent de 60 kDa. En fait, nous observons que l'activité NADH,H⁺ oxydase et la production d'HGA sont bien visibles en sortie de DEAE/EMD 650S. Par contre en sortie de colonne d'affinité, la production d'HGA est très difficilement décelable; l'activité NADH,H⁺ oxydase reste cependant dépendante de l'ajout du 4-HPA dans le milieu réactionnel. Si nous partons de l'hypothèse que l'enzyme est monomérique, la perte de l'activité catalytique permettant la production d'HGA peut s'expliquer en supposant qu'une partie de la protéine a été endommagée (ex : perte d'un cofacteur fortement lié) lors de son passage sur la colonne Red. Le site catalysant l'oxydation du NADH,H⁺ ne serait pas touché. On peut aussi supposer que l'enzyme recherchée est un hétérodimère. La perte de l'activité catalytique s'expliquerait alors par la perte du monomère responsable de la production d'HGA. Dans la littérature de nombreuses monooxygénases hétérodimériques à flavine ont été identifiées, ayant toutes un substrat aromatique, dans des espèces bactériennes variées (Adachi *et al.,* 1964; Arunachalam *et al.,* 1992, 1994; Prieto *et al.,* 1993; Prieto & Garcia, 1994; Arunachalam & Massey, 1994; Takizawa *et al.,* 1995; Xun, 1996 ; Xun & Sandvik, 2000). Cependant, il existe deux hypothèses pour expliquer le fonctionnement de ces enzymes hétérodimériques:
(1) Arunachalam *et al.* (1992, 1994) proposent que la 4-hydroxyphénylacétate 3-hydroxylase de *P. putida* soit constituée d'une flavoprotéine homodimérique de 65 kDa ainsi que d'une protéine de couplage de 38,5 kDa. La flavoprotéine seule est capable d'oxyder le NADH,H⁺ indépendamment de la présence de 4-HPA. Cette oxydation du NADH,H⁺ permet de renouveler le "pool" de NAD⁺, mais produit de l'H₂O₂ dans des proportions stoechiométriques. Si la protéine de couplage est ajoutée, le complexe protéique devient capable d'hydroxyler le 4-HPA en acide 3,4-dihydroxyphénylacétique. Ainsi, l'oxydation du NADH,H⁺ n'est pas gaspillée et permet la synthèse d'un métabolite. La protéine de couplage seule n'a pas d'activité enzymatique.
(2) Prieto *et al.* (1993, 1994) et Xun & Sandvik (2000) suggèrent que la 4-HPA 3-hydroxylase de *E. coli* W (ATCC 11105) soit considérée comme un nouveau membre des monooxygénases à flavine à deux composantes mobiles (TC-FDM). Les deux composantes seraient d'une part la 4-hydroxyphénylacétate 3-hydroxylase, une enzyme monomérique de 59 kDa codée par le gène *Hpa*B*,* et d'autre part une flavine:NADH oxydoréductase monomérique de 19 kDa, codée par le gène *Hpa*C. Dans ce cas, le FAD est réduit au dépend du NADH,H⁺ par la flavine:NADH,H⁺ oxydoréductase. Le FADH₂ est alors utilisé par l'oxygénase pour permettre l'oxydation du substrat en utilisant l'oxygène moléculaire.

L'enzyme que nous avons purifiée oxyde fortement le NADH,H⁺ mais produit très peu d'homogentisate. En outre l'oxydation du NADH,H⁺ est dépendante de l'ajout de 4-HPA. Ceci suggère que nous possédons une enzyme du type de celle décrite par Prieto *et al.* Nous considérons donc que l'enzyme purifiée est la 4-HPA 1-hydroxylase (HPAH) recherchée. Il est possible, que par la suite, il soit nécessaire d'identifier une protéine de couplage pour optimiser l'activité enzymatique. L'approche biochimique peut donc se poursuivre avec la protéine purifiée.

### II.2.3- Obtention des peptides internes et de la séquence N-terminale.

La protéine purifiée est envoyée à l'Institut Pasteur pour être micro-séquencée. C'est ainsi que l'on obtient la séquence N-terminale SHPAISLQAL RGSGADIQSI HIPYER et six peptides internes nommés respectivement peptides N° 11C, 12D, 20A, 22B, 23, 24 en fonction de leur ordre de sortie de colonne: ATDFITPK, LGVGQPMVDK, VVFAGDSAHG VSPFX, VTALEPQAEG AL, IDFQLGWDAD PEEEK, LSVPATLHGS ALNTPDTDTF. Sur la séquence N-terminale, on ne retrouve pas l'acide aminé (méthionine ou valine) correspondant normalement au codon d'initiation du gène (ATG ou GTG). Les analyses d'homologies dans les bases protéiques en utilisant l'algorithme BLASTP ne permettent pas d'identifier de protéines homologues. Sur la base des séquences protéiques obtenues, nous faisons synthétiser les oligonucléotides dégénérés correspondants. Ceux-ci sont utilisés dans des réactions de PCR afin d'identifier une partie du gène codant la protéine HPAH purifiée et partiellement séquencée.

### II.2.4- Obtention du fragment PCR

L'amplification par PCR d'une portion (536 pb) du gène codant la 4-HPA 1-hydroxylase est obtenue en utilisant les amorces dégénérées Hy4R: TCYTCNGGRT CNGCRTCCCA et Hy5F: GGNGTNGGNC ARCCNATGGT qui codent respectivement les peptides 23 et 12D. Ces amorces ont une température d'hybridation de 55,4°C et présentent une dégénérescence de 128 et 512 respectivement. La séquence amplifiée est clonée dans le vecteur pGEMT-easy puis elle est séquencée. L'analyse de la séquence obtenue permet de retrouver, outre les séquences codant les peptides Hy4R et Hy5F, la séquence nucléique codant le peptide interne 22B. Ce dernier élément permet de confirmer que nous avons bien amplifié une partie du gène codant la protéine HPAH purifiée. A ce stade, les recherches d'homologies dans les bases protéiques, en utilisant l'algorithme BLASTX, font ressortir quelques faibles homologies avec des hydroxylases, des oxydases et des monooxygénases. En utilisant la séquence de 536 pb amplifiée par PCR, nous allons pouvoir cribler une banque cosmidique de *P. acidovorans* afin de rechercher le cosmide contenant le gène complet.

### II.2.5- Criblage de la banque cosmidique de P. acidovorans

Le criblage de la banque cosmidique, en utilisant comme sonde la séquence obtenue ci-dessus, a permis d'identifier 4 groupes de cosmides considérés comme différents sur la base de leurs profils de restriction et d'hybridation après transfert par la technique de Southern. Les cosmides N° 1, 2, 6 forment le premier groupe, les cosmides N° 3, 7, 9 forment le second tandis que les cosmides N°5 et 8 forment le troisième. Le dernier groupe est représenté par le cosmide N°4. Les résultats d'hydridation suggèrent en outre que le gène *hpa*H recherché est présent en un seul exemplaire dans le génome de *Pseudomonas acidovorans.* Nous avons identifié des cosmides comportant au moins une partie du gène codant la protéine HPAH purifiée. Entre temps, nous avons observé que *P. putida* était incapable de croître sur 4-HPA mais pouvait croître en utilisant l'HGA comme seule source de carbone. Nous possédons donc là un excellent crible pour la complémentation fonctionnelle ; nous pourrons ainsi définir lequel de ces cosmides comporte le gène fonctionnel codant l'activité 4-HPA 1-hydroxylase.

### II.2.6- Complémentation fonctionnelle avec les cosmides

Les neuf cosmides précédemment identifiés sont introduits dans *P. putida* par électroporation. Les clones obtenus sont alors repiqués sur milieu M63 contenant du 4-HPA comme seule source de carbone. Au bout de 7-8 jours seules les bactéries possédant le cosmide N°8 ont réussi à croître. C'est à dire que seul le cosmide N°8 contient toute l'information exprimable permettant la conversion du 4-HPA en HGA utilisable par *P. putida.* Le cosmide est alors dénommé Ccos8. La transformation avec l'ensemble des cosmides est répétée. C'est toujours le cosmide 8 qui permet la complémentation après un certain délai (6-10 jours). Afin de pouvoir avancer dans notre approche de détermination du fragment d'ADN minimum exprimant l'activité 4-HPA 1-hydroxylase, il nous faut sous-cloner le Ccos8. La sélection du sous-clone intéressant se fera en utilisant le crible de la complémentation fonctionnelle.

### II.2.7- Sous clonage par complémentation fonctionnelle

La digestion par *Not* I du cosmide permet d'obtenir 6 fragments d'ADN de taille comprise entre 1,7 et 10 kb. Ces fragments sont clonés dans pBBR1MCS-Gm-Not-U. Cinq sous-clones de Ccos8 sont obtenus. L'analyse par restriction montre que les fragments de 4 et 10 kb ne sont pas sous clonés. En revanche, nous observons que la bande de 5 kb observée initialement était en fait une bande double de 5,1 et 5,2 kb. Ces clones sont passés, par conjugaison tri-parentale, de *E*. *coli à P. putida.* Au bout de 5 jours, seul *P. putida* contenant le sous-clone correspondant à la bande de 5,2 kb du cosmide Ccos8 a poussé sur M63 contenant le 4-HPA comme seule source de carbone. Nous venons donc d'identifier le fragment minimal comportant l'activité 4-HPA 1-hydroxylase. Les clones correspondants à la bande de 5,2 kb sont nommés 5kbC. Pour confirmer le résultat de la complémentation fonctionnelle, nous provoquons l'élimination du plasmide p5kbC en utilisant la stratégie des origines de réplication incompatibles et en forçant l'élimination du plasmide p5kbC par la pression de sélection des antibiotiques utilisés. Nous observons que *P. putida* perd la capacité à croître sur 4-HPA comme seule source de carbone lorsqu'il a perdu le plasmide p5kbC. Nous en concluons que l'activité enzymatique 4-HPA 1-hydroxylase est bien portée par le plasmide p5kbC. Nous pouvons donc faire séquencer l'insert de 5,2 kb, ce qui devrait nous permettre d'identifier le gène *hpa*H fonctionnel.

### II.2.8- Analyse de la séquence de 5,2 kb

L'insert de 5,2 kb du plasmide p5kbC est séquencé. Une recherche d'homologie nucléique (BLASTN) permet d'identifier ainsi trois parties dans l'insert. La première partie comprise entre les bases N° 1 et 1465 est parfaitement homologue d'une partie du plasmide Birmingham IncP-alpha. Il s'agit donc vraisemblablement d'une séquence issue de pLAFR5. Une seconde partie nucléique comprise entre les bases N° 1466 et 1695 présente une homologie parfaite avec une partie du plasmide de clonage M13mp8/pUC8. Cette séquence fait donc encore partie du pLAFR-5 ; en effet le multi-site de clonage de pLAFR-5 provient de pUC8 (Keen *et al.,* 1988). Ainsi, les sites *Eco* RI et *Sma* I (Figure 7) en position respective 1689 et 1695 sont vraisemblablement les sites de clonage du pLAFR-5. La troisième partie, comprise entre les bases 1696 et 5264 (soit 3568 pb) ne présente pas d'homologies fortes. Cette partie d'ADN provient du génome de *P. acidovorans.* Lorsque la séquence de 5,2 kb est analysée en utilisant l'agorithme BLASTX, on identifie des protéines probables (Figure 7). Ainsi la protéine codée par le **gène 1** présente de faibles homologies avec à des béta-lactamases, des déhydrases et des cyclases. La protéine purifiée est codée par le **gène 2** puisque l'on retrouve les séquences codant les peptides internes précédemment obtenus ; c'est donc vraisemblablement la 4-HPA 1-hydroxylase. Les alignements protéiques montrent que cette protéine présente quelques homologies avec des oxygénases et des hydroxylases. La protéine potentiellement codée par le **gène 3** ne présente pas d'homologies avec les bases de données. Enfin le **gène 4** code vraisemblablement un régulateur d'opéron.

Faisons maintenant à une analyse plus fine du gène *hpa*H*.* D'après la séquence protéique N-terminale obtenue, le codon initiation ATG de la protéine 4-HPA 1-hydroxylase se trouve en fait 78 pb en aval d'un codon initiateur GTG en phase avec l'ATG. La séquence Shine-Dalgarno AGGA, permettant la fixation des ribosomes, est retrouvée en amont de l'ATG initiateur mais pas en amont du codon initiateur GTG ; ce qui confirme que la région codante commence au codon initiateur ATG. La portion comprise entre les codons GTG et ATG ne correspond donc vraisemblablement pas à une préprotéine. Ainsi défini, le gène *hpa*H est long de 1737 pb et se termine par le codon stop TGA. Le gène est constitué à 70,9 % de bases GC.

Maintenant que nous avons défini avec précision les limites du gène *hpa*H*,* analysons le produit de sa traduction : la protéine HPAH

### II.2.9- Analyse de la protéine HPAH

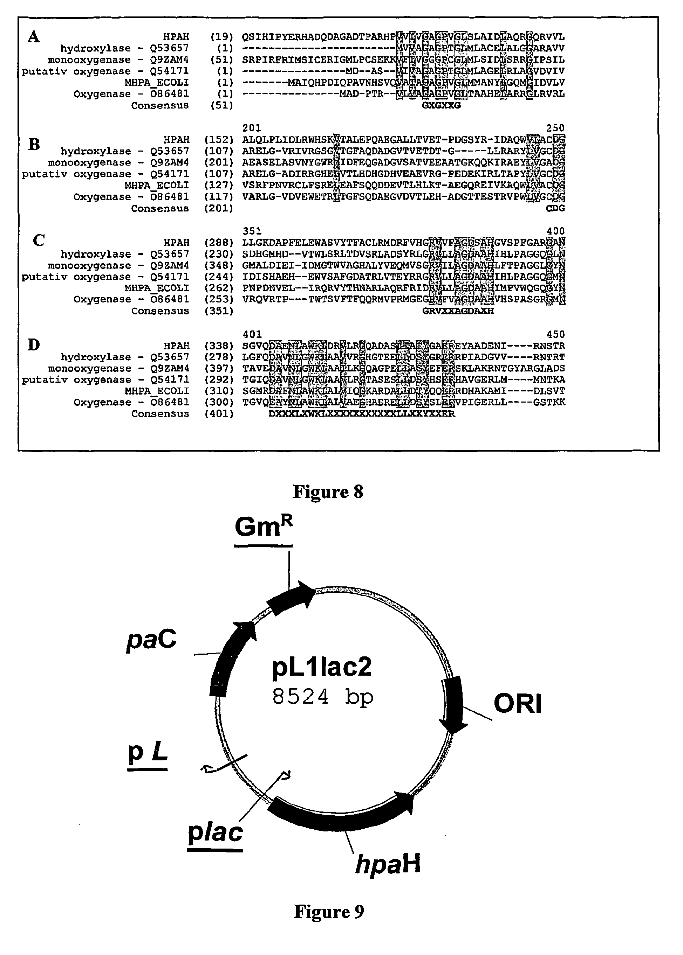

La séquence *hpa*H est traduite en utilisant le sytème universel des codons. Nous obtenons ainsi une protéine de 563 acides aminés, ce qui représente un poids moléculaire de 62,2 kDa. Les recherches d'homologies protéique (BLASTP) montre que l'HPAH présente environ 15 à 25 % d'identité essentiellement avec des protéines d'organismes à Gram positif codant pour des activités enzymatiques apparemment très différentes de celle recherchée. Ainsi on retrouve une oxygénase de *Streptomyces argillaceus,* la 3-(3-hydroxy-phényl)propionate hydroxylase (EC 1.14.13.-) d'E. *coli,* la 2.4-dihydroxybenzoate monooxygénase de *Sphingomonas sp.,* l'enzyme catalysant la 6-hydroxylation de la tétracycline chez *Streptomyces aureofaciens,* une oxygénase potentielle de *Streptomyces fradiae.* En fait, l'HPAH présente des homologies avec les protéines de la famille des phénol monooxygénases (*phe* A) et celles de la famille des 2,4-dichlorophénol hydroxylase (*tƒd*B). L'alignement correspondant aux protéines précitées est réalisé en utilisant l'algoritme ClustalW (Figure 8). Il permet de mettre en évidence des boîtes très conservées. On relèvera entre autre trois motifs d'interaction avec le FAD. Le premier (GXGXXG) correspond au motif structurel β-α-β qui permet l'interaction de la partie ADP du FAD avec la protéine. Le deuxième motif (A/C)DG est impliqué dans la fixation du FAD tandis que le troisième motif G (R) VXX (A) GD (A) XH permet l'interaction avec la partie flavine du FAD. Bien que l'enzyme utilise du NADH, H⁺ le site de fixation correspondant (GDH) n'est pas identifié. Cette absence de site de fixation au NADH,H⁺ est une caractéristique souvent observée chez d'autres monooxygénases à FAD. Enfin, on observe un motif (DXXXLXWKLX XXXXXXXXXX LLXXYXXER) que l'on retrouve aussi chez d'autres hydroxylases (Ferrandez *et al.,* 1997), mais dont la signification n'est pas éclaircie. Bien que la 3-(3-hydroxyphényl)-propionate hydroxylase d'*E. coli* catalyse une réaction d'hydroxylation sur un substrat structurellement proche du 4-HPA, les informations acquises par ces analyses bioinformatiques ne permettent pas de s'assurer que nous avons bien identifié la 4-HPA 1-hydroxylase. La seule manière de le faire, c'est d'exprimer le gène *hpa*H et d'étudier son activité enzymatique.

### II.2.10- Identité des protéines impliquées dans l'activité 4-HPA 1-hydroxylase

### II.2.10.1- Expression du gène hpaH codant la 4-HPA 1-Hydroxylase

Afin de confirmer que le gène *hpa*H code l'activité 4-HPA 1-hydroxylase, il est nécessaire d'exprimer le gène. Pour ce faire, une stratégie de clonage en deux étape est utilisée permettant d'éliminer les gènes N° 1 et 3 et de placer le gène *hpa*H sous la dépendance du promoteur *lac* du vecteur originel pBBR1MCS-Gm-Not-U. Le plasmide obtenu est dénommé pBBR1MCS FT12Δ1. Un extrait brut est réalisé à partir d'une culture sur milieu riche de *P*. *putida* transformée avec ce plasmide. La recherche d'activité par spectrophotométrie (à 340 et 292 nm) montre que le clone possède certes l'activité NADH,H⁺ oxydase induite par l'ajout de 4-HPA, mais ne possède pas la capacité de synthèse de l'homogentisate à partir du 4-HPA. En revanche on observe l'apparition d'une molécule Z ayant un temps de rétention très proche (tr = 1,2 minutes *versus* 1,4 minutes) mais un spectre UV très différent de celui de l'HGA. Nous posons l'hypothèse que l'HPAH oxyde le NADH,H⁺ pour réduire son cofacteur FAD. La réoxydation du FAD se fait au détriment du 4-HPA puisque c'est l'ajout du 4-HPA qui initie la réaction. Le 4-HPA est donc convertit en métabolite Z. Le spectre UV de ce métabolite suggère que le cycle n'est plus aromatique mais peut être cependant insaturé. Nous présentons en figure 2 une hypothèse structurale pour le métabolite Z. Cette expérience montre que le promoteur *lac* est fonctionnel chez *P. putida* en absence d'inducteur IPTG, ce qui suggère que le répresseur *lac*I est naturellement absent chez *P. putida.* Nous démontrons en outre que la protéine initialement purifiée (HPAH) est réellement une NADH,H⁺ oxydase dépendante du 4-HPA qui convertit le 4-HPA en un métabolite Z. La HPAH ne produit pas d'HGA. Il est donc nécessaire d'identifier la ou les protéines partenaires de cette NADH,H⁺ oxydase dépendante du 4-HPA et dont l'ajout permet de restorer l'activité 4-HPA 1-hydroxylase.

### II.2.10.2- Identification de la protéine HPAC par gel filtration

Nous avons vu que l'activité 4-HPA 1-hydroxylase disparaissait lors de la purification de l'HPAH sur colonne d'affinité Red. Nous posons donc l'hypothèse que la ou les protéines partenaires de la NADH,H+ oxydase dépendante du 4-HPA n'ont pas été retenus par la résine d'affinité Red 120 agarose et sont donc récupérées dans le "flow-through". Nous décidons donc de purifier le "flow-through" et de rechercher la ou les protéines qui ajoutées à la HPAH permettent de restorer l'activité 4-HPA 1-hydroxylase. Pour ce faire, le "flow-through" est concentrée par ultrafiltration (Macrosep^{™} 10K) puis chargée sur une colonne de gel filtration S75. Un débit de 1 mL.min⁻¹ est appliqué et les fractions de 1 mL sont collectées. On réalise alors des réactions enzymatiques mettant en présence 50 µL de chaque fraction et 10 µL d'HPAH préalablement purifié sur colonne Red , dans les conditions réactionnelles normales. Les réactions stoppées sont alors analysées par HPLC. On observe que les fractions 90 à 108, lorsque additionnées à de la protéine HPAH, permettent de produire davantage du métabolite Z. La production du métabolite Z est détectée dans ces même fractions en l'absence d'apport d'HPAH. Par ailleurs, sur les gels d'acrylamide correspondants à ces fractions, nous observons une protéine de poids moléculaire équivalent à HPAH. Nous concluons que le "flow-through" contenait encore un peu de protéine HPAH. Lorsque les fractions 109 à 143 sont additionnées à de la protéine HPAH, on observe la production d'HGA. Plus la production d'HGA est forte, plus celle du métabolite Z est faible. Le maximum de production d'homogentisate est obtenu pour les fractions 116 à 128. Le dépôt sur gel acrylamide de des fractions comprises entre 95 et 145 montre qu'une protéine est fortement enrichie dans les fractions 109 à 143, c'est à dire que le profil chromatographique de cette protéine coïncide avec le profil de production d'HGA. Nous décidons de dénommer cette protéine HPAC. La protéine HPAC est excisées du gel puis microséquencée en N-terminal. La séquence obtenue, MTTKTFA, montre que cette protéine est codée par le **gène 1** (Figure 7) que nous dénommons dorénavant *hpa*C. Cette expérience montre que l'activité 4-HPA 1-hydroxylase implique deux protéines, l'HPAH et l'HPAC. Cependant, nous n'avons pas défini la nature de l'interaction entre ces deux protéines : (1) HPAH et HPAC sont elles toutes deux des enzymes ou bien (2) est-ce l'HPAH qui possède une activité enzymatique modifiable en fonction de l'interaction avec l'HPAC.

### II.2.10.3- Nature des interactions entre HPAH et HPAC

L'expérience précédente démontre que les protéines HPAH et HPAC sont nécessaires pour reconstituer l'activité 4-HPA 1-hydroxylase. Deux hypothèses pour expliquer le rôle respectif de ces protéines sont posées. Dans ce paragraphe nous présentons les résultats qui suggèrent que l'HPAC est une enzyme à part entière. Les fractions 100, 101 et 102 de la gel filtration sont rassemblés. Elles contiennent la HPAH c'est à dire l'activité NADH,H+ oxydase qui permet de produire le métabolite Z à partir du 4-HPA. Par ailleurs, les fractions 123, 124 et 125 de la gel filtration sont rassemblées. Elles contiennent la HPAC. Différentes réactions enzymatiques sont réalisées en utilisant l'HPAH et/ou l'HPAC. Ces réactions sont réalisées en deux temps. Une première réaction est réalisée avec l'HPAH (respectivement HPAC), elle est stoppée au bout de 30 minutes par un traitement thermique (100°C, 10 min). On ajoute alors la HPAC (respect. HPAH) et la réaction est poursuivie pour 30 minutes. La réaction est finalement arrêtée par un ajout d'acide perchlorique. Des réactions sont aussi réalisées en remplaçant l'une des enzymes par de l'eau. Enfin, des expériences équivalentes sont réalisées en filtrant les réactions sur Nanosep^{™} 10 kD (Pall Filtron) au lieu de les bouillir.

Le tableau N°1 synthétise les résultats obtenus.

| Expérience N° | "Enzyme" N°1 | "Enzyme" N°2 | Métabolite observé |
|---|---|---|---|
| | HPAH, HPAC | / | HGA |
| **A** | HPAH | H₂O | métabolite Z |
| **B** | HPAH | HPAC | HGA |
| **C** | H₂O | HPAC | / |
| **D** | HPAC | H₂O | / |
| **E** | HPAC | HPAH | métabolite Z |
| **F** | H₂O | HPAH | Métabolite Z |

Nous observons que la seule manière de produire de l'HGA c'est d'avoir les deux protéines HPAH et HPAC simultanément ou successivement dans cet ordre. Lorsque l'HPAH est seule, ou lorsque l'HPAC est introduite avant l'HPAH, seul le métabolite Z est détectable. Enfin, la protéine HPAC n'a aucune activité enzymatique sur le 4-HPA. Ces résultats suggèrent que le métabolite Z est un intermédiaire réactionnel. L'HPAH convertirait le 4-HPA en métabolite Z, cette réaction permettant l'oxydation du NADH,H⁺. Le métabolite Z serait alors convertit en HGA par la HPAC. Les interactions physiques entre les deux protéines n'apparaissent pas nécessaires puisque la protéine HPAH peut être dénaturée ou éliminée par filtration avant ajout de l'HPAC. Nous avons montré *in vitro* que l'activité 4-HPA 1-hydroxylase dépendait des protéines HPAC et HPAH. Cependant la protéine HPAC n'est pas pure en sortie de gel filtration, elle est seulement enrichie. Il reste donc possible qu'en réalité se soit une autre protéine contenue dans cet extrait enrichi qui convertisse le métabolite Z en HGA. Pour éliminer les doutes, nous décidons de cloner les deux gènes (*hpa*C et *hpa*H) sur un même vecteur, nous devrions dans ce cas produire l'activité 4-HPA 1-hydroxylase et donc être capable de faire croître *P. putida* sur milieu minimum contenant du 4-HPA comme seule source de carbone.

### II.2.10.4- Complémentation fonctionnelle de P. putida par hpaH et hpaC

Le plasmide pL1lac2 (Figure 9) est un vecteur pBBR1MCS-Gm^{R} contenant le gène *hpa*C sous la dépendance du promoteur de l'HPPD de *P. fluorescens* et en opposition, le gène *hpaH* sous la dépendance d'un promoteur *lac.* Le plasmide est introduit dans *P. putida* par électroporation. Les bactéries sont alors étalées sur milieu minimum contenant ou non du 4-HPA comme seule source de carbone. Après 5 jours, les colonies sont visibles seulement sur boîtes contenant le 4-HPA comme seule source de carbone. Au bout de 8 jours, les colonies sont de belles tailles. L'ADN plasmidique extrait à partir de ces colonies confirme la présence du plasmide pL1lac2 intègre. Par ailleurs *P. putida* est incapable de croître sur 4-HPA lorsque la bactérie est transformée avec le vecteur pBBR1MCS-GM^{R} contenant soit le gène *hpa*C soit le gène *hpa*H*.* La complémentation fonctionnelle obtenue dans cette expérience confirme que les gènes *hpa*C et *hpa*H sont nécessaires et suffisants pour instaurer l'activité 4-HPA 1-hydroxylase recherchée.

### Exemple III : constructions des différentes cassettes d'expression cytosolique.

### III.1 HPAC

Le gène HPAC a été isolé de Pseudomonas acidovorans par PCR sur un plasmide dérivé (p5kbC) d'une banque cosmidique d'ADN génomique, en utilisant les oligonucléotides suivants:
Start ORF1 (AflIII): GCAGGATGCA CATGTCCACC AAGAC
ORF1 Fin (HindIII): CGGACGCAAG CTTGCATCAG CCTTC

La réaction a été effectuée selon les conditions standards. Le fragment amplifié d'une taille de 993 pb a été sous-cloné dans le plasmide pGEMTeasy (Promega) selon le protocole du fournisseur. Le plasmide pOZ150 ainsi obtenu a été séquencé. La cassette obtenue par digestion EcoRI + SpeI a été clonée dans le plasmide pBluescriptII-KS+ ouvert par les mêmes enzymes pour donner le plasmide pEPA13. Le promoteur CsVMV est isolé du plasmide pCH27, dérivé du plasmide pUC19 contenant la cassette d'expression d'un gène de tolérance herbicide sous le contrôle du CsVMV. Pour cela une PCR standard a été réalisée sur thermocycler avec la Pfu polymérase générant des bouts francs; 1 cycle de 5 min à 95°C, 30 cycles [95°C 30 sec, 57°C 30 sec, 72°C 1 min], 72°C 3 min. Les amorces utilisées sont:
N-CsVMV: GCCCTCGAGG TCGACGGTAT TGATCAGCTT CC introduisant les sites XhoI et BclI
C-CsVMV: CGCTCTAGAA TTCAGATCTA CAAAC (EcoRI)

Le fragment de 565 pb généré est digéré par XhoI+EcoRI avant d'être inséré dans le plasmide pEPA13 préalablement digéré par XhoI+EcoRI; le plasmide pEPA14 est obtenu. Le terminateur Nos est isolé du plasmide pRD11, dérivé de pBlueScript II-SK(-) dans lequel est cloné le terminateur Nos, par digestion HindII+NotI. Le fragment de 292 pb obtenu est cloné dans le plasmide pEPA14 ouvert par les mêmes enzymes, donnant pEPA15.

Cassette pEPA15 = promoteur CsVMV-hpa C- terminateur Nos (Figure 10 ; SEQ ID NO 19)

### III.2. HPAH

Le gène HPAH a isolé de Pseudomonas acidovorans par PCR sur un plasmide dérivé (p5kbC) d'une banque cosmidique d'ADN génomique, en utilisant les oligonucléotides suivants:
Start ORF2 (AflIII): CAGAGGACGA ACAACATGTC CCACC
ORF2 Fin 3(HindIII): CTGTGGATGA AGCTTAAGAG GTTCAGGC

La réaction a été effectuée selon les conditions standard. Le fragment amplifié d'une taille de 1729pb a été sous-cloné bouts-francs dans le plasmide pBlueScript II SK digéré par EcoRV. Le plasmide pEPA16 ainsi obtenu a été séquencé. Le promoteur CaMV 35S est isolé du plasmide pCH14, dérivé du plasmide pBI 121 contenant la cassette d'expression GUS: promoteur CaMV 35S-GUS-terminateur Nos. Pour cela une PCR standard a été réalisée sur thermocycler avec la Pfu polymérase générant des bouts francs; 1 cycle de 5 min à 95°C, 30 cycles [95°C 30 sec, 63°C 30 sec, 72°C 1 min], 72°C 3 min. Les amorces utilisées sont:
N-CaMV: GCATGCCTCG AGCCCACAGA TGG introduisant le site XhoI
C-CaMV: CCACCCGGGG ATCCTCTAGA G introduisant le site BamHI

Le fragment de 839 pb généré est digéré par XhoI+BamHI avant d'être inséré dans le plasmide pEPA16 préalablement digéré par XhoI + BclI; Le plasmide pEPA17 est ainsi obtenu. Le terminateur Nos est isolé du plasmide pRD11 par PCR, sous les mêmes conditions que précédemment, pour 1 cycle de 5 min à 95°C, 30 cycles [95°C 30 sec, 57°C 30 sec, 72°C 1 min], 72°C 3 min., avec les amorces suivantes:
N-Nos: CAAGCTTATC GATACCGTCG ACG introduisant HindIII
C-Nos: GAATTGCGGC CGCAATTCCC GACCTAGGA ACATAG introduisant NotI et AvrII.

Le fragment de 305 pb obtenu est digéré par NotI + HindIII avant d'être cloné dans le plasmide pEPA17 ouvert par les mêmes enzymes, donnant pEPA18.

Cassette pEPA18 = promoteur CaMV 35S-hpa H- terminateur Nos (Figure 11 ; SEQ ID NO17).

### III.3. HPPO

Le gène HPPO a été isolé d'Arthrobacter globiformis par PCR sur le cosmide 2A issu d'une banque cosmidique d'ADN génomique, en utilisant les oligonucléotides suivants:
N-term-HPPO-ScaI: GAATTCAGTA CTTCACTTAC AGTGTCCGGC introduisant les sites de restriction EcoRI et ScaI.
C-term-HPPO-AsuII-XhoI: GAATTCTCGA GTTCGAACAA ACTGAGTAGC AGCTCA introduisant les sites EcoRI, XhoI et AsuII

La réaction a été effectuée selon les conditions standard. Le fragment de 1800 pb obtenu est cloné dans le vecteur pGEMT-easy (Promega) selon le protocole du fournisseur. Le plasmide pOZ151 ainsi obtenu a été séquencé. La cassette obtenue par digestion SphI + XhoI a été clonée dans le plasmide pBBR1-MCS (Gm) ouvert par les mêmes enzymes pour donner le plasmide pEPA20. Le promoteur simple histone est isolé du plasmide pCH9, dérivé du plasmide pUC 19 contenant la cassette d'expression de l'EPSPS: promoteur simple histone-intron2-OTP-EPSPS-terminateur Histone. Pour cela une PCR standard a été réaliséeavec Pfu polymérase générant des bouts francs; 1 cycle de 5 min à 95°C, 5 cycles [95°C 30 sec, 45°C 30 sec, 72°C 1 min], 30 cycles [95°C 30 sec, 65°C 30 sec, 72°C 1 min], 72°C 3 min. Les amorces utilisées sont:
N-SH: GCTTGCATGC CTAGGTCGAG GAGAAATATG introduisant les sites SphI et AvrII
C-SH:CATGAGGGGT TCGAAATCGA TAAGC

Le fragment de 970 pb généré est digéré par SphI avant d'être inséré dans le plasmide pEPA20 préalablement digéré par SphI + ScaI; Dans le plasmide pEPA21 obtenu, l'ATG d'initiation du gène HPPO est recréé derrière le promoteur Simple Histone. Le terminateur Histone est isolé du même plasmide pCH9 par PCR, sous les mêmes conditions que précédemment, pour 1 cycle de 5 min à 95°C, 35 cycles [95°C 30 sec, 55°C 30 sec, 72°C 1 min], 72°C 3 min., avec les amorces suivantes:
N-Hister: CTAGACCTAG GGGATCCCCC GATC introduisant AvrII
C-Hister: CCCACTAGTG TTTAAATGAT CAGTCAGGCC GAAT introduisant SpeI et BclI.

Le fragment de 726 pb obtenu est digéré par SpeI + AvrII avant d'être cloné dans le plasmide pEPA21 ouvert par SpeI, donnant pEPA22.

Cassette pEPA22 = promoteur simple histone-hppO-teminateur histone (Figure 12 ; SEQ ID NO 15).

### III.4. Association des gènes

La cassette contenant le gène HPAC est extraite de pEPA15 par digestion NotI et clonée dans pEPA18 (NotI+Bsp120I) pour former pEPA19 (Figure 13 ; SEQ ID NO 21). Ce dernier est digéré par AvrII pour cloner la cassette extraite dans les sites AvrII+SpeI de pEPA22. Le plasmide contenant les trois constructions est pEPA23 (Figure 14 ; SEQ ID NO 22).

### III.5. Vecteur binaire

Afin de transformer les plantes par *Agrobacterium,* les trois constructions sont extractibles par BclI afin d'être introduites dans un vecteur binaire d*'Agrobactéries.*

### Abréviations :

- 3,4-DHPA: acide 3,4-dihydoxyphénylacétique
- 4-HPA: acide 4-hydroxyphénylacétique
- ADN: acide désoxyribonucléique
- APcI: Ionisation chimique à Pression Atmosphérique
- ARN: acide ribonucléique
- ARNm: acide ribonucléique messager
- BET: bromure d'éthidium
- BLAST: *Basic Local Alignment Search Tool*
- BSA: albumine de sérum bovin
- C¹⁰⁰: carbénicilline (100 µg/mL)
- CRLD: Centre de Recherche La Dargoire
- Da: Dalton
- DKN: dicétonitrile de l'isoxaflutole
- DMSO: diméthylsulfoxyde
- dATP: 2'désoxyadénosine 5'-triphosphate
- dCTP: 2'désoxycytidine 5'-triphosphate
- dGTP: 2'désoxyguanosine 5'-triphosphate
- dNTP: 2'-désoxynucléotides 5'-triphosphate
- dTTP: 2'désoxythymidine 5'-triphosphate
- DTE: dithioerithriol
- DTT: 1,4-dithiothréitol
- EDTA: acide éthylène diamine tétraacétique
- FAD: flavine adénine dinucléotide
- FPLC: *" fast protein liquid chromatography* "
- Gm²⁰: gentamycine (20µg/mL)
- HGA: acide homogentisique
- HPLC: chromatographie liquide haute performance
- HPP: acide hydroxyphénylpyruvique
- HPPD: acide hydroxyphénylpyruvrique dioxygénase
- HPPO: hydroxyphénylpyruvate oxydase
- IFT: isoxaflutole
- IPTG: isopropyl-β-thiogalactopyranoside
- Kn⁵⁰: kanamycine (50 µg/mL)
- kb: kilo bases
- Km: constante de Michaelis Menten
- L-DOPA: 3,4-dihydroxyphénylalanine
- LB: milieu de Luria Bertani
- min: minutes
- mJ: milli-joules
- MNDD: *manganese dependant dioxygenase*
- *Mnd*D: gène codant la MNDD
- NAD⁺(H,H⁺): nicotinamide adénine dinucléotide (forme oxydée/forme réduite)
- OGM: Organisme génétiquement modifié
- OTP: *Optimised Transit Peptid ;* peptide de transit optimisé
- pb: paire de bases
- pBBR1MCS-Gm: plasmide pBBR1MCS résistant à la gentamycine
- PCR: réaction de polymérisation en chaîne
- ppm: partie par million; mg.L⁻¹
- PVDF: polyvinylène difluoride
- qsp: quantité suffisante pour
- Q.r.: coefficient respiratoire
- Rif¹⁰⁰: Rifampicine (100 µg/mL)
- RMN: résonance magnétique nucléaire
- SDS: dodécyle sulfate de sodium
- sec: seconde
- TBE: tris borate EDTA
- TEV: *"tobacco etch virus "*
- TFA: acide trifluoroacétique
- TrEMBL: banque génomique EMBL traduite (*translated EMBL bank*)
- Tris: tris(hydroxyméthyl)aminométhane
- U.V.: ultra-violet
- *vs*: *versus*
- X-gal: 5-bromo-4-chloro-3-β-D-galactopyranoside

### Références

Abe, H. ; Uchiyama, M. ; Sato, R. (1974) Isolation of phenylacetic acid and its p-hydroxyderivative as auxin-like substances from Undaria pinnatifida. Agric. Biol. Chem. 38 : 897-898
Adachi, K.; Takeda, Y.; Senoh, S.; Kita, H. (1964) Metabolism of p-hydroxyphenylacetic acid in Pseudomonas ovalis. Biochem. Biophys. Acta 93: 483-493
Appert, C.; Logemann, E.; Hahlbrock, K.; Schmid, J.; Amrhein, N.; (1994) Structural and catalytic properties of the four phenylalanine ammonia-lyase isoenzymes from parsley (Petroselinum crispum Nym.); Eur. J. Biochem.; 225:491-499
Arunachalam, U.; Massey, V.; Vaidyanathan, C.S. (1992) p-hydroxyphenylacetate 3-hydroxylase. J. Biol. Chem. 267: 25848-25855
Arunachalam, U.; Massey, V. (1994) Studies on the oxidative half-reaction of p-hydroxyphenylacetate 3-hydroxylase. J. Biol. Chem. 269: 11795-11801
Arunachalam, U.; Massey, V.; Miller, S.M. (1994) Mechanism of p-hydroxyphenylacetate 3-hydroxylase. J. Biol. Chem. 269: 150-155
Aubert, S. (1994) Effet multiples du glycérol sur le métabolisme de la cellule végétale non chlorophylienne. These. Université Joseph Fourier - Grenoble - France
Aubert, S.; Gout, E.; Bligny, R.; Marty-Mazars, D.; Barrieu, F.; Alabouvette, J; Marty, F.; Douce, R. (1996a) Ultrastructural and biochemical characterization of autophagy in higher plant cells subjected to carbon deprivation: control by the supply of mitochondria with respiratory substrates. J. Cell Biol. 133: 1251-1263
Aubert, S.; Alban, C.; Bligny, R.; Douce, R. (1996b) Induction of beta-methylcrotonyl-coenzyme A carboxylase in higher plant cells during carbohydrate starvation: evidence for a role of MCCase in leucine metabolism. FEBS Lett. 383: 175-180
Aubert, S.; Bligny, R.; Douce, R. (1996c). NMR studies of metabolism in cell suspensions and tissue cultures, in " Nuclear Magnetic Resonance in Plant Physiology " (Y. Shachar-Hill and P. Pfeffer, Eds.), pp.109-154, American Society of Plant Physiologists, Rockville, USA.
Aubert, S.; Bligny, R.; Day, D.A.; Whelan, J.; Douce, R. 1997. Induction of alternative oxidase synthesis by herbicides inhibiting branched-chain amino acid synthesis. Plant J. 11:649-657
Aubert, S. ; Curien, G. ; Bligny, R. ; Gout, E. ; Douce, R. (1998) Transport, compartimentation and metabolism of homoserine in higher plant cells. Carbon-13 and phosphorus-31-nuclear magnetic resonance studies. Plant Physiol. 116 : 547-557
Aubert, S.; Pallett, K. (2000) Combined use of 13C- and 19F-NMR to analyse the mode of action and the metabolism of the fluoride herbicide isoxaflutole. Plant Physiol. Biochem., 38 : 517-523
Ausubel, F.M.; Brent, R.; Kingston, R.E.; Moore, D.D.; Seidman, J.G.; Smith, J.A.; Struhl, K. (1995) Current protocols in molecular biology(volume 1-4). Wiley ed., Massachusetts General Hospital & Harward Medical School.
Bate, N.J.; Orr, J.; Ni, W.; Meromi, A.; Nadler-Hassar, T.; Doerner, P.W.; Dixon, R.A.; Lamb, C.J.; Elkind, Y. (1994) Quantitative relationship between phenylalanine ammonia-lyase levels and phenylpropanoid accumulation in transgenic tobacco identifies a rate-determining step in natural product synthesis. Proc. Natl. Acad. Sci. USA 91: 7608-12
Battersby, A.R.; Chrystal, E.J.; Staunton, J. (1980) Studies of enzyme-mediated reactions. Part 12. Stereochemical course of the decarboxylation of (2S)-tyrosine to tyramine by microbial, mammalian and plant systems. J. Chem. Soc. 1: 31-42
Bickel, H.; Palme, L.; Schultz, G. (1978) Incorporation of shikimate and other precursors into aromatic amino acids and prenylquinones of isolated spinach chloroplasts. Phytochemistry. 17: 119-124
Bickel, H.; Buchholz, G.; Schultz, G. (1979) On the compartimentation of the biosynthesis of aromatic amino acids and prenylquinones in higher plants. In Advances in the biochemistry and physiology of plant lipids, Appelqvist, L.A. Liljenberg, C., eds. Elsevier, Amsterdam pp 369-375
Biswas, I. ; Gruss, A. ; Ehrlich, S.D. ; Maguin, E. (1993) High-efficiency gene inactivation and replacement system for Gram-positive bacteria. J. Bacteriol. 175 : 3628-3635
Blakley, E.R. (1977) The catabolism of L-tyrosine by an Arthrobacter sp., Can. J. Microbiol. 23 : 1128-1139
Bligny, R. ; Leguay, J.J. (1987) Techniques of cell cultures. Meth. Enzymol. 148 : 3-16
Boehringer Mannheim (1995) The DIG system user's guide for filter hybridization
Boldt, Y.R.; Sadowsky, M.J.; Ellis, L.B.M.; Que, L.; Wackett, L.P. (1995) A Manganese-dependent Dioxygenase from Arthrobacter globiformis CM-2 belongs to the major extradiol dioxygenase family. J. Bacteriol. 177: 1225-1232
Borresen, T ; Klausen, N.K. ; Larsen, L.M. ; Sorensen, H. (1989) Purification and characterisation of tyrosine decarboxylase and aromatic-L-amino-acid decarboxylase. Biochem. Biophys. Acta 993 : 108-115
Bradford, M.M. (1976) A rapid and sensitive method for the quantification of microgram quantities of protein utilizing the principle of protein-dye binding. Anal. Biochem. 72 : 248-254
Brouquisse, R.; James, F.; Pradet, A.; Raymond, P. (1992) Asparagine metabolism and nitrogen distribution during protein degradation in sugar-starved maize root tips. Planta. 188: 384-395
Callis, J. (1995) regulation of proteine degradation. Plant Cell. 7 : 845-857
Campos-Garcia J., Najera R., Camarena L., Soberon-Chavez G. (2000) The Pseudomonas aeruginosa motR gene involved in regulation of bacterial motility. FEMS Microbiol Lett. 184: 57-62
Chan, M.T.; Chao, Y.C.; Yu, S.M. (1994) Novel gene expression system for plant cells based on induction of alpha-amylase promoter by carbohydrate starvation. J. Biol Chem. 269: 17635-17641
Chang, A.K. ; Duggleby, R.G. (1997) Expression, purification and characterization of Arabidopsis thaliana acetohydroxyacid synthase. Biochem. J. 327 : 161-169
Chevalier, C.; Bourgeois, E.; Just, D.; Raymond, P.; (1996) Metabolic regulation of asparagine synthetase gene expression in maize (Zea mays L.) root tips. Plant J. 9: 1-11
Chua, N.H. (1980) electrophoretic analysis of chloroplast proteins. Methods Enzymol. 69 : 434-446
Coligan, J.E.; Dunn, B.M.; Ploegh, H.L.; Speicher, D.W.; Wingfield, P.T. Current protocols in protein science. Wiley ed.
Coligan, J.E. (1997) Chapter 11 : Chemical analysis in Current Protocols in Protein Science. Coligan, J.E. ; Dunn, B.M. ; Ploegh, H.L. ; Speicher, D.W. ; Wingfield, P.T. (eds.) Wiley. Vol. 1.
David, C.; Daro, A.; Szalai, E.; Atarhouch, T.; Mergeay, M.(1996) Formation of polymeric pigments in the présence of bacteria and comparison with chemical oxidative coupling-II. Catabolism of tyrosine and hydroxyphenylacetic acid by Alcaligenes eutrophus CH34 and mutants. Eur. Polym. J., 32: 669-679
De Lorenzo V., Herrero M., Jakubzik U., Timmis K.N. (1990) Mini-Tn5 transposon derivatives for insertion mutagenesis, promoter probing, and chromosomal insertion of cloned DNA in gram-negative eubacteria. J Bacteriol. 172: 6568-6572
D'Souza LM, Willson RC, Fox GE (2000) Expression of marker RNAs in Pseudomonas putida Curr Microbiol. 40: 91-95
Despeghel, J.P. ; Delrot, S. (1983) Energetics of amino acids uptake by Vicia ƒaba leaf tissue. Plant Physiol. 71 : 1-6
Dey & Harborne (1997) Plant Biochemistry, page 389, Ed. P.M. Dey &J.B. Harborne, Academic Press
Fedi S., Brazil D., Dowling D.N., O'Gara F. (1996) Construction of a modified mini-Tn5 lacZY non-antibiotic marker cassette: ecological evaluation of a lacZY marked Pseudomonas strain in the sugarbeet rhizosphere. FEMS Microbiol Lett. 135: 251-257
Feretti, L. ; Sgaramella, V. (1981) Specific and reversible inhibition of the blunt end joining activity of the T4 DNA ligase. Nucleic Acid Res. 9 : 3695-3705
Ferrandez, A. ; Garcia, J.L. ; Diaz, E. (1997) Genetic characterization and expression in heterologous hosts of the 3-(3-hydroxyphenyl)-propionate catabolic pathway of Escherichia coli K12.J. Bacteriol. 179 : 2573-2581
Filleur, S.; Daniel-Vedele, F. (1999) Expression analysis of a high affinity nitrate transporter isolated from Arabidopsis thaliana by differential display. Planta 207: 461-469
Flodin, C.; Whitfield, F.B. (1999) 4-hydroxybenzoic acid: a likely precursor of 2,4,6-tribromophenol in Ulva lactuca. Phytochemistry. 51: 249-255
Flügge, U.-I. (1998) Metabolite transporters in plastids. Curr. Opinion Plant Biotech., 1: 201-206
Folch, J. ; Lees, M. ; Sloane-Stanley, G.H. (1957) A simple method for the isolation and purification of lipids from animal tissues. J Biol Chem 226: 497-509
Frommer, W.B. ; Kwart, M. ; Himer, B. ; Fischer, W.N. ; Hummel, S. ; Ninnemann, O. (1994) Transporters for nitrogenous compounds in plants. Plant Mol. Biol. 26 : 1651-1670
Gaines, C.G.; Byng, G.S.; Whitaker, R.J.; Jensen, R.A. (1982) L-tyrosine regulation and biosynthesis via arogenatedehydrogenase in suspension-cultured cells of Nicotiana silvestris speg. et comes. Planta,156: 233-240
Galan, B. ; Diaz, E. ; Prieto, M.A. (2000) Functional analysis of the small component of the 4-hydroxyphenylacetate 3-monooxygenase of Escherichia coli W : a prototype of a new flavin :NAD(P)H reductase subfamily.J. Bacteriol. 182 : 627-636
Garcia, I.; Rodgers, M.; Lenne, C.; Rolland, A.; Sailland, A.; Matringe, M. (1997) Subcellular localisation and purification of a p-hydroxyphenylpyruvate dioxygenase from cultured carrot cells and characterisation of the corresponding cDNA. Biochem. J.; 325: 761
Garcia, I. ; Rodgers, M. ; Pépin, R.; Ksieh, T.-F., Matringe, M. (1999) Characterization and subcellular compartmentation of recombinant 4-hydroxyphenylpyruvate dioxygenase from Arabidopsis in transgenic tobacco. Plant Physiol. 119 : 1507-1516
Gazzarini, S.; Lejay, L.; Gojon, A.; Ninnemann, O.; Frommer, W.B.; von Wiren, N. (1999) Three functional transporters for constitutive, diurnally regulated and starvation-induced uptake of ammonium into Arabidopsis roots. Plant Cell. 11: 937-948
Genix, P.; Bligny, R.; Martin, J.B.; Douce, R. (1990) Transient accumulation of asparagine in sycamore cells after a long period of sucrose starvation. Plant Physiol. 94: 717-722
Georgalaki, M.D. ; Sarantinopoulos, P. ; Ferreira, E.S. ; De Vuyst, L. ; Kalantzopoulos, G. ; Tsakalidou, E. (2000) Biochemical properties of Streptococcus macedonicus strains isolated from Greek Kasseri cheese. J. Appl. Microbiol. 88: 817-825
Goodchild, J.A.; Givan, C.V. (1990) Influence of ammonium and extemal pH on the amino and organic acid content of suspension culture cells of Acer pseudoplatanus L. Physiol. plant 78: 29-37
Goodwin & Mercer (1988) Introduction to plant biochemistry, 2nd edition. Pergamon Press p. 356
Gout, E.; Bligny, R.; Genix, P.; Tissut, M.; Douce, R. (1992). Effect of glyphosate on plant cell metabolism. 31P and 13C NMR studies. Biochimie. 74: 875-882
Greenberg, D.M. ( ) Metabolic pathways. Amino acids and tetrapyrroles, 3rd edition, Academic Press, vol. III : p. 148
Gross, D. (1975) Growth regulating substances of plant origin. Phytochemistry. 14 : 2105-2112
Grunstein, M.; Hogness, D.S. (1975) Colony hybridization : a method for the isolation of cloned DNAs that contain a specific gene. Proc. Natl. Acad. Sci. U.S.A. 72 : 3961-3965
Hahlbrock, K.; Scheel, D.; (1989) Physiology and molecular biology of phenylpropanoid metabolism. Ann. Rev. Plant Physiol. Plant Mol. Biol.; 40: 347-369
Hareland, W.A.; Crawford, R.L.; Chapman, P.J.; Dagley, S. (1975) Metabolic function and properties of 4-hydroxyphenylacetic acid 1-hydroxylase from Pseudomonas acidovorans. J.Bacteriol.,121: 272-285
Hess, J.L. (1993) Vitamine E, α-Tocophérols. In Antioxidans in Higher Plants Edited by Alscher, R.; Hess, J.; Boca Raton: CRC: p.:111-134
Hill, C.M. ; Duggleby, R.G. (1998) Mutagenesis of Escherichia coli acetohydroxyacid synthase isoenzyme II and characterization of three herbicide-insensitive forms. Biochem. J. 335 : 653-661
Homeyer, U.; Litek, K.; Huchzermeyer, B.; Schultz, G. (1989) Uptake of phenylalanine into isolated barley vacuoles is driven by both tonoplast adenosine triphosphatase and pyrophosphatase. Plant Physiol. 89: 1388-1393
Jones, D.; Keddie, R.M. (1991) The genus Arthrobacter. In: Thé procaryotes (Balows, A.; Trüper, H.G.; Dworkin, M;; Harder, W.; Schleifer, K.H., eds.) 2nd eds., Springer-Verlag, New-York
Journet E.P., Bligny R., Douce R. (1986) Biochemical changes during sucrose deprivation in higher plant cells. J. Biol. Chem. 261: 3193-3199
Junge, K.; Gosink, J.J.; Hoppe, H.-G.; Staley, J.T. (1998) Arthrobacter, Brachybacterium and Planococcus isolates identified from antarctic sea ice brine. Description of Planococcus mcmeekinii, sp. nov.. System. Appl. Microbiol., 21: 306-314
Kaiser, G. ; Martinoia, E. ; Wiemken, A. (1982) Rapid appearance of photosynthetic products in the vacuoles isolated from barley mesophyll protoplasts by a new fast method. Z. Pflanzenphysiol. 107 : 103-113
Keen, N.T., Tamaki, S., Kobayashi, D., Trollinger, D., (1988), Improved broad-host-range plasmids for DNA cloning in gram-negative bacteria, Gene, 70, 191-197
Kindl, H. (1969) Biosynthesis and metabolism of hydroxyphenylacetic acids in higher plants. Eur. J. Biochem.. 7: 340-347
Koch, C.; Schumann, P.; Stackebrandt, E. (1995) Reclassification of Micrococcus agilis (Ali-Cohen 1889) to the genus Arthrobacter as Arthrobacter agilis comb. nov. and emendation-of the genus Arthrobacter. Int. J. Syst. Bacteriol. 45: 837-839
Kovach ME, Phillips RW, Elzer PH, Roop RM 2nd, Peterson KM (1994) pBBR1MCS: a broad-host-range cloning vector. Biotechniques 16: 800-802
Kovach ME, Elzer PH, Hill DS, Robertson GT, Farris MA, Roop RM 2nd, Peterson KM (1995) Four new derivatives of the broad-host-range cloning vector pBBR1MCS, carrying different antibiotic-resistance cassettes. Gene 166: 175-176
Kruk, J.; Strzalka, K. (1998) Identification of plastoquinone-C in spinach and maple leaves by reverse-phase high-performance liquid chromatography. Phytochemistry 49: 2267-2271
Laemmli, U.K. (1970) Cleavage of structural proteins during the assembly of the head of bacteriophage T4. Nature. 227: 680-683
Lamport, D.T.A.; Northcote, D.H. (1960) Hydroxyproline in primary cell walls of higher plants. Nature: 188: 665-666
Laursen, R.A. (1971) Solid-phase Edman degradation. An automatic peptide sequencer. Eur. J. Biochem. 20 : 89-102.
Li, Z.-C; Bush, D.R. (1990) ΔpH-dependent amino acid transport into plasma membrane vesicles isolated from sugar beet leaves. I. Evidence for carrier-mediated, electrogenic flux through multiple transport systems. Plant Physiol. 94: 268-277
Li, Z.-C; Bush, D.R. (1991) ΔpH-dependent amino acid transport into plasma membrane vesicles isolated from sugar beet (Beta vulgaris L.) leaves. II. Evidence for multiple aliphatic, neutral amino acid symports. Plant Physiol. 96: 1338-1344
Liu, D.-L.; Xu, S.-X.; Wang, W.-F. (1998) a novel lignan glucoside from Forsythia suspensa Vahl. J. Chin. Pharmaceut. Sci. 7: 49-51
Löffelhardt, W. (1977) The biosynthesis of phenylacetic acids in blue-green alga Anacystis nidulans: evidence for the involvement of a thylakoïd-bound L-amino acid oxidase. Z. Naturforsch. 32: 345-350
Luscombe, B.M.; Palett, K.E.; Loubierre, P.; Millet, J.-C.; Melgarejo, J.; Vrabel, T.E. (1995) RPA 201772 a novel herbicide for broad leaf and grass weeds control in maize and sugar cane, Proc. Brighton Crop Prot. Conf Weeds, 1: 35
Luscombe, B.M.; Pallett, K.E.; (1996) Isoxaflutole for weed control in maize, Pesticide Outlook, December, 29
Lutterbach, R.; Stöckigt, J. (1994) in vivo investigation of plant cell metabolism by means of natural-abundance 13C-NMR spectroscopy. Helv. Chim. Acta, 77: 2153-2161
Lutterbach, R.; Stöckigt, J.;(1995), Dynamics of the biosynthesis of methylursubin in plant cells employing in vivo 13C-NMR without labelling: Phytochemistry, 40: 801-806
MacLaughlin, P.J.; Weihrauch, J.L. (1979) Vitamine E content of foods. J. Am. Diet. Assoc.75: 647-665
Maniatis, T.; Fritsch, E.F.; Sambrook, J. (1982) Molecular cloning - A laboratory manual eds Cold Spring Harbor Laboratory
Martin, M. ; Gibello, A. ; Femandez, J. ; Ferrer, E. ; Garrido-Pertierra, A. (1991) Catabolism of 3- and 4-hydroxyphenylacetic acid by Klebsiella pneumoniae. J. Gen. Microbiol. 132 : 621-628.
Mayer, M.P. ; Beyer, P. ; Kleinig, H. (1990) Quinone compounds are able to replace molecular oxygen as terminal electron acceptor in phytoen desaturation in chromoplasts of Narcissus pseudonarcissus L. Eur. J. Biochem. 191 : 359-363
Mayer,M.P.; Nievelstein, V.; Beyer, P. (1992) Purification and characterization of a NADPH dependent oxidoreductase from chromoplasts of Narcissus pseudonarcissus: a redox-mediator possibly involved in carotene desaturation. Plant Physiol. Biochem.30: 389-398
Mazelis, M. (1980) Amino acid metabolism. In "the biochemistry of plants" (Stumpf, PK, Conn, E.E; eds.), vol.5: amino acids and derivatives. Academic press, London, New York, pp: 1-55
Michal, G. - ed.- (1999) Biochemical Pathways, An atlas of biochemistry and molecular biology, Wiley & Spektrum eds., p. 60
Miflin, B.J. ; Lea, P.J. (1982) Ammonium assimilation and amino acid metabolism. In A.B. Boulter, B. Parthiers, eds, Encyclopedia of Plant Physiology, Vol. 14, Nucleic Acids and Proteins in Plants 1. Springer Verlag, Berlin, PP : 5-64
Moreno-Arribas, V. ; Lonvaud-Funel, A. (1999) Tyrosine decarboxylase activity of Lactobacillus brevis IOEB 9809 isolated from wine and L. brevis ATCC 367. FEMS Microbiology Letters. 180 : 55-60
Moreno-Arribas, V. ; Torlois, S. ; Joyeux, A. ; Bertrand, A. ; Lonvaud-Funel, A. (2000) Isolation, properties and behaviour of tyramine-producing lactic acid bacteria from wine. J. Appl. Microbiol. 88 : 584-593
Morot-Gaudry, J.F. (1997) Assimilation de l'azote chez les plantes. Aspects physiologique, biochimique et moléculaire. INRA Editions.
Mouillon, J.M.; Aubert, S.; Bourguignon, J.; Gout, E.; Douce, R.; Rebeillé, F. (1999) Glycine and serine catabolism in non-photosynthetic higher plant cells: their role in C1 metabolism. Plant J. 20: 197-205
Murashige, T.; Skoog (1962) A revised medium for rapid growth and bio assays with tabacco tissue cultures. Physiol. Plant. 15: 473
Negrel, J.; Javelle, F.;(1995); Induction of phenylpropanoid and tyramine metabolism in pectinase or pronase elicited cell suspension cultures of tabacco (Nicotiana tabacum); Physiologia Plantarum, 95: 569-574
Nester, E.W.; Montoya, A.L. (1976) An enzyme common to histidine and aromatic amino acid biosynthesis in Bacillus subtilis. J. Bacteriol. 126: 699-705
Norris, S.R.; Barette, T.R.; DellaPenna, D. (1995) Genetic dissection of carotenoid synthesis in Arabidopsis defines plastoquinone as an essential component of phytoene desaturation. Plant Cell 7: 2139-2149
Pallett, K.E.; Little, J.P.; Sheekey, M.; Veerasekaran, P. (1998) The mode of action of Isoxaflutole. I. Physiological effects, metabolism and selectivity. Pestic. Biochem. Physiol. 62 : 113-124
Prieto, M.A.; Perez-Randa, A.; Garcia, J.L. (1993) Characterization of an Escherichia coli aromatic hydroxylase with a broad substrat range. J. Bacteriol. 175: 2162-2167
Prieto, M.A.; Garcia, J.L.; (1994) Molecular characterization of 4-hydroxyphenylacetate 3-hydroxylase of Escherichia coli. J. Biol. Chem. 269: 22823-22829
Prieto, M.A. ; Diaz, E. ; Garcia, J.L. (1996) Molecular characterization of the 4-hydroxyphenylacetate catabolic pathway of Escherichia coli W : engineering a mobile aromatic degradative cluster. J. Bacteriol. 178 : 111-120
Prieto M.A., Kellerhals M.B., Bozzato G.B., Radnovic D., Witholt B., Kessler B. (1999) Engineering of stable recombinant bacteria for production of chiral medium-chain-length poly-3-hydroxyalkanoates. Appl Environ Microbiol. 65: 3265-3271
Roberts, J.K.M. (2000) NMR adventures in the metabolic labyrinth within plants. Trends Plant Sci. 5 : 30-34
Roby, C.; Martin, J.B.; Bligny, R.; Douce, R. (1987) Biochemical changes during sucrose deprivation in higher plant cells. Phosphorus-31 nuclear resonance magnetic studies. J. Biol. Chem. 262: 5000-5007
Sailland, A.; Matringe, M.; Rolland, A.; Pallett, K. (1995) Gène de l'hydroxy-phényl pyruvate dioxygénase et obtention de plantes contenant ce gène résistantes aux herbicides. WO 96/38567
Sailland, A., Derose, R. (1999) Method for enzymatic preparation of homogentisate. WO9934008 A 19990708
Sambrook; Fritsch; Maniatis. Molecular cloning. A laboratory manual, 2nd edition
Schroeder, C.; Sommer, J.; Humpfer, E.; Stöckigt, J. (1997) Inverse correlated 1H-13C in vivo NMR as a probe to follow the metabolism of unlabelled vanillin by plant cell cultures. Tetrahedron, 53: 927-934
Schoenle, E.J. ; Adams, L.D. ; Sammons, D.W. (1984) Insulin-induced rapid decrease of a major protein in fat cell plasma membranes. J. Biol. Chem. 259 : 12112-12116
Shachar-Hill, Y.; Pfeffer, P.E.; Germann, M.W. (1996) Following plant metabolism in vivo and in extracts with heteronuclear two-dimensional nuclear magnetic resonance spectroscopy. Analytic. Biochem., 243: 110-118
Singer, M. ; Berg, P. (1992) Genes & Genomes. Ed. VIGOT, Paris
Sparnins, V.L. ; Dagley, S. (1975) Alternative routes of aromatic catabolism in Pseudomonas acidovorans and Pseudomonas putida : Gallic acid as a substrate and inhibitor of dioxygenases. J. Bacteriol. 124 : 1374-1381
Stafford, H.A.; (1994) Anthocyanins and betalains : evolution of the mutually exclusive pathways. Plant Sci.; 101: 91-98
Suemori, A.; Nakajima, K.; Kurane, R.; Nakamura, Y. (1995) L-Phenylalanine and L-Tyrosine degradative pathways in Rhodococcus erythropolis. Report Nat. Inst. Biosci. Hum. Tech. 3: 33-36
Suemori, A.; Nakajima, K.; Kurane, R.; Nakamura, Y. (1996) Purification and characterization of o-hydroxyphenylacetate 5-hydroxylase, m-hydroxyphenylacetate 6-hydroxylase and p-hydroxyphenylacetate 1-hydroxylase from Rhodococcus erythropolis. Journal of Fermentation and Bioengineering, 81: 133-137
Swiatek, L.; Grabias, B.; Kalemba, D. (1998) Phenolic acids in certain medicinal plants of the genus Artemisia. Pharm. Pharmacol. Lett. 4: 158-160
Swiatek, L. (1977) kwasy fenolowe I Glukozydy irydoidowe w niektorych krajowych gatunkach leczniczych z rodzaju Plantago. Herba Polonica XXIII (3): 201-209
Takizawa, N.; Yokoyama, H.; Yanagihara, K.; Hatta, T.; Kiyohara, H. (1995) A locus of Pseudomonas pickettii DTP0602. had, that encodes 2,4,6-trichlorophenol 4-dechlorinase with hydroxylase activity, and hydroxylation of various chlorophenols by the enzyme. J. Ferment. Bioeng. 80: 318-326
Trieu-Cuot, P. ; Carlier, C. ; Poyart-Salmeron, C. ; Courvalin, P. (1991) An integrative vector exploiting the transposition properties of Tn1545 for insertional mutagenesis and cloning of genes from Gram-positive bacteria. Gene 106: 21-27
Tseng, T.C.; Tsai, T.H.; Lue, M.Y.; Lee, H.T. (1995) Identification of sucrose-regulated genes in cultured rice cells using mRNA differential display. Gene. 161: 179-1782
Vertes, A.; Asai, Y.; Inui, M.; Kobayashi, M.; Kurusu, Y.; Yukawa, H., (1994) Transposon mutagenesis of Coryneform bacteria, Mol. Gen. Genet., 245, 397-405
Vierstra, R.D. (1993) Protein degradation in plants. Ann. Rev. Plant Physiol. Plant Mol. Biol. 44 : 385-410
Viviani, F.; Little, J.; Pallett, K.E. (1998) Mode of action of Isoxaflutole - 2-Characterisation of the inhibition of the carrot 4-hydroxyphenylpyruvate dioxygenase by the diketonitrile derivative of isoxaflutole; Pestic. Biochem. Physiol. 62 : 125-134
Whistance, G.R.; Threlfall, D.R. (1970) Biosynthesis of phytoquinones. Homogentisic acid: a precursor of plastoquinones, tocopherols and alpha-tocopherolquinone in higher plants, green algae and blue-green algae. Biochem. J. 117: 593-600
Xun, L.Y. (1996) Purification and characterization of chlorophenol 4-monooxygenase from Burkholderia cepacia AC1100. J. Bacteriol. 178: 2645-2649
Xun, L. & Sandvik, E.R. (2000) Characterization of 4-hydroxyphenylacetate 3-hydroxylase (HpaB) of Escherichia coli as a reduced Flavin Adenine Dinucleotide-utilizing monooxygenase. Appl. Env. Microbiol. 66 : 481-486

## Revendications

1. Procédé pour rendre les plantes tolérantes à un herbicide inhibiteur de l'acide hydroxyphényl pyruvique dioxygénase (HPPD) **caractérisé en ce que** sont exprimées dans lesdites plantes au moins une enzyme insensible au dit herbicide inhibiteur d'HPPD convertissant l'acide hydroxyphényl-pyruvique (HPP) en acide 4-hydroxyphénylacétique (4-HPA) et une HPAH et une HPAC insensibles au dit herbicide inhibiteur d'HPPD convertissant l'4-HPA en homogentisate et dont une au moins de ces deux enzymes est hétérologue, et pour lequel l'HPAH comprend une séquence d'acides aminés sélectionnée parmi le groupe constitué de la SEQ ID NO 8 et de ses fragments, et l'HPAC comprend une séquence d'acides aminés sélectionnée parmi le groupe constitué des SEQ ID NO 10, SEQ ID NO 12 et SEQ ID NO 14, et de leurs fragments.

2. Procédé pour rendre les plantes tolérantes à un herbicide inhibiteur de l'acide hydroxyphényl pyruvique dioxygénase (HPPD) **caractérisé en ce que** sont exprimées dans lesdites plantes au moins une enzyme insensible au dit herbicide inhibiteur d'HPPD convertissant l'acide hydroxyphényl-pyruvique (HPP) en acide 4-hydroxyphénylacétique (4-HPA) et une HPAH et une HPAC insensibles au dit herbicide inhibiteur d'HPPD convertissant l'4-HPA en homogentisate et dont une au moins de ces deux enzymes est hétérologue, et pour lequel l'HPAH est codée par une séquence d'acide nucléique comprenant une séquence sélectionnée parmi le groupe constitué des séquences codantes des SEQ ID NO 7 et SEQ ID NO 17, de leurs fragments et des séquences capables de s'hybrider de manière sélective dans des conditions de milieu très sévères aux dites SEQ ID NO 7ou 17, et l'HPAC est codée par une séquence d'acide nucléique comprenant une séquence sélectionnée parmi le groupe constitué des séquences codantes des SEQ ID NO 9, SEQ ID NO 11, SEQ ID NO 13, et SEQ ID NO 19, de leurs fragments et des séquences capables de s'hybrider de manière sélective dans des conditions de milieu très sévères aux dites SEQ ID NO 9, 11, 13 ou 19.

3. Procédé selon la revendication 1 ou 2, **caractérisé en ce qu'**est exprimé dans la plante une HPP oxydase convertissant l'HPP en 4-HPA.

4. Procédé selon la revendication 3, **caractérisé en ce que** l'HPP oxydase comprend la séquence d'acides aminés sélectionnée parmi le groupe constitué des SEQ ID NO 2, SEQ ID NO 4 et SEQ ID NO 6, et de leurs fragments.

5. Procédé selon la revendication 3, **caractérisé en ce que** l'HPP oxydase est codée par une séquence d'acide nucléique comprenant une séquence sélectionnée parmi le groupe constitué des séquences codantes des SEQ ID NO 1, SEQ ID NO 3, SEQ ID NO 5 et SEQ ID NO15, de leurs fragments et des séquences capables de s'hybrider de manière sélective dans des conditions de milieu très sévères aux dites SEQ ID NO 1, 3, 5, ou 15.

6. Cellule végétale comprenant une première séquence d'acide nucléique codant pour une enzyme insensible à un herbicide inhibiteur d'HPPD convertissant l'HPP en 4-HPA, une seconde séquence d'acide nucléique codant pour une HPAH fonctionnelle insensible au dit herbicide inhibiteur d'HPPD comprenant une séquence d'acides aminés sélectionnée parmi le groupe constitué de la SEQ ID NO 8, et de ses fragments, et une troisième séquence d'acide nucléique codant pour une HPAC fonctionnelle insensible au dit herbicide inhibiteur d'HPPD comprenant la séquence d'acides aminés sélectionnée parmi le groupe constitué de SEQ ID NO 10, SEQ ID NO 12 et SEQ ID NO 14, de leurs fragments, et pour laquelle au moins une des enzymes HPAH ou HPAC est hétérologue.

7. Cellule végétale comprenant une première séquence d'acide nucléique codant pour une enzyme insensible à un herbicide inhibiteur d'HPPD convertissant l'HPP en 4-HPA, une seconde séquence d'acide nucléique codant pour une HPAH fonctionnelle insensible au dit herbicide inhibiteur d'HPPD comprenant une séquence d'acide nucléique sélectionnée parmi le groupe constitué des séquences codantes des SEQ ID NO 7 et SEQ ID NO 17, de leurs fragments et des séquences capables de s'hybrider de manière sélective dans des conditions de milieu très sévères aux dites SEQ ID NO 7 ou 17, et une troisième séquence d'acide nucléique codant pour une HPAC fonctionnelle insensible au dit herbicide inhibiteur d'HPPD comprenant une séquence d'acide nucléique sélectionnée parmi le groupe constitué des séquences codantes des SEQ ID NO 9, SEQ ID NO 11, SEQ ID NO 13 et SEQ ID NO 19 , de leurs fragments et des séquences capables de s'hybrider de manière sélective dans des conditions de milieu très sévères aux dites SEQ ID NO 9, 11, 13 ou 19, et pour laquelle au moins une des enzymes HPAH ou HPAC est hétérologue.

8. Cellule végétale selon la revendication 6 ou 7, **caractérisée en ce que** l'enzyme convertissant l'HPP en 4-HPA est une HPP oxydase.

9. Cellule végétale selon la revendication 8, **caractérisé en ce que** HPP oxydase comprend la séquence d'acides aminés sélectionnée parmi le groupe constitué des SEQ ID NO 2, SEQ ID NO 4 et SEQ ID NO 6, et de leurs fragments.

10. Cellule végétale selon la revendication 8, **caractérisé en ce que** l'HPP oxydase est codée par une séquence d'acide nucléique comprenant une séquence sélectionnée parmi le groupe constitué des séquences codantes des SEQ ID NO 1, SEQ ID NO 3, SEQ ID NO 5 et SEQ ID NO 15, de leurs fragments et des séquences capables de s'hybrider de manière sélective dans des conditions de milieu très sévères aux dites SEQ ID NO 1, 3, 5, ou 15.

11. Plante transformée comprenant une cellule végétale selon l'une des revendications 6 à 10.

12. Graine de plante transformée selon la revendication 11, **caractérisée en ce qu'**elle comprend une cellule végétale selon l'une des revendications 6 à 10.

13. Procédé d'obtention de plante transformée telle que définie dans la revendication 11, **caractérisé en ce que** l'on introduit dans son génome au moins une cassette d'expression comprenant une séquence d'acide nucléique codant pour une HPAH fonctionnelle insensible au dit herbicide inhibiteur d'HPPD comprenant la séquence d'acides aminés sélectionnée parmi le groupe constitué de la SEQ ID NO 8 et de ses fragments, ou au moins une séquence d'acide nucléique codant pour une HPAC fonctionnelle insensible au dit herbicide inhibiteur d'HPPD comprenant la séquence d'acides aminés sélectionnée parmi le groupe constitué des SEQ ID NO 10, SEQ ID NO 12 et SEQ ID NO 14, et de leurs fragments.

14. Procédé d'obtention de plante transformée telle que définie dans la revendication 11, **caractérisé en ce que** l'on introduit dans leur génome au moins une cassette d'expression comprenant une séquence d'acide nucléique codant pour une HPAH fonctionnelle insensible au dit herbicide inhibiteur d'HPPD comprenant la séquence d'acide nucléique sélectionnée parmi le groupe constitué des séquences codantes SEQ ID NO 7 et SEQ ID NO 17, de leurs fragments et des séquences capables de s'hybrider de manière sélective dans des conditions de milieu très sévères aux dites SEQ ID NO 7 ou 17, ou au moins une séquence d'acide nucléique codant pour une HPAC fonctionnelle insensible au dit herbicide inhibiteur d'HPPD comprenant la séquence d'acide nucléique sélectionnée parmi le groupe constitué des séquences codantes SEQ ID NO 9, SEQ ID NO 11, SEQ ID NO 13 et SEQ ID 19, de leurs fragments et des séquences capables de s'hybrider de manière sélective dans des conditions de milieu très sévères aux dites SEQ ID NO 9, 11, 13 ou 19.

15. Procédé de désherbage sélectif de plantes, notamment de cultures, comprenant les étapes suivantes :
a) on fait pousser des plantes transformées dans une zone de culture,
b) on applique sur cette zone de culture un herbicide inhibiteur d'HPPD, **caractérisé en ce que** les plantes transformées sont telles que décrites dans la revendication 11.

16. Procédé de contrôle des mauvaises herbes dans une surface d'un champ où sont présents des graines ou des plantes transformées comprenant l'application dans la dite surface du champ d'une dose d'un herbicide inhibiteur d'HPPD toxique pour lesdites mauvaises herbes et n'affectant pas de manière substantielle les graines ou plantes transformées, **caractérisées en ce que** les graines transformées sont telles que décrites dans la revendication 12 ou les plantes transformées sont telles que décrites dans la revendication 11.

17. Procédé de culture de plantes transformées comprenant le semis de graines transformées dans une surface d'un champ approprié pour la culture desdites plantes, l'application sur ladite surface dudit champ en cas de présence de mauvaise herbe d'une dose d'un herbicide ayant pour cible l'HPPD toxique pour les mauvaises herbes et n'affectant pas de manière substantielle lesdites graines ou lesdites plantes transformées, puis la récolte des plantes cultivées lorsqu'elles arrivent à la maturité souhaitée et éventuellement la séparation des graines des plantes récoltées, **caractérisée en ce que** les graines transformées sont telles que décrites dans la revendication 12.

18. Procédé selon les revendications 16 ou 17, **caractérisé en ce que** l'herbicide est appliqué en pré semis et /ou en post-levée.

## Claims

1. Process for making plants tolerant to a hydroxyphenylpyruvic acid dioxygenase (HPPD)-inhibiting herbicide, **characterized in that**, expressed in said plants are at least one enzyme insensitive to said HPPD-inhibiting herbicide, which enzyme converts hydroxyphenylpyruvic acid (HPP) to 4-hydroxyphenylacetic acid (4-HPA), and one HPAH and one HPAC which are insensitive to said HPPD-inhibiting herbicide and which convert 4-HPA to homogentisate, and in which at least one of these two enzymes is heterologous, and for which the HPAH comprises an amino acid sequence selected from the group constituted of SEQ ID NO 8 and fragments thereof, and the HPAC comprises an amino acid sequence selected from the group constituted of SEQ ID NO 10, SEQ ID NO 12 and SEQ ID NO 14, and fragments thereof.

2. Process for making plants tolerant to a hydroxyphenylpyruvic acid dioxygenase (HPPD)-inhibiting herbicide, **characterized in that**, expressed in said plants are at least one enzyme insensitive to said HPPD-inhibiting herbicide, which enzyme converts hydroxyphenylpyruvic acid (HPP) to 4-hydroxyphenylacetic acid (4-HPA), and one HPAH and one HPAC which are insensitive to said HPPD-inhibiting herbicide and which convert 4-HPA to homogentisate, and in which at least one of these two enzymes is heterologous, and for which the HPAH is encoded by a nucleic acid sequence comprising a sequence selected from the group constituted of the coding sequences of SEQ ID NO 7 and SEQ ID NO 17, fragments thereof, and the sequences capable of hybridizing selectively to said SEQ ID NO 7 or 17 under very strict medium conditions, and the HPAC is encoded by a nucleic acid sequence comprising a sequence selected from the group constituted of the coding sequences of SEQ ID NO 9, SEQ ID NO 11, SEQ ID NO 13 and SEQ ID NO 19, fragments thereof, and the sequences capable of hybridizing selectively to said SEQ ID NO 9, 11, 13 or 19 under very strict medium conditions.

3. Process according to Claim 1 or 2, **characterized in that** an HPP oxidase which converts HPP to 4-HPA is expressed in the plant.

4. Process according to Claim 3, **characterized in that** the HPP oxidase comprises the amino acid sequence selected from the group constituted of SEQ ID NO 2, SEQ ID NO 4 and SEQ ID NO 6, and fragments thereof.

5. Process according to Claim 3, **characterized in that** the HPP oxidase is encoded by a nucleic acid sequence comprising a sequence selected from the group constituted of the coding sequences of SEQ ID NO 1, SEQ ID NO 3, SEQ ID NO 5 and SEQ ID NO 15, and fragments thereof, and the sequences capable of hybridizing selectively to said SEQ ID NO 1, 3, 5 or 15 under very strict medium conditions.

6. Plant cell comprising a first nucleic acid sequence encoding an enzyme insensitive to an HPPD-inhibiting herbicide, which enzyme converts HPP to 4-HPA, a second nucleic acid sequence encoding a functional HPAH insensitive to said HPPD-inhibiting herbicide, comprising an amino acid sequence selected from the group constituted of SEQ ID NO 8 and fragments thereof, and a third nucleic acid sequence encoding a functional HPAC insensitive to said HPPD-inhibiting herbicide, comprising the amino acid sequence selected from the group constituted of SEQ ID NO 10, SEQ ID NO 12 and SEQ ID NO 14, and fragments thereof, and for which at least one of the HPAH or HPAC enzymes is heterologous.

7. Plant cell comprising a first nucleic acid sequence encoding an enzyme insensitive to an HPPD-inhibiting herbicide, which enzyme converts HPP to 4-HPA, a second nucleic acid sequence encoding a functional HPAH insensitive to said HPPD-inhibiting herbicide, comprising a nucleic acid sequence selected from the group constituted of the coding sequences of SEQ ID NO 7 and SEQ ID NO 17, fragments thereof, and the sequences capable of hybridizing selectively to said SEQ ID NO 7 or 17 under very strict medium conditions, and a third nucleic acid sequence encoding a functional HPAC insensitive to said HPPD-inhibiting herbicide, comprising a nucleic acid sequence selected from the group constituted of the coding sequences of SEQ ID NO 9, SEQ ID NO 11, SEQ ID NO 13 and SEQ ID NO 19, fragments thereof, and the sequences capable of hybridizing selectively to said SEQ ID NO 9, 11, 13 or 19 under very strict medium conditions, and for which at least one of the HPAH or HPAC enzymes is heterologous.

8. Plant cell according to Claim 6 or 7, **characterized in that** the enzyme which converts HPP to 4-HPA is an HPP oxidase.

9. Plant cell according to Claim 8, **characterized in that** the HPP oxidase comprises the amino acid sequence selected from the group constituted of SEQ ID NO 2, SEQ ID NO 4 and SEQ ID NO 6, and fragments thereof.

10. Plant cell according to Claim 8, **characterized in that** the HPP oxidase is encoded by a nucleic acid sequence comprising a sequence selected from the group constituted of the coding sequences of SEQ ID NO 1, SEQ ID NO 3, SEQ ID NO 5 and SEQ ID NO 15, and fragments thereof, and the sequences capable of hybridizing selectively to said SEQ ID NO 1, 3, 5 or 15 under very strict medium conditions.

11. Transformed plant comprising a plant cell according to one of Claims 6 to 10.

12. Seed of a transformed plant according to Claim 11, **characterized in that** it comprises a plant cell according to one of Claims 6 to 10.

13. Process for obtaining a transformed plant as defined in Claim 11, **characterized in that** at least one expression cassette comprising a nucleic acid sequence encoding a functional HPAH insensitive to said HPPD-inhibiting herbicide, comprising the amino acid sequence selected from the group constituted of SEQ ID NO 8 and fragments thereof, or at least one nucleic acid sequence encoding a functional HPAC insensitive to said HPPD-inhibiting herbicide, comprising the amino acid sequence selected from the group constituted of SEQ ID NO 10, SEQ ID NO 12 and SEQ ID NO 14, and fragments thereof, is introduced into the genome of said plant.

14. Process for obtaining a transformed plant as defined in Claim 11, **characterized in that** at least one expression cassette comprising a nucleic acid sequence encoding a functional HPAH insensitive to said HPPD-inhibiting herbicide, comprising the nucleic acid sequence selected from the group constituted of the coding sequences SEQ ID NO 7 and SEQ ID NO 17, fragments thereof, and the sequences capable of hybridizing selectively to said SEQ ID NO 7 or 17 under very strict medium conditions, or at least one nucleic acid sequence encoding a functional HPAC insensitive to said HPPD-inhibiting herbicide, comprising the nucleic acid sequence selected from the group constituted of the coding sequences SEQ ID NO 9, SEQ ID NO 11, SEQ ID NO 13 and SEQ ID NO 19, fragments thereof, and the sequences capable of hybridizing selectively to said SEQ ID NO 9, 11, 13 or 19 under very strict medium conditions, is introduced into the genome of said plant.

15. Process for selected weeding of plants, in particular of crops, comprising the following steps:
a) transformed plants are grown in a cultivation zone,
b) an HPPD-inhibiting herbicide is applied to this cultivation zone,
**characterized in that** the transformed plants are as described in Claim 11.

16. Process of weed killing in an area of a field where transformed plants or seeds are present, comprising the application, in said area of the field, of a dose of an HPPD-inhibiting herbicide which is toxic for said weeds and which does not substantially affect the transformed seeds or plants, **characterized in that** the transformed seeds are as described in Claim 12 or the transformed plants are as described in Claim 11.

17. Process for growing transformed plants, which comprises sowing transformed seeds in an area of a field suitable for growing said plants, applying to said area of said field, in the event of weeds being present, a dose of a herbicide having HPPD as the target, which is toxic for the weeds and which does not substantially affect said transformed seeds or said transformed plants, then harvesting the plants grown when they reach the desired maturity and, optionally, separating the seeds from the harvested plants, **characterized in that** the transformed seeds are as described in Claim 12.

18. Process according to Claims 16 or 17, **characterized in that** the herbicide is applied pre-sowing and/or post-emergence.

## Patentansprüche

1. Verfahren, mit dem Pflanzen tolerant gegenüber einem Herbizid gemacht werden, das ein Inhibitor der Hydroxyphenylbrenztraubensäure-Dioxygenase (HPPD) ist, **dadurch gekennzeichnet, dass** in den Pflanzen Folgendes exprimiert wird: mindestens ein Enzym, das gegenüber dem HPPD-Inhibitor-Herbizid unempfindlich ist und das Hydroxyphenylbrenztraubensäure (HPP) in 4-Hydroxyphenylessigsäure (4-HPA) umwandelt, sowie eine HPAH und eine HPAC, die gegenüber dem HPPD-Inhibitor-Herbizid unempfindlich sind und die 4-HPA in Homogentisat umwandeln, und wobei mindestens eines dieser zwei Enzyme heterolog ist und wobei die HPAH eine Aminosäuresequenz umfasst, die ausgewählt ist aus der Gruppe, bestehend aus der SEQ ID NR: 8 und ihren Fragmenten, und die HPAC eine Aminosäuresequenz umfasst, die ausgewählt ist aus der Gruppe, bestehend aus den SEQ ID NR: 10, SEQ ID NR: 12 und SEQ ID NR: 14 und ihren Fragmenten.

2. Verfahren, mit dem Pflanzen tolerant gegenüber einem Herbizid gemacht werden, das ein Inhibitor der Hydroxyphenylbrenztraubensäure-Dioxygenase (HPPD) ist, **dadurch gekennzeichnet, dass** in den Pflanzen Folgendes exprimiert wird: mindestens ein Enzym, das gegenüber dem HPPD-Inhibitor-Herbizid unempfindlich ist und das Hydroxyphenylbrenztraubensäure (HPP) in 4-Hydroxyphenylessigsäure (4-HPA) umwandelt, sowie eine HPAH und eine HPAC, die gegenüber dem HPPD-Inhibitor-Herbizid unempfindlich sind und die 4-HPA in Homogentisat umwandeln, und wobei mindestens eines dieser zwei Enzyme heterolog ist und wobei die HPAH durch eine Nukleinsäuresequenz codiert wird, die eine Sequenz umfasst, die ausgewählt ist aus der Gruppe, bestehend aus den codierenden Sequenzen der SEQ ID NR: 7 und SEQ ID NR: 17, aus ihren Fragmenten und aus Sequenzen, die unter sehr stringenten Mediumbedingungen selektiv an die SEQ ID NR: 7 oder 17 hybridisieren können, und die HPAC durch eine Nukleinsäuresequenz codiert wird, die eine Sequenz umfasst, die ausgewählt ist aus der Gruppe, bestehend aus den codierenden Sequenzen der SEQ ID NR: 9, SEQ ID NR: 11, SEQ ID NR: 13 und SEQ ID NR: 19, aus ihren Fragmenten und aus Sequenzen, die unter sehr stringenten Mediumbedingungen selektiv an die SEQ ID NR: 9, 11, 13 oder 19 hybridisieren können.

3. Verfahren nach Anspruch 1 oder 2, **dadurch gekennzeichnet, dass** in der Pflanze eine HPP-Oxidase exprimiert wird, die HPP in 4-HPA umwandelt.

4. Verfahren nach Anspruch 3, **dadurch gekennzeichnet, dass** die HPP-Oxidase die Aminosäuresequenz umfasst, die ausgewählt ist aus der Gruppe, bestehend aus den SEQ ID NR: 2, SEQ ID NR: 4 und SEQ ID NR: 6 und ihren Fragmenten.

5. Verfahren nach Anspruch 3, **dadurch gekennzeichnet, dass** die HPP-Oxidase durch eine Nukleinsäuresequenz codiert wird, die eine Sequenz umfasst, die ausgewählt ist aus der Gruppe, bestehend aus den codierenden Sequenzen der SEQ ID NR: 1, SEQ ID NR: 3, SEQ ID NR: 5 und SEQ ID NR: 15, ihren Fragmenten und aus Sequenzen, die unter sehr stringenten Mediumbedingungen selektiv an die SEQ ID NR: 1, 3, 5 oder 15 hybridisieren können.

6. Pflanzenzelle, die Folgendes umfasst: eine erste Nukleinsäuresequenz, die für ein Enzym codiert, das gegenüber einem HPPD-Inhibitor-Herbizid unempfindlich ist und das HPP in 4-HPA umwandelt, eine zweite Nukleinsäuresequenz, die für eine funktionelle HPAH codiert, die gegenüber dem HPPD-Inhibitor-Herbizid unempfindlich ist und die eine Aminosäuresequenz umfasst, die ausgewählt ist aus der Gruppe, bestehend aus der SEQ ID NR: 8 und ihren Fragmenten, und eine dritte Nukleinsäuresequenz, die für eine funktionelle HPAC codiert, die gegenüber dem HPPD-Inhibitor-Herbizid unempfindlich ist und die die Aminosäuresequenz umfasst, die ausgewählt ist aus der Gruppe, bestehend aus SEQ ID NR: 10, SEQ ID NR: 12 und SEQ ID NR: 14 und ihren Fragmenten, und wobei mindestens eines der Enzyme HPAH oder HPAC heterolog ist.

7. Pflanzenzelle, die Folgendes umfasst: eine erste Nukleinsäuresequenz, die für ein Enzym codiert, das gegenüber einem HPPD-Inhibitor-Herbizid unempfindlich ist und das HPP in 4-HPA umwandelt, eine zweite Nukleinsäuresequenz, die für eine funktionelle HPAH codiert, die gegenüber dem HPPD-Inhibitor-Herbizid unempfindlich ist und die eine Nukleinsäuresequenz umfasst, die ausgewählt ist aus der Gruppe, bestehend aus den codierenden Sequenzen der SEQ ID NR: 7 und SEQ ID NR: 17, ihren Fragmenten und aus Sequenzen, die unter sehr stringenten Mediumbedingungen selektiv an die SEQ ID NR: 7 oder 17 hybridisieren können, und eine dritte Nukleinsäuresequenz, die für eine funktionelle HPAC codiert, die gegenüber dem HPPD-Inhibitor-Herbizid unempfindlich ist und die eine Nukleinsäuresequenz umfasst, die ausgewählt ist aus der Gruppe, bestehend aus den codierenden Sequenzen der SEQ ID NR: 9, SEQ ID NR: 11, SEQ ID NR: 13 und SEQ ID NR: 19, aus ihren Fragmenten und aus Sequenzen, die unter sehr stringenten Mediumbedingungen selektiv an die SEQ ID NR: 9, 11, 13 oder 19 hybridisieren können, und wobei mindestens eines der Enzyme HPAH oder HPAC heterolog ist.

8. Pflanzenzelle nach Anspruch 6 oder 7, **dadurch gekennzeichnet, dass** das Enzym, das HPP in 4-HPA umwandelt, eine HPP-Oxidase ist.

9. Pflanzenzelle nach Anspruch 8, **dadurch gekennzeichnet, dass** die HPP-Oxidase die Aminosäuresequenz umfasst, die ausgewählt ist aus der Gruppe, bestehend aus den SEQ ID NR: 2, SEQ ID NR: 4 und SEQ ID NR: 6 und ihren Fragmenten.

10. Pflanzenzelle nach Anspruch 8, **dadurch gekennzeichnet, dass** die HPP-Oxidase durch eine Nukleinsäuresequenz codiert wird, die eine Sequenz umfasst, die ausgewählt ist aus der Gruppe, bestehend aus den codierenden Sequenzen der SEQ ID NR: 1, SEQ ID NR: 3, SEQ ID NR: 5 und SEQ ID NR: 15, ihren Fragmenten und Sequenzen, die unter sehr stringenten Mediumbedingungen selektiv an die SEQ ID NR: 1, 3, 5 oder 15 hybridisieren können.

11. Transformierte Pflanze, die eine Pflanzenzelle nach einem der Ansprüche 6 bis 10 umfasst.

12. Transformierter Pflanzensamen nach Anspruch 11, **dadurch gekennzeichnet, dass** er eine Pflanzenzelle nach einem der Ansprüche 6 bis 10 umfasst.

13. Verfahren zum Erhalten einer transformierten Pflanze nach Anspruch 11, **dadurch gekennzeichnet, dass** man in ihr Genom mindestens eine Expressionskassette einbringt, die eine Nukleinsäuresequenz umfasst, die für eine funktionelle HPAH codiert, die gegenüber dem HPPD-Inhibitor-Herbizid unempfindlich ist und die die Aminosäuresequenz umfasst, die ausgewählt ist aus der Gruppe, bestehend aus der SEQ ID NR: 8 und ihren Fragmenten, oder mindestens eine Nukleinsäuresequenz, die für eine funktionelle HPAC codiert, die gegenüber dem HPPD-Inhibitor-Herbizid unempfindlich ist und die die Aminosäuresequenz umfasst, die ausgewählt ist aus der Gruppe, bestehend aus SEQ ID NR: 10, SEQ ID NR: 12 und SEQ ID NR: 14 und ihren Fragmenten.

14. Verfahren zum Erhalten einer transformierten Pflanze nach Anspruch 11, **dadurch gekennzeichnet, dass** man in ihr Genom mindestens eine Expressionskassette einbringt, die eine Nukleinsäuresequenz umfasst, die für eine funktionelle HPAH codiert, die gegenüber dem HPPD-Inhibitor-Herbizid unempfindlich ist und die die Nukleinsäuresequenz umfasst, die ausgewählt ist aus der Gruppe, bestehend aus den codierenden Sequenzen SEQ ID NR: 7 und SEQ ID NR: 17, aus ihren Fragmenten und aus Sequenzen, die unter sehr stringenten Mediumbedingungen selektiv an die SEQ ID NR: 7 oder 17 hybridisieren können, oder mindestens eine Nukleinsäuresequenz, die für eine funktionelle HPAC codiert, die gegenüber dem HPPD-Inhibitor-Herbizid unempfindlich ist und die die Nukleinsäuresequenz umfasst, die ausgewählt ist aus der Gruppe, bestehend aus den codierenden Sequenzen SEQ ID NR: 9, SEQ ID NR: 11, SEQ ID NR: 13 und SEQ ID NR: 19, ihren Fragmenten und aus Sequenzen, die unter sehr stringenten Mediumbedingungen selektiv an die SEQ ID NR: 9, 11, 13 oder 19 hybridisieren können.

15. Verfahren zur selektiven Unkrautvernichtung von Pflanzen, insbesondere von Kulturpflanzen, bei dem man in den folgenden Schritten:
a) transformierte Pflanzen in einer Kulturzone austreiben lässt,
b) auf diese Kulturzone ein HPPD-Inhibitor-Herbizid aufbringt, **dadurch gekennzeichnet, dass** es sich bei den transformierten Pflanzen um Pflanzen nach Anspruch 11 handelt.

16. Verfahren zur Bekämpfung von Unkraut auf einer Ackeroberfläche, auf der sich transformierte Samen oder transformierte Pflanzen befinden, umfassend das Aufbringen auf die Ackeroberfläche einer Dosis des HPPD-Inhibitor-Herbizids, die für das Unkraut toxisch ist und die transformierten Samen oder transformierten Pflanzen nicht wesentlich beeinträchtigt, **dadurch gekennzeichnet, dass** es sich bei den transformierten Samen um Samen nach Anspruch 12 oder bei den transformierten Pflanzen um Pflanzen nach Anspruch 11 handelt.

17. Verfahren zur Kultur von transformierten Pflanzen, wobei man transformierte Samen auf einer Ackeroberfläche aussät, die für die Kultur dieser Pflanzen geeignet ist, man auf die Ackeroberfläche im Fall des Vorliegens von Unkraut eine Dosis eines auf HPPD abzielenden Herbizids aufbringt, die für das Unkraut toxisch ist und die transformierten Samen oder die transformierten Pflanzen nicht wesentlich beeinträchtigt, und man anschließend die kultivierten Pflanzen erntet, wenn sie die gewünschte Reife erreicht haben, und gegebenenfalls die Samen der geernteten Pflanzen abtrennt, **dadurch gekennzeichnet, dass** es sich bei den transformierten Samen um Samen nach Anspruch 12 handelt.

18. Verfahren nach den Ansprüchen 16 oder 17, **dadurch gekennzeichnet, dass** das Herbizid vor der Aussaat und/oder nach dem Auflaufen aufgebracht wird.
